# EUROPEAN PATENT APPLICATION

(11) **EP 1 881 001 A1**
(43) Date of publication of application: **23.01.2008**
(21) Application number: 07112898.7
(22) Date of filing: 20.07.2007
(51) Int. Cl.: C07K 5/02, C07D 215/00, A61K 38/55, A61K 38/05, A61K 38/06, A61K 38/07, C07K 5/06

(54) **HCV NS-3 serine protease inhibitors**

(30) Priority: 20.07.2006 EP 06117589
(71) Applicant: Tibotec Pharmaceuticals Ltd., Eastgate Little Island Co Cork (IE); MEDIVIR AB, 141 44 Huddinge (SE)
(72) Inventor: Ivanov, Vladimir, S-141 44, Huddinge (SE); Nilsson, Karl Magnus, S-141 44, Huddinge (SE); Rosenquist, Åsa Annica Kristina, S-141 44, Huddinge (SE); Samuelsson, Bengt Bertil, S-141 44, Huddinge (SE); Raboisson, Pierre Jean-Marie Bernard, 1331, Rosieres (BE); De Kock, Herman, 2370, Arendonk (BE); Vendeville, Sandrine Marie Helene, 1150, Woluwe-Saint-Pierre (BE); Simmen, Kenneth Alan, 3080, Tervuren (BE)
(74) Representative: Wante, Dirk Paul Maria

(57) **Abstract**

HCV inhibitors, compositions comprising these compounds as active ingredient, as well as processes for preparing these compounds, having the formula I 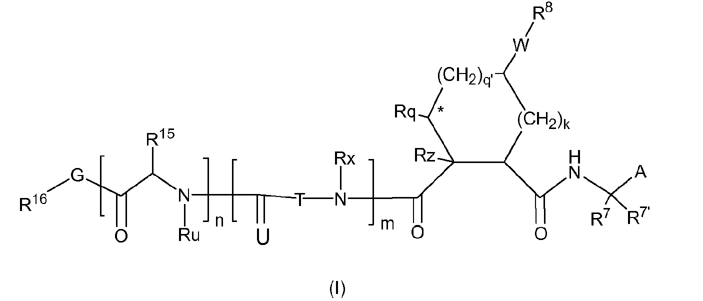 wherein A is

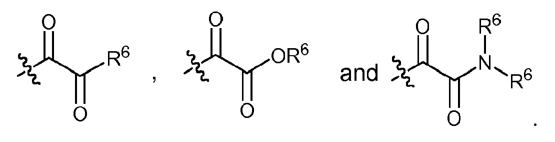

## Description

This invention concerns HCV inhibitors, compositions comprising these compounds as active ingredient, as well as processes for preparing these compounds.

Hepatitis C virus (HCV) is the leading cause of chronic liver disease worldwide and has become a focus of considerable medical research. HCV is a member of the *Flaviviridae* family of viruses in the *hepacivirus* genus, and is closely related to the *flavivirus* genus, which includes a number of viruses implicated in human disease, such as dengue virus and yellow fever virus, and to the animal *pestivirus* family, which includes bovine viral diarrhea virus (BVDV). The HCV genome comprises both 5' and 3' untranslated regions that adopt RNA secondary structures, and a central open reading frame encoding a single polyprotein. The latter is a precursor to ten gene products, which are generated from the precursor polyprotein by an orchestrated series of co- and posttranslational endoproteolytic cleavages mediated by both host and viral proteases. The viral structural proteins include the core nucleocapsid protein, and two envelope glycoproteins E1 and E2. The non-structural (NS) proteins encode some essential viral enzymatic functions (helicase, polymerase, protease), as well as proteins of unknown function. Replication of the viral genome is mediated by an RNA-dependent RNA polymerase, encoded by non-structural protein 5b (NSSB). In addition to the polymerase, the viral helicase and protease functions, both encoded in the bifunctional NS3 protein, have been shown to be essential for replication of HCV RNA. In addition to the NS3 serine protease, HCV also encodes a metalloproteinase in the NS2 region.

Following the initial acute infection, a majority of infected individuals develops chronic hepatitis because HCV replicates preferentially in hepatocytes but is not directly cytopathic. In particular, the lack of a vigorous T-lymphocyte response and the high propensity of the virus to mutate appear to promote a high rate of chronic infection. Chronic hepatitis can progress to liver fibrosis leading to cirrhosis, end-stage liver disease, and HCC (hepatocellular carcinoma), making it the leading cause of liver transplantations. There are 6 major HCV genotypes and more than 50 subtypes, which are differently distributed geographically. HCV type 1 is the predominant genotype in Europe and the US. The extensive genetic heterogeneity of HCV has important diagnostic and clinical implications, perhaps explaining difficulties in vaccine development and the lack of response to therapy.

Transmission of HCV can occur through contact with contaminated blood or blood products, for example following blood transfusion or intravenous drug use. The introduction of diagnostic tests used in blood screening has led to a downward trend in post-transfusion HCV incidence. However, given the slow progression to the end-stage liver disease, the existing infections will continue to present a serious medical and economic burden for decades.

Current HCV therapies are based on (pegylated) interferon-alpha (IFN-α) in combination with ribavirin. This combination therapy yields a sustained virologic response in more than 40% of patients infected by genotype 1 viruses and about 80% of those infected by genotypes 2 and 3. Beside the limited efficacy on HCV type 1, this combination therapy has significant side effects and is poorly tolerated in many patients. Major side effects include influenza-like symptoms, hematologic abnormalities, and neuropsychiatric symptoms. Hence there is a need for more effective, convenient and better-tolerated treatments.

Recently, two HCV protease inhibitors have gained attention, namely BILN-2061 disclosed in WO00/59929 and VX-950 disclosed in WO03/87092. It has already become apparent that the sustained administration of BILN-2061 or VX-950 selects HCV mutants that are resistant to the respective drug, so called drug escape mutants. These have characteristic mutations, notably D168V, D168Y and/or A165S. Accordingly, additional drugs with different resistance patterns are required to provide failing patients with treatment options, and combination therapy is likely to be the norm in the future. Experience with HIV drugs, and with HIV protease inhibitors in particular, has further emphasized that sub-optimal pharmacokinetics and complex dosage regimes quickly result in inadvertent compliance failures, resulting in the emergence of drug escape mutants. Achieving the necessary pharmacokinetics and drug metabolism to allow such through levels provides a stringent challenge to drug design.

There is a need for HCV inhibitors that may overcome the disadvantages of current HCV therapy such as side effects, limited efficacy, the emerging of resistance, and compliance failures. The present invention concerns inhibitors of HCV replication that not only show good activity as HCV inhibitors but also exhibit improved cell permeability, which also results in an enhanced bioavailability.

### Brief description of the invention

In one aspect of the invention provides compounds of the formula I: 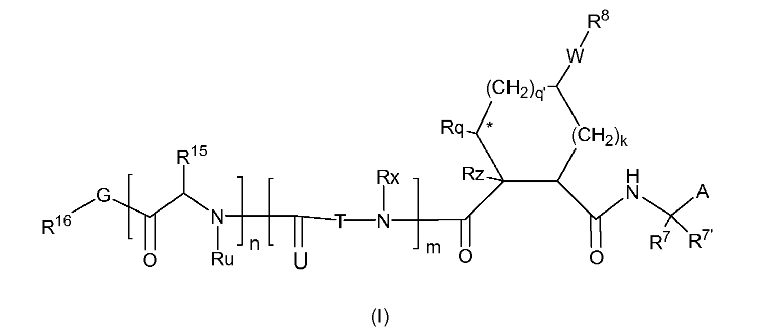 pharmaceutically acceptable salts, or stereoisomeric forms thereof, wherein
- **A**: is selected from 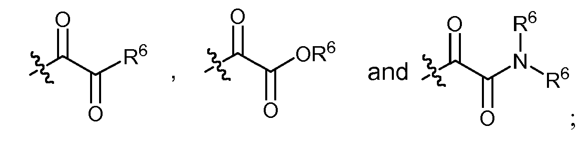 wherein
- each **R⁶**: independently is hydrogen, hydroxy, C₁₋₆alkoxy, C₃₋₇cycloalkyl, aryl, Het, C₁₋₆alkyl or C₂₋₆alkenyl, the latter two being optionally substituted with C₃₋₇cycloalkyl, aryl or with Het; wherein one, two or three hydrogen atoms in the C₁₋₆alkyl, C₂₋₆alkenyl or C₃₋₇cycloalkyl moieties, or one, two or three of the optional substituents of Het or aryl may be optionally replaced by one, two or three substituents selected from halogen, -O-R⁴, nitro, cyano, polyhaloC₁₋₆alkyl, polyhaloC₁₋₆alkoxy, oxo, thioxo, =N(R⁴), =N(OR⁴), -N(R⁴)₂, -SR⁴, -SOR⁴, -SO₂R⁴, -SO₂N(R⁴)₂, -C(O)R⁴, -C(O)C(O)R⁴, -C(O)C(O)-OR⁴, -C(O)C(O)N(R⁴)₂, -C(O)CH₂C(O)R⁴, -C(O)OR⁴, -OC(O)R⁴, -C(O)N(R⁴)₂, -OC(O)N(R⁴)₂, -N(R⁴)N(R⁴)COR⁴, -N(R⁴)N(R⁴)C(O)OR⁴, -N(R⁴)N(R⁴)CON(R⁴)₂, -N(R⁴)SO₂R⁴, -N(R⁴)SO₂N(R⁴)₂, -N(R⁴)C(O)OR⁴, -N(R⁴)C(O)R⁴, -N(R⁴)C(O)N(R⁴)₂, -N(COR⁴)COR⁴, -N(OR⁴)R⁴, -C(=NH)N(R⁴)₂, -C(O)N(OR⁴)R⁴, -C(=NOR⁴)R⁴; and
- each **R⁴**: is independently selected from hydrogen, aryl, C₃₋₇cycloalkyl, or C₁₋₆alkyl optionally substituted with aryl, C₃₋₇cycloalkyl or with Het; or two **R⁶** groups taken together with the nitrogen to which they are bonded form a radical (N)Het;
- **R**⁷: is C₃₋₇cycloalkyl, aryl, Het, C₁₋₆alkyl or C₂₋₆alkenyl, the latter two being optionally substituted with C₃₋₇cycloalkyl, aryl, Het, with 1-3 halo atoms, amino, mono- or di-C₁₋₆alkylamino, -SH; or R⁷ is J;
- **R**^{7'}: is H; or
- **R**^{7'} **and R**⁷: taken together with the carbon atom to which they are attached form a C₃₋₇cycloalkyl ring which may be optionally substituted with R^{7a} wherein;
- **R**^{7a}: is C₁₋₆alkyl, C₃₋₇cycloalkyl, C₂₋₆alkenyl each of which may be optionally substituted with 1, 2 or 3 halo atoms; or R^{7a} can be J;
- **q'**: is 0, 1, 2 or 3; and **k** is 0, 1, 2 or 3;
- **Rz**: is H, or together with one hydrogen atom on the carbon atom marked with an asterisk forms an additional bond;
- **Rq**: is H or C₁₋₆alkyl;
- **W**: is -CH₂-, -O-, -OC(=O)NH-, -OC(=O)-, -S-, -NH-, -NR²-, -NHS(=O)₂-, -NHC(=O)NH-, -NHC(=O)-, -NHC(=S)NH-, or a bond;
- **R**⁸: is a ring system containing 1 or 2 saturated, partially saturated or unsaturated rings each of which has 4-7 ring atoms and each of which has 0 to 4 hetero atoms independently selected from S, O and N, the ring system being optionally spaced from W by a C₁₋₃alkylene group; or R⁸ is C₁₋₆alkyl; any of which R⁸ groups can be optionally mono-, di-, tri-, or tetra-substituted with R⁹;
- each **R**⁹: independently is halo, oxo, cyano, azido, nitro, -C(=O)NH₂, polyhaloC₁₋₆alkyl, C₃₋₇cycloalkyl, aryl, Het, C₁₋₆alkyl being optionally substituted with C₃₋₇cycloalkyl, with aryl or with Het; or **R**⁹ is also -Y-NR²R³, -Y-O-R³, -Y-C(=O)R³, -Y-(C=O)NR²R³, -Y-NR²C(=O)R³, -Y-NHS(=O)ₚR³, -Y-S(=O)ₚR³, -Y-S(=O)ₚNR²R³, -Y-C(=O)OR³, -Y-NR²C(=O)OR³; wherein said C₃₋₇cycloalkyl, aryl, or Het, is optionally substituted with one, two or three R¹⁰ radicals, wherein
- each **Y**: is independently a bond or C₁₋₄alkylene;
- each **R²**: is independently H or C₁₋₆alkyl;
- each **R³**: is independently H, C₃₋₇cycloalkyl, aryl, Het, C₁₋₆alkyl, the latter being optionally substituted with C₃₋₇cycloalkyl, aryl or with Het;
- or **R² and R³**: and the nitrogen to which they both are attached define (N)Het;
- each **p**: is independently 1 or 2;
- each **R¹⁰**: independently is C₁₋₆alkyl, C₃₋₇cycloalkyl, C₁₋₆alkoxy, amino, mono- or diC₁₋₆alkylamino, aminocarbonyl or mono- or diC₁₋₆alkylaminocarbonyl, amido, sulfonyl, (C₁₋₃alkyl)sulfonyl, nitro, cyano, hydroxy, mercapto, halo, haloC₁₋₆alkyl, carboxyl, pyrrolidinyl, piperidinyl, piperazinyl, 4-C₁₋₆alkylpiperazinyl (e.g. 4-methylpiperazinyl) or morpholinyl; and wherein the morpholinyl and piperidinyl groups may be optionally substituted with one or two C₁₋₆alkyl radicals;
- **Rx**: is H, C₁₋₆alkyl or J;
- **T**: is -CHR¹¹-, -NRd-, wherein **Rd** is H**,** C₁₋₆alkyl, or J;
- **R¹¹**: is H, C₁₋₆alkyl, C₃₋₇cycloalkyl, aryl, Het, any of which can be substituted with halo, oxo, cyano, azido, nitro, C₁₋₆alkyl, C₃₋₇cycloalkyl, aryl, Het, -C(=O)NH₂, -Y-NR²R³, -Y-O-R³, -Y-C(=O)R³, -Y-(C=O)NR²R³, -Y-NR²C(=O)R³, -Y-NHSOₚR³, -Y-S(=O)ₚR³, -Y-S(=O)ₚNR²R³, -Y-C(=O)OR³, -Y-NR²C(=O)OR³; or R¹¹ is J;
- **J**,: if present, is a single 3 to 10-membered saturated or partially unsaturated alkylene chain extending from the R⁷/R^{7'} C₃₋₇cycloalkyl or from the carbon atom to which R⁷ is attached to one of Rj, Rx, Ry or R¹¹ to form a macrocycle, which chain is optionally interrupted by one to three heteroatoms independently selected from: -O-, -S- or -NR¹²-, and wherein 0 to 3 carbon atoms in the chain are optionally substituted with R¹⁴; wherein;
- **R¹²**: is H, C₁₋₆alkyl, C₃₋₇cycloalkyl, or -C(=O)R¹³;
- **R¹³**: is C₃₋₇cycloalkyl, aryl, Het, C₁₋₆alkyl; the latter being optionally substituted with C₃₋₇cycloalkyl, aryl or with Het;
- **R¹⁴**: is independently selected from the group consisting of H, C₁₋₆alkyl, haloC₁₋₆alkyl, C₁₋₆alkoxy, hydroxy, halo, amino, oxo, mercapto, and C₁₋₆thioalkyl;
- **Ru**: is independently H or C₁₋₆alkyl;
- **m**: is 0 or 1; **n** is 0 or 1;
- **U**: is O or is absent;
- **R¹⁵**: is H, -Y-C(=O)R³, -Y-C(=O)OR³, -Y-S(=O)ₚR³, C₃₋₇cycloalkyl, aryl, Het, or C₁₋₆alkyl optionally substituted with C₃₋₇cycloalkyl, aryl, or with Het; wherein any of C₃₋₇cycloalkyl, aryl, Het, and C₁₋₆alkyl, can be substituted with halo, oxo, cyano, azido, nitro, C₁₋₆alkyl, amino, mono- or diC₁₋₆alkylamino, hydroxy, C₁₋₆alkoxy, C₁₋₆alkylcabonyl, aminocarbonyl, mono- or diC₁₋₆alkylaminocarbonyl, C₁₋₆alkylthio, mercapto, carboxyl, C₁₋₆alkoxycarbonyl;
- **G**: is -O-, -NRy-, -NRjNRj-, where one Rj is H or C₁₋₆alkyl and the other Rj is H, C₁₋₆alkyl or J;
- **Ry**: is H, C₁₋₆alkyl; or Ry is J;
- **R¹⁶**: is H, -Y-C(=O)R³, -Y-C(=O)OR³, -Y-S(=O)ₚR³, C₃₋₇cycloalkyl, aryl, Het, or C₁₋₆alkyl optionally substituted with C₃₋₇cycloalkyl, aryl, or with Het; wherein any of C₃₋₇cycloalkyl, aryl, Het, and C₁₋₆alkyl, can be substituted with halo, oxo, cyano, azido, nitro, C₁₋₆alkyl, amino, mono- or diC₁₋₆alkylamino, hydroxy, C₁₋₆alkoxy, C₁₋₆alkylcabonyl, aminocarbonyl, mono- or diC₁₋₆alkylaminocarbonyl, C₁₋₆alkylthio, mercapto, carboxyl, C₁₋₆alkoxycarbonyl;
- **aryl**: as a group or part of a group is phenyl, naphthyl, indanyl, or 1,2,3,4-tetrahydronaphthyl, each of which may be optionally substituted with one, two or three substituents selected from halo, C₁₋₆alkyl, polyhaloC₁₋₆alkyl, hydroxy, C₁₋₆alkoxy, polyhaloC₁₋₆alkoxy, C₁₋₆alkoxyC₁₋₆alkyl, carboxyl, C₁₋₆alkylcarbonyl, C₁₋₆alkoxycarbonyl, cyano, nitro, amino, mono- or diC₁₋₆alkylamino, aminocarbonyl, mono- or diC₁₋₆alkylaminocarbonyl, azido, mercapto, C₃₋₇cycloalkyl, phenyl, pyridyl, thiazolyl, pyrazolyl, pyrrolidinyl, piperidinyl, piperazinyl, 4-C₁₋₆alkylpiperazinyl, 4-C₁₋₆alkylcarbonyl-piperazinyl, and morpholinyl; wherein the morpholinyl and piperidinyl groups may be optionally substituted with one or with two C₁₋₆alkyl radicals; and the phenyl, pyridyl, thiazolyl, pyrazolyl groups may be optionally substituted with 1, 2 or 3 substituents each independently selected from C₁₋₆alkyl, C₁₋₆alkoxy, halo, amino, mono- or diC₁₋₆alkylamino;
- **Het**: as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, C₁₋₆alkyl, polyhalo-C₁₋₆alkyl, hydroxy, C₁₋₆alkoxy, polyhaloC₁₋₆alkoxy, C₁₋₆alkoxyC₁₋₆alkyl, carboxyl, C₁₋₆alkylcarbonyl, C₁₋₆alkoxycarbonyl, cyano, nitro, mercapto, amino, mono- or diC₁₋₆alkylamino, aminocarbonyl, mono- or diC₁₋₆alkylaminocarbonyl, C₃₋₇cycloalkyl, phenyl, pyridyl, thiazolyl, pyrazolyl, pyrrolidinyl, piperidinyl, piperazinyl, 4-C₁₋₆alkylpiperazinyl, 4-C₁₋₆alkylcarbonyl-piperazinyl, and morpholinyl; wherein the morpholinyl and piperidinyl groups may be optionally substituted with one or with two C₁₋₆alkyl radicals; and the phenyl, pyridyl, thiazolyl, pyrazolyl groups may be optionally substituted with 1, 2 or 3 substituents each independently selected from C₁₋₆alkyl, C₁₋₆alkoxy, halo, amino, mono- or diC₁₋₆alkylamino;
- **(N)Het**: as a group or part of a group is a 3 to 7 membered saturated, partially unsaturated or completely unsaturated heterocyclic ring containing 1 to 4 heteroatoms each independently selected from nitrogen, oxygen and sulfur, of which at least one is nitrogen, which nitrogen is linked to the group -CO-CO- in radical A; said heterocyclic ring being optionally condensed with a benzene ring; and wherein the group (N)Het as a whole may be optionally substituted with one, two or three substituents each independently selected from the group of substituents specified in the definition of Het.

Without wishing to be bound by theory, or the ascription of tentative binding modes for specific variables, the notional concepts P1, P2, P3 and P4 as used herein are provided for convenience only and have substantially their conventional meanings, as illustrated by Schechter & Berger, (1976) Biochem Biophys Res Comm 27 157-162, and denote those portions of the inhibitor believed to fill the S1, S2, S3 and S4 subsites, respectively, of the enzyme, where S1 is adjacent the cleavage site and S4 remote from the cleavage site. Regardless of binding mode, the compounds defined by Formula I and subsets thereof are intended to be within the scope of the invention. For example it is expected that capping group R¹⁶-G may interact with the S3 and S4 subsites especially when m and/or n is 0.

Embodiments of the invention can be represented as R¹⁶-G-P4-P3-P2-P1, wherein P3 and/or P4 may be absent. P1, P3 and P4 each represent a building block constituted of a derivative of a natural or unnatural amino acid, P2 is a substituted carbocyclic residue and G-R¹⁶ is a capping group. The building blocks are typically linked together by amide bonds, which are reversed relative to each other on each side of the P2 building block in the compounds of the invention.

The compounds and compositions of the invention can be used in methods of medical treatment or prophylaxis of HCV infections in humans. Accordingly, a further aspect of the invention is the use of a compound as defined above in therapy such as the manufacture of a medicament for the prophylaxis or treatment of flavivirus infections in humans or animals. Exemplary flavivirus include BVDV, dengue, and HCV.

In the compounds of the invention the amide bond linking the P2 and P3 together is reversed relative to the amide bond linking the P1 and P2, i.e. the amino acid derivatives, P1 and P3, on each side of the P2 scaffold are both coupled through their amino functions to the acid groups on each side of the P2 moiety. This means that the side chains of the P3 and P4 (including the R¹⁶ cap to the extent this interacts with S3 or S4) point in the opposite direction compared to in a native peptide substrate. Another consequence of the reversed P3 and P4 amino acids is that the side chains of these amino acids are displaced one atom outwardly relative to a native peptide substrate.

Change of direction of the P3 and P4 side chains in this fashion would be expected to favour non-natural D stereochemistries for the pocket filling groups (e.g. side chains) of P3 and/or P4 and/or R¹⁶. Indeed, such compounds are typically highly active and within the scope of the invention. However, it has been surprisingly found that even compounds of the invention bearing L-amino acid side chains at P3 and/or P4 exhibit good activity, notwithstanding that the respective entity must approach the S3 or S4 pocket from a different angle relative to a native peptide substrate. Accordingly L-stereochemistry at R¹¹ and/or R¹⁵ and/or the corresponding configuration at R¹⁶ is favored. The different angle of approach to the S3 and/or S4 pockets also has implications for the ability of the compounds of the invention to avoid resistance patterns exhibited by prior art HCV protease inhibitors which hitherto have all had a conventional peptide backbone of natural or non-natural L-amino acid residues. As with the reverse transcriptase of HIV, the RNA dependent RNA polymerase NS5A of HCV has a very poor proof reading capacity. This in turn means that the HCV polymerase is highly error prone and it is likely that characteristic resistance patterns will arise when HCV antivirals are administered over long periods.

### Description of the Invention

As used in the foregoing and hereinafter, the following definitions apply unless otherwise noted.

The term halo is generic to fluoro, chloro, bromo and iodo, particularly fluoro, chloro and bromo.

The term "polyhaloC₁₋₆alkyl" as a group or part of a group, e.g. in polyhaloC₁₋₆alkoxy, is defined as mono- or polyhalo substituted C₁₋₆alkyl, in particular C₁₋₆alkyl substituted with up to one, two, three, four, five, six, or more halo atoms, such as methyl or ethyl with one or more fluoro atoms, for example, difluoromethyl, trifluoromethyl, trifluoroethyl. Preferred is trifluoromethyl. Also included are perfluoroC₁₋₆alkyl groups, which are C₁₋₆alkyl groups wherein all hydrogen atoms are replaced by fluoro atoms, e.g. pentafluoroethyl. In case more than one halogen atom is attached to an alkyl group within the definition of polyhaloC₁₋₆alkyl, the halogen atoms may be the same or different.

As used herein "C₁₋₄alkyl" as a group or part of a group defines straight or branched chain saturated hydrocarbon radicals having from 1 to 4 carbon atoms such as for example methyl, ethyl, 1-propyl, 2-propyl, 1-butyl, 2-butyl, 2-methyl-1-propyl; "C₁₋₆alkyl" encompasses C₁₋₄alkyl radicals and the higher homologues thereof having 5 or 6 carbon atoms such as, for example, 1-pentyl, 2-pentyl, 3-pentyl, 1-hexyl, 2-hexyl, 2-methyl-1-butyl, 2-methyl-1-pentyl, 2-ethyl-1-butyl, 3-methyl-2-pentyl, and the like. Of interest amongst C₁₋₆alkyl is C₁₋₄alkyl. C₁₋₃alkyl has the same meaning as C₁₋₄alkyl, but is limited to radicals having 1 to 3 carbon atoms. The term "C₂₋₆alkenyl" as a group or part of a group defines straight and branched chained hydrocarbon radicals having saturated carbon-carbon bonds and at least one double bond, and having from 2 to 6 carbon atoms, such as, for example, ethenyl (or vinyl), 1-propenyl, 2-propenyl (or allyl), 1-butenyl, 2-butenyl, 3-butenyl, 2-methyl-2-propenyl, 2-pentenyl, 3-pentenyl, 2-hexenyl, 3-hexenyl, 4-hexenyl, 2-methyl-2-butenyl, 2-methyl-2-pentenyl and the like. Of interest amongst C₂₋₆alkenyl is C₂₋₄alkenyl. The term "C₂₋₆alkynyl" as a group or part of a group defines straight and branched chained hydrocarbon radicals having saturated carbon-carbon bonds and at least one triple bond, and having from 2 to 6 carbon atoms, such as, for example, ethynyl, 1-propynyl, 2-propynyl, 1-butynyl, 2-butynyl, 3-butynyl, 2-pentynyl, 3-pentynyl, 2-hexynyl, 3-hexynyl and the like. Of interest amongst C₂₋₆alkynyl is C₂₋₄alkynyl.

C₃₋₇cycloalkyl includes cyclopropyl, cyclobutyl, cyclopentyl, cyclohexyl and cycloheptyl, as well as the analogs having a double bond, such as cyclopentenyl, cyclohexenyl and cycloheptenyl, wherein the latter may also be designated as "C₃₋₇cycloalkenyl". Of interest amongst C₃₋₇cycloalkenyl is C₅₋₇cycloalkenyl.

C₁₋₆alkanediyl defines bivalent straight and branched chain saturated hydrocarbon radicals having from 1 to 6 carbon atoms such as, for example, methylene, ethylene, 1,3-propanediyl, 1,4-butanediyl, 1,2-propanediyl, 2,3-butanediyl, 1,5-pentanediyl, 1,6-hexanediyl and the like. Of interest amongst C₁₋₆alkanediyl is C₁₋₄alkanediyl.

C₁₋₆alkoxy means C₁₋₆alkyloxy wherein C₁₋₆alkyl is as defined above.

The term "U is absent" means that the group -C(=U)- which is linked to -T- is methylene (-CH₂-).

The term "which chain is optionally interrupted by one to three heteroatoms" means that one, two or three carbon atoms in said chain could be replaced by O, S or NR¹². Preferably, if more than one carbon is replaced, the replaced carbon is not adjacent to a heteroatom.

"Saturated cyclic amino" encompasses pyrrolidinyl, piperidinyl, morpholinyl, piperazinyl, 4-C₁₋₆alkyl-piperazinyl, 4-C₁₋₅alkylcarbonyl-piperazinyl, and the like.

As used herein, the term (=O) or oxo refers to a carbonyl moiety when attached to a carbon atom, a sulfoxide moiety when attached to a sulfur atom and a sulfonyl moiety when two of said terms are attached to a sulfur atom. Whenever a ring or ring system is substituted with an oxo group, the carbon atom to which the oxo is linked is a saturated carbon.

The bivalent radical W can be -O-CO-, -O-C(=O)-NH-; these bivalent radicals in particular are linked to the nitrogen-containing ring by their oxygen atom.

The radical Het is a heterocycle as specified in this specification and claims. Examples of Het comprise, for example, pyrrolidinyl, piperidinyl, morpholinyl, piperazinyl (in particular 4-substituted piperazinyl such as 4-C₁₋₆alkylpiperazinyl, 4-C₁₋₆alkyl-carbonylpiperazinyl, 4-C₁₋₆alkylcarbonyloxy-piperazinyl, e.g. 4-methylpiperazinyl or 4-methylcarbonylpiperazinyl), pyrrolyl, pyrazolyl, imidazolyl, oxazolyl, isoxazolyl, thiazinolyl, isothiazinolyl, thiazolyl, isothiazolyl, oxadiazolyl, thiadiazolyl, triazolyl (including 1,2,3-triazolyl, 1,2,4-triazolyl), tetrazolyl, furanyl, thienyl, pyridyl, pyrimidyl, pyridazinyl, triazinyl, or any of such heterocycles condensed with a benzene ring, such as indolyl, indazolyl (in particular 1H-indazolyl), indolinyl, quinolinyl, tetrahydroquinolinyl (in particular 1,2,3,4-tetrahydroquinolinyl), isoquinolinyl, tetrahydroisoquinolinyl (in particular 1,2,3,4-tetrahydroisoquinolinyl), quinazolinyl, quinoxalinyl, cinnolinyl, phthalazinyl, benzimidazolyl, benzoxazolyl, benzisoxazolyl, benzothiazinolyl, benzisothiazinolyl, benzothiazolyl, benzoxadiazolyl, benzothiadiazolyl, benzo-1,2,3-triazolyl, benzo-1,2,4-triazolyl, benzotetrazolyl, benzofuranyl, benzothienyl, benzopyrazolyl, and the like. Of interest amongst the Het radicals are those which are non-saturated, in particular those having an aromatic character. Of further interest are those Het radicals that are monocyclic.

The radical (N)Het comprises any of the heterocycles of the previous paragraph having a ring nitrogen. (N)Het in particular is pyrrolidinyl, piperidinyl, morpholinyl, piperazinyl (in particular 4-substituted piperazinyl such as 4-C₁₋₆alkylpiperazinyl, 4-C₁₋₆alkyl-carbonylpiperazinyl, 4-C₁₋₆alkylcarbonyloxy-piperazinyl, e.g. 4-methylpiperazinyl or 4-methylcarbonylpiperazinyl).

Each of the Het or R⁸ radicals mentioned in the previous and the following paragraph may be optionally substituted with the number and kind of substituents mentioned in the definitions of the compounds of formula I or any of the subgroups of compounds of formula I. Some of the Het radicals mentioned in this and the following paragraph may be substituted with one, two or three hydroxy substituents. Such hydroxy substituted rings may occur as their tautomeric forms bearing keto groups. For example a 3-hydroxypyridazine moiety can occur in its tautomeric form 2*H* pyridazin-3-one. Examples of keto-substituted Het radicals are 1,3-dihydro-benzimidazol-2-one, 1,3-dihydro-indol-2-one, 1*H*-indole-2,3-dione, 1*H*-benzo[*d*]isoxazole, 1*H*-benzo[*d*]-isothiazole, 1*H*-quinolin-2-one, 1*H*-quinolin-4-one, 1*H*-quinazolin-4-one, 9*H*-carbazole, and 1*H*-quinazolin-2-one. Where Het is piperazinyl, it preferably is substituted in its 4-position by a substituent linked to the 4-nitrogen with a carbon atom, e.g. 4-C₁₋₆alkyl, 4-polyhaloC₁₋₆alkyl, C₁₋₆alkoxyC₁₋₆alkyl, C₁₋₆alkylcarbonyl, C₃₋₇cycloalkyl.

R⁸ can be as specified above or in particular can be a saturated, a partially unsaturated or completely unsaturated 5 or 6 membered monocyclic or 9 to 12 membered bicyclic heterocyclic ring system as specified in this specification and claims. Examples of said monocyclic or bicyclic ring system comprise any of the rings mentioned in the above paragraph as examples of the radical Het and additionally any of the monocyclic heterocycles mentioned in the above paragraph that are condensed with pyridyl or pyrimidinyl such as, for example, pyrrolopyridine (in particular 1*H*-pyrrolo[2,3-*b*]-pyridine, 1*H*-pyrrolo[2,3-*c*]pyridine), naphtyridine (in particular 1,8-naphtyridine), imidazopyridine (in particular 1*H*-imidazo[4,5-*c*]pyridine, 1*H*-imidazo[4,5-*b*]pyridine), pyridopyrimidine, purine (in particular 7*H*-purine) and the like.

Interesting Het radicals comprise, for example pyrrolidinyl, piperidinyl, morpholinyl, piperazinyl, pyrrolyl, pyrazolyl, imidazolyl, oxazolyl, isoxazolyl, thiazolyl, isothiazolyl, oxadiazolyl, thiadiazolyl, triazolyl (including 1,2,3-triazolyl, 1,2,4-triazolyl), tetrazolyl, furanyl, thienyl, pyridyl, pyrimidyl, pyridazinyl, pyrazolyl, triazinyl, or any of such heterocycles condensed with a benzene ring, such as indolyl, indazolyl (in particular 1H-indazolyl), indolinyl, quinolinyl, tetrahydroquinolinyl (in particular 1,2,3,4-tetrahydroquinolinyl), isoquinolinyl, tetrahydroisoquinolinyl (in particular 1,2,3,4-tetrahydroisoquinolinyl), quinazolinyl, phthalazinyl, benzimidazolyl, benzoxazolyl, benzisoxazolyl, benzothiazolyl, benzoxadiazolyl, benzothiadiazolyl, benzofuranyl, benzothienyl.

The Het radicals pyrrolidinyl, piperidinyl, morpholinyl, piperazinyl, 4-substituted piperazinyl preferably are linked via their nitrogen atom (i.e. 1-pyrrolidinyl, 1-piperidinyl, 4-morpholinyl, 1-piperazinyl, 4-substituted 1-piperazinyl).

The radicals Het or (N)Het may be optionally substituted with the substituents mentioned above or in particular they each may be optionally substituted with one, two or three substituents each independently selected from the group consisting of halo, C₁₋₆alkyl, polyhalo-C₁₋₆alkyl, hydroxy, C₁₋₆alkoxy, polyhaloC₁₋₆alkoxy, C₁₋₆alkoxy-C₁₋₆alkyl, carboxyl, C₁₋₆alkylcarbonyl, C₁₋₆alkoxycarbonyl, cyano, nitro, amino, mono- or diC₁₋₆alkylamino, aminocarbonyl, and mono- and di C₁₋₆alkylaminocarbonyl.

The radical positions on any molecular moiety in the definitions may be at any position on such moiety as long as it is chemically stable. Radicals used in the definitions of the variables include all possible isomers unless otherwise indicated. For instance pyridyl includes 2-pyridyl, 3-pyridyl and 4-pyridyl; pentyl includes 1-pentyl, 2-pentyl and 3-pentyl. When any variable occurs more than one time in any constituent, each definition is independent.

Whenever used hereinafter, the term "compounds of formula I", or "the present compounds" or similar terms, is meant to include the compounds of formula I, the salts and stereoisomeric forms thereof.

The compounds of formula I have several centers of chirality and exist as stereochemically isomeric forms. The term "stereochemically isomeric forms" as used herein defines all the possible compounds made up of the same atoms bonded by the same sequence of bonds but having different three-dimensional structures which are not interchangeable, which the compounds of formula I may possess. With reference to the instances where (*R*) or (*S*) are used to designate the absolute configuration of a chiral atom within a substituent, the designation is done taking into consideration the whole compound and not the substituent in isolation.

Unless otherwise mentioned or indicated, the chemical designation of a compound encompasses the mixture of all possible stereochemically isomeric forms, which said compound might possess. Said mixture may contain all diastereomers and/or enantiomers of the basic molecular structure of said compound. All stereochemically isomeric forms of the compounds of the present invention both in pure form or mixed with each other are intended to be embraced within the scope of the present invention.

Pure stereoisomeric forms of the compounds and intermediates as mentioned herein are defined as isomers substantially free of other enantiomeric or diastereomeric forms of the same basic molecular structure of said compounds or intermediates. In particular, the term "stereoisomerically pure" concerns compounds or intermediates having a stereoisomeric excess of at least 80% (i.e. minimum 80% of one isomer and maximum 20% of the other possible isomers) up to a stereoisomeric excess of 100% (i.e. 100% of one isomer and none of the other), more in particular, compounds or intermediates having a stereoisomeric excess of 90% up to 100%, even more in particular having a stereoisomeric excess of 94% up to 100% and most in particular having a stereoisomeric excess of 97% up to 100%. The terms "enantiomerically pure" and "diastereomerically pure" should be understood in a similar way, but then with regard to the enantiomeric excess, and the diastereomeric excess, respectively, of the mixture in question.

Pure stereoisomeric forms of the compounds and intermediates of this invention may be obtained by the application of art-known procedures. For instance, enantiomers may be separated from each other by the selective crystallization of their diastereomeric salts with optically active acids or bases. Examples thereof are tartaric acid, dibenzoyltartaric acid, ditoluoyltartaric acid and camphosulfonic acid. Alternatively, enantiomers may be separated by chromatographic techniques using chiral stationary phases. Said pure stereochemically isomeric forms may also be derived from the corresponding pure stereochemically isomeric forms of the appropriate starting materials, provided that the reaction occurs stereospecifically. Preferably, when a specific stereoisomer is desired, said compound will be synthesized by stereospecific methods of preparation. These methods will advantageously employ enantiomerically pure starting materials. The diastereomeric racemates of the compounds of formula I can be obtained separately by conventional methods. Appropriate physical separation methods that may advantageously be employed are, for example, selective crystallization and chromatography, e.g. column chromatography.

For some of the compounds of formula I, the salts and the intermediates used in the preparation thereof, the absolute stereochemical configuration is not experimentally determined. A person skilled in the art is able to determine the absolute configuration of such compounds using art-known methods such as, for example, X-ray diffraction.

The present invention is also includes all isotopes of atoms occurring in the present compounds. By way of general example and without limitation, isotopes of hydrogen include tritium and deuterium. Isotopes of carbon include C-13 and C-14.

For therapeutic use, salts of the compounds of formula I are those wherein the counterion is pharmaceutically acceptable. However, salts of acids and bases that are non-pharmaceutically acceptable may also find use, for example, in the preparation or purification of a pharmaceutically acceptable compound. All salts, whether pharmaceutically acceptable or not are included within the ambit of the present invention.

The pharmaceutically acceptable acid and base addition salts as mentioned hereinabove are meant to comprise the therapeutically active non-toxic acid and base addition salt forms which the compounds of formula I are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxy-butanedioic acid), tartaric, citric, methanesulfonic, ethanesulfonic, benzenesulfonic, *p*-toluenesulfonic, cyclamic, salicylic, *p*-aminosalicylic, pamoic and the like acids. Conversely said salt forms can be converted by treatment with an appropriate base into the free base form.

The compounds of formula I containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, *N*-methyl-D-glucamine, hydrabamine salts, and salts with amino acids such as, for example, arginine, lysine and the like.

The term addition salt as used hereinabove also comprises the solvates, which the compounds of formula I, as well as the salts thereof, are able to form. Such solvates are for example hydrates, alcoholates and the like.

Some of the compounds of formula I may also exist in tautomeric forms. Such forms although not explicitly indicated in the above formula are intended to be included within the scope of the present invention.

Embodiments of the invention concern those compounds of formula I or any of the subgroups of compounds of formula I, wherein
R⁶ is aryl, C₃₋₇cycloalkyl, or C₁₋₆alkyl optionally substituted with aryl, C₃₋₇cycloalkyl, or with Het; or
two R⁶ groups, which are bound to the same nitrogen atom, form together with that nitrogen a 3- to 7-membered nitrogen-containing heterocyclic ring; and furthermore
R⁶ is optionally substituted with up to three substituents selected from halogen, -O-R⁴, nitro, cyano, polyhaloC₁₋₆alkyl, polyhaloC₁₋₆alkoxy, oxo, thioxo, =N(R⁴), =N(OR⁴), -N(R⁴)₂, -SR⁴, -SOR⁴, -SO₂R⁴, -SO₂N(R⁴)₂, -C(O)R⁴, -C(O)C(O)R⁴, -C(O)C(O)-OR⁴, -C(O)C(O)N(R⁴)₂, -C(O)CH₂C(O)R⁴, -C(O)OR⁴, -OC(O)R⁴, -C(O)N(R⁴)₂, -OC(O)N(R⁴)₂, -N(R⁴)N(R⁴)COR⁴, -N(R⁴)N(R⁴)C(O)OR⁴, -N(R⁴)N(R⁴)CON(R⁴)₂, -N(R⁴)SO₂R⁴ -N(R⁴)SO₂N(R⁴)₂, -N(R⁴)C(O)OR⁴, -N(R⁴)C(O)R⁴, -N(R⁴)C(O)N(R⁴)₂, -N(COR⁴)COR⁴, -N(OR⁴)R⁴, -C(=NH)N(R⁴)₂, -C(O)N(OR⁴)R⁴, -C(=NOR⁴)R⁴; wherein
- each **R⁴**: is independently selected from hydrogen, aryl, C₃₋₇cycloalkyl, or C₁₋₆-alkyl optionally substituted with aryl, C₃₋₇cycloalkyl, or with Het.

In one embodiment, the -NR⁶R⁶ group in radical A of the compounds of formula I is cyclopropylamino or a radical of formula: 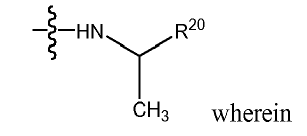
- **R²⁰**: is C₁₋₄alkyl, C₁₋₄alkenyl, aryl; aryl in particular is phenyl optionally substituted with one or two halogen atoms; specifically
- **R²⁰**: is ethyl, isopropyl, vinyl, 4-fluorophenyl, phenyl, 4-pyridyl, 4-chlorophenyl, 2,4-dichlorophenyl, 2,4-difluorophenyl, 3,4-dichlorophenyl, 3,4-difluorophenyl;
or in particular R²⁰ is 4-fluorophenyl, phenyl, 4-pyridyl, 4-chlorophenyl, 2,4-dichlorophenyl, 3,4-difluorophenyl;
or more in particular R²⁰ is 4-fluorophenyl, 4-chlorophenyl, 4-pyridyl. Preferred are those compounds wherein the -NR⁶R⁶ group in radical A is cyclopropylamino.

In another embodiment, A in the compounds of formula I is -C(O)-C(O)-R⁶.

Preferably, any of R⁶ are phenyl, pyridyl, C₁₋₆alkyl (in particular C₃₋₆alkyl), C₃₋₇cycloalkyl, hydroxy, C₁₋₆alkoxy, wherein each phenyl or pyridyl may be optionally substituted as specified above and in particular is substituted with 1, 2 or 3 halogen atoms.

Preferably R⁶ is selected from: phenyl, isopropyl, methoxy, hydroxy, cyclopropylamino or a radical of formula 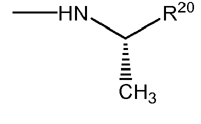 wherein R²⁰ is as defined above.

More preferably, any of R⁶ is isopropyl.

In another embodiment, A in the compounds of formula I is -C(O)-C(O)-OR⁶, wherein R⁶ is as specified above and in particular R⁶ is H or methyl.

In a preferred embodiment, R⁶ is selected from hydrogen, C₁₋₆alkyl, aryl, C₃₋₇cycloalkyl, C₅₋₇cycloalkenyl or Het.

In another embodiment, A in the compounds of formula I is -C(O)-C(O)-N(R⁶)₂ wherein R⁶ is as specified above and in particular R⁶ is selected from hydrogen, C₃₋₇cycloalkyl, or Het. Alternatively, one R⁶ is hydrogen and the other R⁶ is C₁₋₆alkyl (in particular C₁₋₃alkyl), arylC₁₋₆alkyl (in particular arylC₁₋₃alkyl), wherein the C₁₋₆alkyl (or C₁₋₃alkyl) moiety may be optionally substituted with -CO₂H; C₃₋₇cycloalkyl; HetC₁₋₆alkyl (in particular HetC₁₋₃alkyl; and/or wherein Het is a 5-membered ring); C₃₋₆alkenyl; or both R⁶ groups are C₁₋₆alkyl (in particular are C₁₋₃alkyl).

In another embodiment, A in the compounds of formula I is -C(O)-C(O)-R⁶ wherein R⁶ is as specified above and in particular wherein R⁶ is hydrogen, C₁₋₆alkyl, aryl, or C₃₋₇cycloalkylC₁₋₆alkyl (in particular C₃₋₇cycloalkylC₁₋₃alkyl), wherein the C₃₋₇cycloalkyl preferably is a cyclopropyl group. The aryl group is optionally substituted with the number and kind of substituents specified above or in particular with 1, 2 or 3 halogens, which preferably are chloro or fluoro.

In another embodiment, A in the compounds of formula I is -C(O)-C(O)-OR⁶, wherein R⁶ is hydrogen or C₁₋₆alkyl.

In still another embodiment, A in the compounds of formula I is -C(O)-C(O)-N(R⁶)₂ wherein R⁶ is hydrogen, C₁₋₆alkyl, C₂₋₆alkenyl, arylC₁₋₆alkyl or HetC₁₋₆alkyl, wherein R⁶ is optionally substituted with 1, 2 or 3 substituents selected from those mentioned above as possible substituents of R⁶ and in particular are selected from halogen, carboxyl and Het. Preferred substituents on the aryl groups are halogen (preferably chloro or fluoro) and preferred substituents on the alkyl groups are carboxyl and Het.

In another embodiment, A in the compounds of formula I is -CO-CO-R⁶ wherein R⁶ is isopropyl, phenyl, cyclopropylmethyl, hydroxy, methoxy, cyclopropylamino-, or A is -CO-CO-N(R⁶)₂, wherein the -N(R⁶)₂ group in radical A is 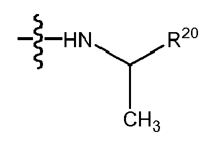 wherein
R²⁰ is ethyl, isopropyl, vinyl, 4-fluorophenyl, phenyl, methyl, 4-pyridyl, 4-chlorophenyl, 2,4-dichlorophenyl, 2,4-difluorophenyl, 3,4-dichlorophenyl, 3,4-difluorophenyl, carboxyl, or wherein the -N(R⁶)₂ group in radical A is 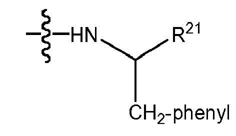 wherein
R²¹ is carboxyl or tetrazolyl (in particular 1H-tetrazol-5-yl).

In another embodiment, A in the compounds of formula I is -C(O)-C(O)-N(R⁶)₂ wherein the two R⁶ groups together with the nitrogen to which they are attached form a ring which can be represented by: 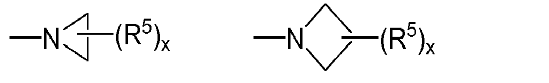 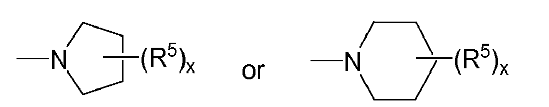 wherein each **x** independently is a number from 0 to 3; each **R**⁵ independently is an optional substituent on the ring, selected from H, halo, C₁₋₆alkyl, polyhalo-C₁₋₆alkyl, hydroxy, C₁₋₆alkoxy, polyhaloC₁₋₆alkoxy, C₁₋₆alkoxyC₁₋₆alkyl, carboxyl, C₁₋₆alkylcarbonyl, cyano, nitro, amino, mono- or diC₁₋₆alkylamino, aminocarbonyl, and mono- or diC₁₋₆alkylaminocarbonyl.

Preferably the two R⁶ groups together with the nitrogen to which they are attached form a ring which can be represented by: 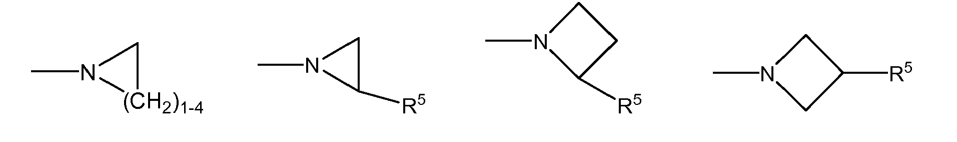 wherein each R⁵ independently is methyl, carboxyl, methoxycarbonyl, t-butoxycarbonyl, aminocarbonyl, mono- or dimethylaminocarbonyl.

In yet another embodiment, A in the compounds of formula I is -C(O)-C(O)-N(R⁶)₂, wherein the two R⁶ groups together with the nitrogen to which they are attached form a ring which can be represented by: 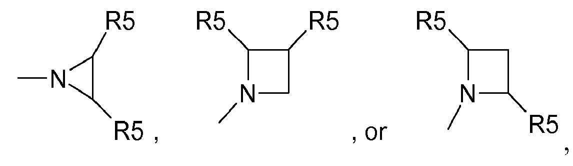 wherein
each R⁵ is independently selected from H, halo, C₁₋₆alkyl, polyhalo-C₁₋₆alkyl, hydroxy, C₁₋₆alkoxy, polyhaloC₁₋₆alkoxy, C₁₋₆alkoxyC₁₋₆alkyl, carboxyl, C₁₋₆alkylcarbonyl, cyano, nitro, amino, mono- or diC₁₋₆alkylamino, aminocarbonyl, and mono- or diC₁₋₆alkylaminocarbonyl.

Preferred values for q' and k in formula I include 1:1, 1:2, 1:3, 2:2, 2:3 (q':k), more preferably 0:2 and 0:0; and most preferably 0:1, in which case the 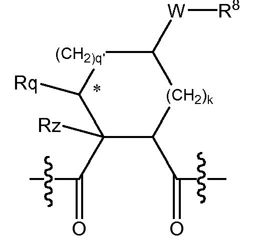 moiety in the compounds of formula I has the structure: 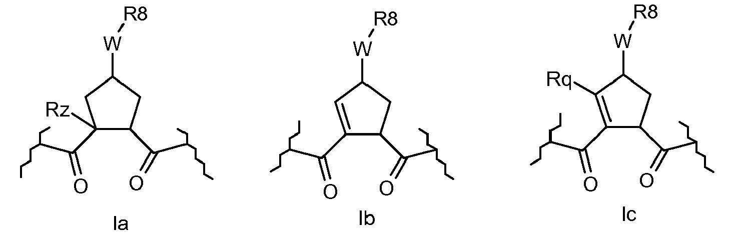 wherein Rz in particular is H, or Rq is H or methyl.

Particularly preferred are compounds with the partial structure Ia.

Embodiments of the invention are those wherein the moiety of formula I, as mentioned above, has the following structure:

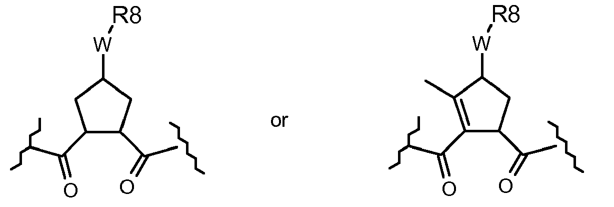

Compounds of the invention may comprise both a P3 and a P4 group, i.e. m and n are each 1. Favored embodiments within Formula I include formula Ida-Idb below:

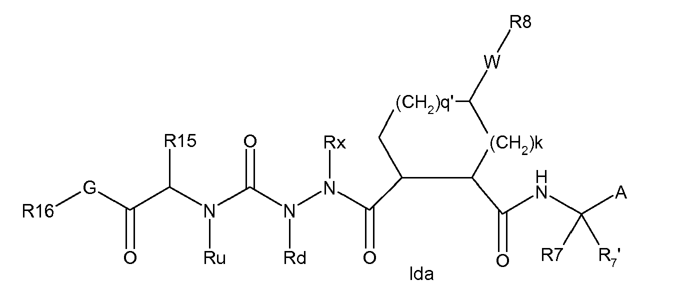

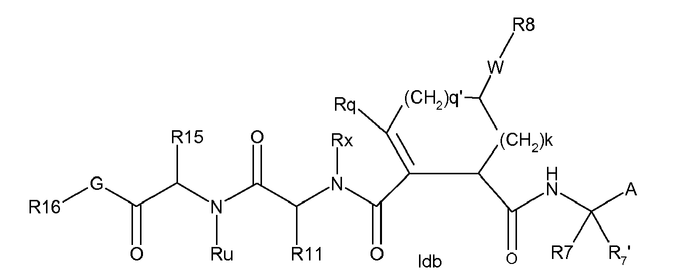

Preferred embodiments are compounds of the formula: 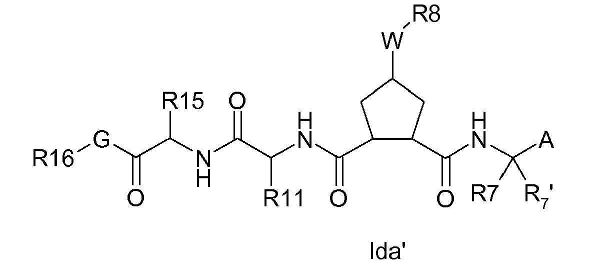 or

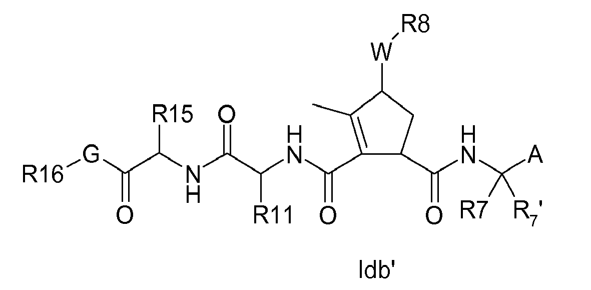

Alternative configurations of the compounds of the invention comprise a P3, but no P4 function, i.e. m is 1 and n is zero. Favoure Embodiments within Formula I include formula Iea-Ieb below:

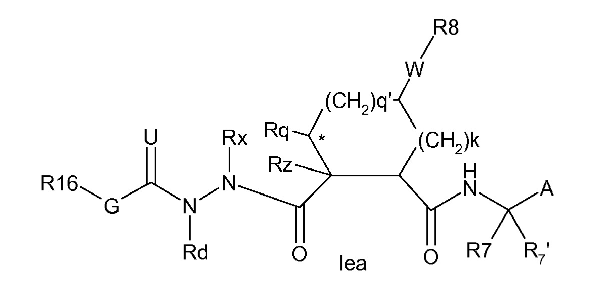

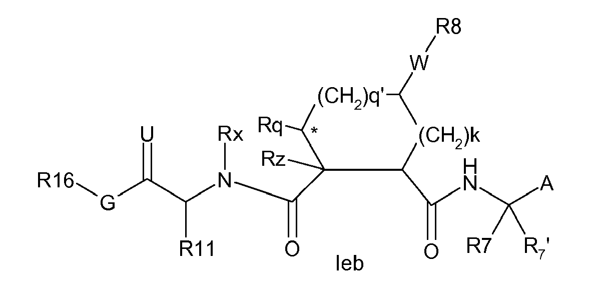

Still further groups of compounds of the invention include those where m and n are zero and thus groups R¹⁶-G abut P2.

Favored embodiments within the compounds of formula I are those wherein m and n are zero include those of formula Ifa below:

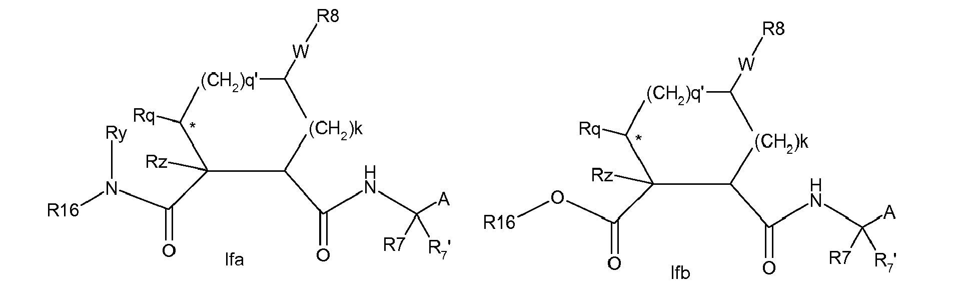

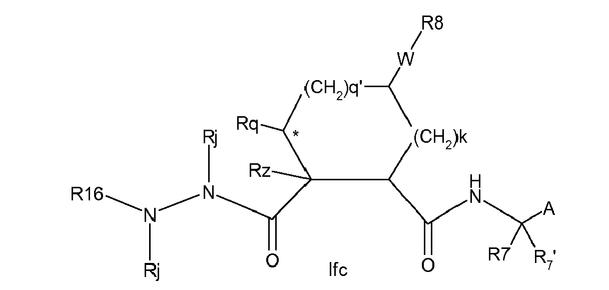

R¹⁶ in compounds Ifa-Ife and elsewhere can be H, C₁₋₆alkyl, C₃₋₇cycloalkyl, aryl, or Het, any of which being optionally substituted, as described above. For example, R¹⁶ can be phenyl substituted as described above.

Alternatively, in embodiments wherein R⁷ and R^{7'} together define a spiro cycloalkyl group, such as spiro-cyclopropyl, the compounds of the invention may be configured as macrocycles, wherein a linking group J extends between one of Rj, Rx, Ry, Rd or R¹¹ of Formula I. Alternatively the macrocycle J may extend from the carbon adjacent to R⁷ to one of Rj , Rx, Ry, Rd or R¹¹.

Embodiments of macrocyclic structures within formula I wherein m is 1 and n is 0 include those of the formula Iga-Igc below:

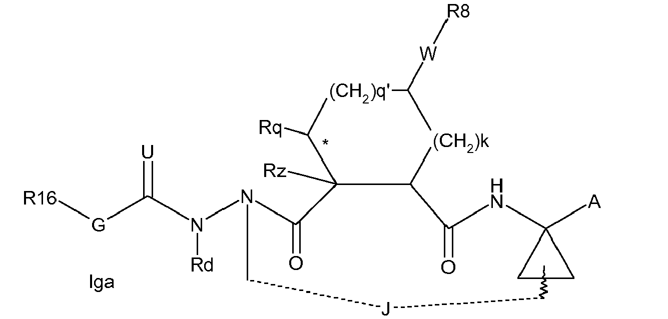

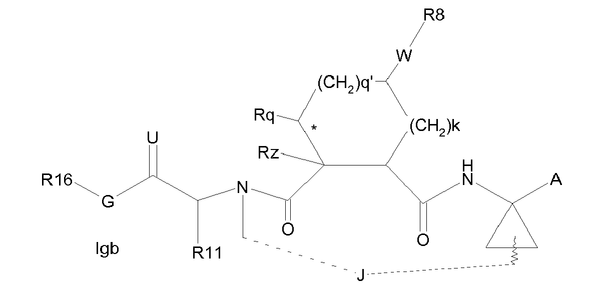

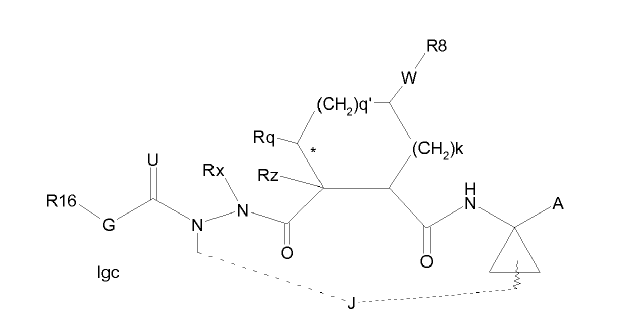

The corresponding structures wherein the J chain bonds to the carbon adjacent to R⁷ are further embodiments of the invention.

Favored embodiments of macrocyclic structures within formula I, comprising both a P3 and P4 group, i.e. wherein both m and n are 1, include those of the formula Iha-Ihc below:

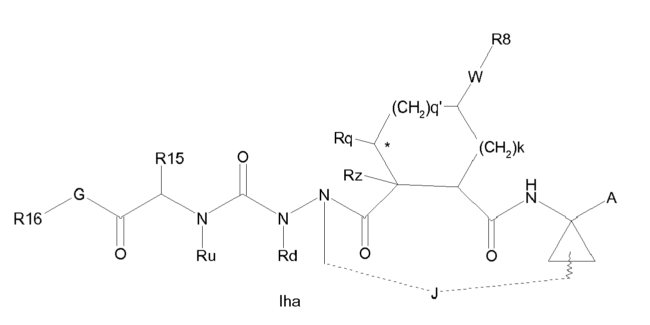

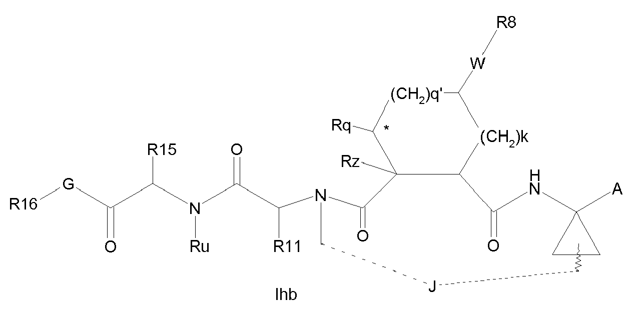

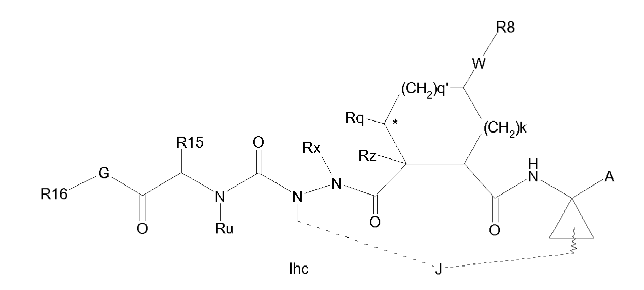

Favored macrocyclic structures within Formula I, wherein both of the P3 and P4 functions are absent, i.e. wherein m and n are each 0, include those of the formulae Ihe-Ihf below. For the sake of completeness, it will be appreciated that the macrocyclic ring and or R¹⁶ may be interacting with P3.

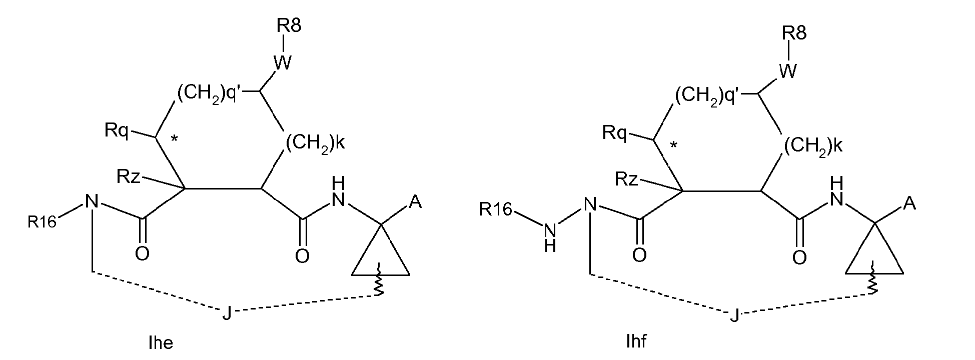

The corresponding structures wherein the J chain bonds to the carbon adjacent to R⁷ are also of interest.

A particular embodiment within formulae Ihe and Ihf are compounds of the formula Ihe'-Ihf : 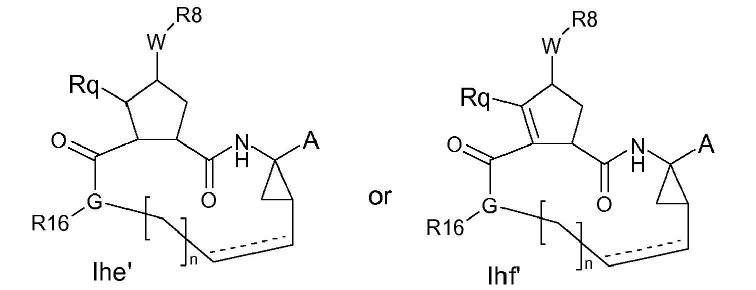 wherein A, W and R⁸ are as defined in the definitions of the compounds of formula I or any of the subgroups of compounds of formula I; G is -NRy- or -NRjRj-, where one Rj is H or C₁₋₆alkyl and the other Rj is H, C₁₋₆alkyl or J; Ry is J; J is as in the above figures; R¹⁶ is H or C₁₋₆alkyl; n is 3,4,5 or 6; the dashed line is an optional double bond.

In preferred embodiments, G is -NRy- and Ry is the J linker. Rq in Ihe' preferably is H; Rq in Ihf' preferably is C₁₋₆alkyl, in particular methyl. Preferably R¹⁶ is H or methyl, isopropyl, isobutyl, and especially H and methyl.

When n is 2, the moiety -CH₂- bracketed by "n" corresponds to ethanediyl in the compounds of formula I or in any subgroup of compounds of formula I. When n is 3, the moiety -CH₂- bracketed by "n" corresponds to propanediyl in the compounds of formula I or in any subgroup of compounds of formula I. When n is 4, the moiety -CH₂- bracketed by "n" corresponds to butanediyl in the compounds of formula I or in any subgroup of compounds of formula I. When n is 5, the moiety-CH₂- bracketed by "n" corresponds to pentanediyl in the compounds of formula I or in any subgroup of compounds of formula I. When n is 6, the moiety -CH₂- bracketed by "n" corresponds to hexanediyl in the compounds of formula I or in any subgroup of compounds of formula I. Particular subgroups of the compounds of formula I are those compounds wherein n is 4 or 5.

The compounds of formula Ihe'-Ihf' have several asymmetric centers. In order to more efficiently refer to each of these asymmetric centers, the numbering system as indicated in the following structural formula will be used.

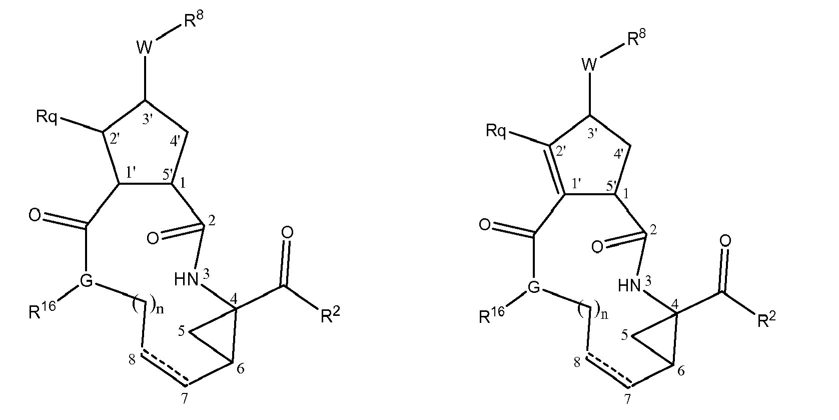

Asymmetric centers are present at positions 1, 4 and 6 of the macrocycle as well as at the carbon atom 3' in the pyrrolidine moiety, or proline residue, bearing the -L-R¹ group. Each of these asymmetric centers can occur in their R or S configuration. The stereochemistry at position 1 preferably corresponds to that of an L-amino acid configuration, i.e. that of L-proline.

The stereochemistry at position 1 preferably corresponds to that of an L-amino acid configuration, i.e. analogous to L-proline, as depicted below:

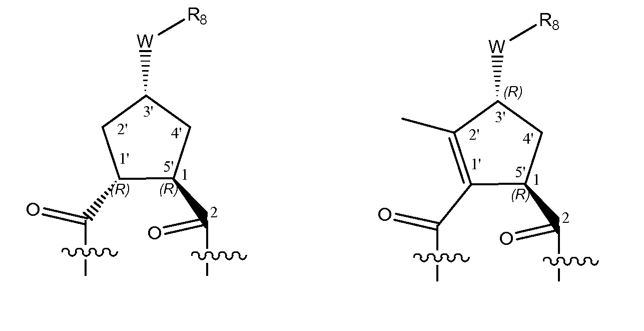

Preferred are the compounds of formula I wherein the substituent at the 1 (or 5') position and the substituent -W-R⁸, for example -O-R⁸ (at position 3') are in a trans configuration. Of particular interest are the compounds of formula I wherein position 1 has the configuration corresponding to L-proline and the -W-R⁸ substituent is in a trans configuration in respect of position 1, as shown in the partial structures immediately above.

The structure of formula I may include a cyclopropyl group as represented in the P1 fragment below: 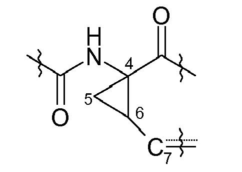 wherein C₇ represents the carbon at position 7 and carbons at position 4 and 6 are asymmetric carbon atoms of the cyclopropane ring.

Notwithstanding other possible asymmetric centers at other segments of the compounds of the invention, the presence of these two asymmetric centers means that the compounds can exist as mixtures of diastereomers, such as the diastereomers of compounds of formula I wherein the carbon at position 7 is configured either syn to the carbonyl or syn to the amide as shown below.

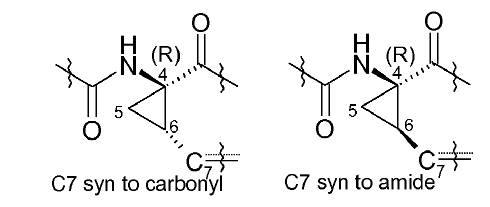

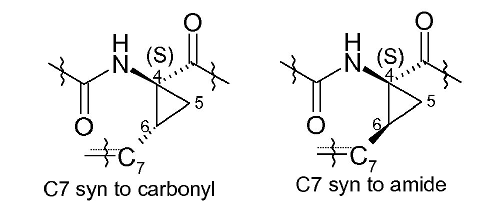

One embodiment concerns compounds of formula I wherein the carbon at position 7 is configured syn to the carbonyl. Another embodiment concerns compounds of formula I wherein the configuration at the carbon at position 4 is R. A specific subgroup of compounds of formula I is that wherein the carbon at position 7 is configured syn to the carbonyl and wherein the configuration at the carbon at position 4 is R.

Preferably the compounds of formula (Ihe' and Ihf') have the stereochemistry as indicated in the structures of formula:

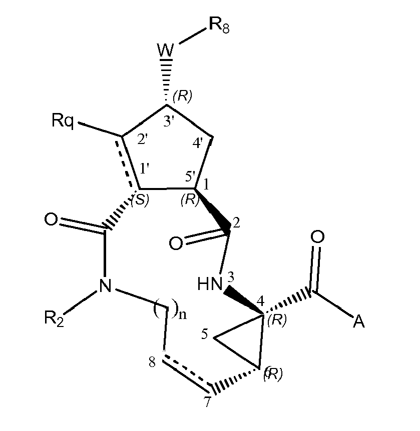

Typically, the stereochemistry of the groups corresponding to the P3 and P4 side chains (ie R¹⁵ and/or R¹¹), if present, will correspond to an L-amino acid configuration, although the invention also extends to D-isomers at one or both of these centers. It is noteworthy that the L configuration is active notwithstanding that the nature of the E moiety means that P3 and P4 are typically translated one atom relative to a conventional polypeptide and the fact that reversal of a peptide residue, as envisaged for P3 and P4 then pitches the amine acid side chain to the opposite side compared to a conventional peptide substrate.

Preferred are the compounds of formula I wherein the substituent at the 1 (or 5') position and the substituent -W-R⁸ (at position 3') are in a trans configuration. Of particular interest are the compounds of formula I, wherein position 1 has the configuration corresponding to L-proline and the -W-R⁸ substituent is in a trans configuration in respect of position 1. Preferably the compounds of formula I have the stereochemistry as indicated in the structure of formula (I-a) or (I-b) as depicted below:

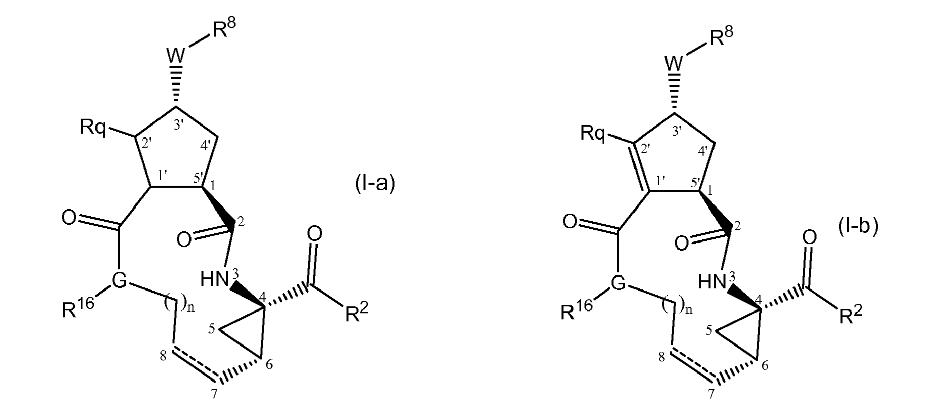

Rq in (I-a) preferably is H; Rq in (I-b) preferably is C₁₋₆alkyl, e.g. methyl. The -CO-G-group in position 1' can be cis or trans towards the -CO-NH- group in position 5' (or position 1); preferably both groups are in cis position.

In general, in the optionally macrocyclic structures such as those illustrated above, linker J is a 3- to 10-atom chain, preferably 5- to 8-atom chain, such as 6- or 7-atom chain, saturated alkylene chain or a partially unsaturated alkylene chain, that is an alkylene chain bearing 1 to 3 unsaturated bonds between adjacent carbons, typically one unsaturation. The length of the chain will, of course, depend on whether J extends from Rd, Rj, Rx, Ry, R¹¹ or the carbon adjacent to R⁷. Suitable chains are described in detail in WO 00/59929. Typically J will be dimensioned to provide a macrocycle of 13 to 16 ring atoms (including those atoms in the P1, P2 and if present P3 groups contributing to the ring). Conveniently, J is dimensioned to provide a macrocycle of 14 or 15 ring atoms.

Conveniently, the J chain contains one or two heteroatoms selected from: O, S, NH, NC₁₋₆alkyl or N-C(=O)C₁₋₆alkyl. More preferably, the J chain optionally contains one heteroatom selected from: NH, or N-C(=O)C₁₋₆alkyl, most preferably N-Acetyl. Most preferably, the chain containing a nitrogen atom is saturated. In an alternative embodiment, J contains one heteroatom selected from O or S. The chain may be substituted with R¹⁴, such as H or methyl.

Typically the J linker structure is saturated. Alternatively, J contains 1 to 3, preferably one double bond, typically spaced one carbon from the cycloalkyl R⁷ function, if present. The double bond may be cis or trans.

Representative examples of J thus include pentylene, hexylene, heptylene, any of which are substituted with C₁₋₆alkyl, C₁₋₆haloalkyl, C₁₋₆alkoxy, hydroxy, halo, amino, oxo, marcapto or C₁₋₇thioalkyl; penten-3-yl, hexen-4-yl, hepten-5-yl, where 3, 4 or 5 refers to a double bond between carbon atoms 3 and 4, 4 and 5, 5 and 6, respectively. Convenient R⁷ and R^{7'} groups include those wherein R^{7'} is H and R⁷ is n-ethyl, n-propyl, cyclopropyl, cyclopropylmethyl, cyclobutyl, cyclobutylmethyl, 2,2-difluoroethyl, or mercaptomethyl. Preferred embodiments include those wherein R⁷ is n-propyl or 2,2-difluoroethyl. Alternative embodiments are those wherein R^{7'} is H and R⁷ is C₃₋₇cycloalkyl or C₁₋₃alkyl substituted with C₃₋₇cycloalkyl, in particular wherein R^{7'} is H and R⁷ is J.

Alternatively, R⁷ and R^{7'} together define a spiro-cycloalkyl function, such as a spiro-cyclobutyl ring, and more preferably a spiro-cyclopropyl ring. "Spiro" in this context simply means that the cycloalkyl ring shares a single carbon atom with the peptidic backbone of the compound. The ring is substituted or unsubstituted. Preferred substituents include mono or di-substitutions with R^{7a} wherein R^{7a} is C₁₋₆alkyl, C₃₋₅cycloalkyl, or C₂₋₆alkenyl, any of which is optionally substituted with halo. Alternatively the substituent may be a J linker as described above. A preferred stereochemistry for a spiro-cyclopropyl ring is as defined below.

Preferred substituents include R^{7'a} groups such as ethyl, vinyl, cyclopropyl (ie a spiro-cyclopropyl substituent to the "spiro" cycloalkyl ring of R⁷/R^{7,}), 1- or 2-bromoethyl, 1-or 2-fluoroethyl, 2-bromovinyl or 2-fluorethyl.

Substituent -W-R⁸ on the cyclic P2 group can be any of the proline substituents described in WO00/59929, WO00/09543, WO00/09558, WO99/07734, WO99/07733, WO02/60926, WO03/35060, WO03/53349, WO03/064416, WO03/66103, WO03/064455, WO03/064456, WO03/62265, WO03/062228, WO03/87092, WO03/99274, WO03/99316, WO03/99274, WO04/03670, WO04/032827, WO04/037855, WO04/43339, WO04/92161, WO04/72243, WO04/93798. WO04/93915, WO04/94452, WO04/10150 WO04/10160 WO04/103996, WO04113365.

W groups of interest include W being -OC(=O)NH-, -OC(=O)-, -NH-, -NR²-, -NHS(O)₂- or -NHC(=O)-, especially -OC(=O)NH- or -NH-. Favored R⁸ groups for such W functions include optionally substituted C₃₋₇cycloalkyl, aryl, Het, or C₁₋₆alkyl optionally substituted with C₃₋₇cycloalkyl, aryl, or with Het, including those described in WO00/09543, WO00/09558 and WO00/174768. For example ester substituents, -W-R⁸ on the cyclic P2 group, include those disclosed in WO01/74768 such as C₁₋₆alkylcarbonyloxy, arylcarbonyloxy, C₁₋₃alkyl substituted with arylcarbonyloxy or Hetcarbonyloxy, particularly (optionally substituted) benzylcarbonyloxy, or Het-carbonyloxy, especially

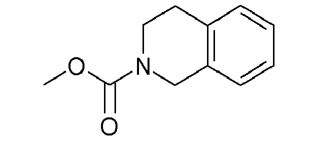

Also included are alternative -W-R⁸ possibilities, for example C₁₋₆alkyl, such as ethyl, isopropyl, C₁₋₃alkyl substituted with C₃₋₇cycloalkyl or with aryl, aryl, C₃₋₇cycloalkyl such as cyclohexyl, or polyhaloC₁₋₆alkyl such as 2,2-difluoroethyl.

Preferred W functions include -S- and especially -O-. Convenient values for R⁸ in such embodiments include aryl, or Het, optionally spaced from W by a C₁₋₃alkylene group; either of which is optionally mono, di, or tri substituted with R⁹, wherein
R⁹ is C₁₋₆alkoxy, nitro, hydroxy, halo, trifluoromethyl, amino or amido (for example amido or amino optionally mono- or di-substituted with C₁₋₆alkyl), carboxyl, aryl, Het, C₁₋₆alkyl optionally substituted with aryl or with Het; wherein the aryl or Het moiety is optionally substituted with one or two R¹⁰; wherein
R¹⁰ is C₁₋₆alkyl, C₃₋₇cycloalkyl, C₁₋₆alkoxy, amino (including substituted amino such as amino mono- or di-substituted with C₁₋₆alkyl or saturated cyclic amino), amido, sulfonylC₁₋₃alkyl, nitro, hydroxy, halo, trifluoromethyl, or carboxyl.

Preferred R⁹ include C₁₋₆alkyl, C₁₋₆alkoxy, amino, (as well as substituted amino such as mono- or di-C₁₋₆alkylamino or saturated cyclic amino), amido (such as -NHC(=O)C₁₋₆alkyl or -C(=O)NHC₁₋₆alkyl), aryl or Het, the aryl or Het being optionally substituted with one R¹⁰; wherein
R¹⁰ is C₁₋₆alkyl, C₃-C₇cycloalkyl, C₁₋₆alkoxy, amino (as well as substituted amino such as mono- or di-C₁₋₆ alkylamino or saturated cyclic amino), amido (such as -NHC(=O)C₁₋₃alkyl or -C(=O)NHC₁₋₃alkyl), halo, or trifluoromethyl.

Preferred R¹⁰ include C₁₋₆alkyl, C₁₋₆alkoxy, amino, amido (such as -NHC(=O)C₁₋₆alkyl or -C(=O)NHC₁₋₆alkyl), and halo. Particularly preferred R¹⁰ include methyl, ethyl, isopropyl, tert-butyl, methoxy, chloro, amino, amido (such as -NH-C(=O)C₁₋₆alkyl, for example -NH-C(=O)-C(CH₃)₃, or -C(=O)NHC₁₋₃alkyl).

Embodiments of R⁸ include 1-naphthylmethyl, 2-naphthylmethyl, benzyl, 1-naphthyl, 2-naphthyl, or quinolinyl, any of which is unsubstituted, mono, or disubstituted with R⁹ as defined, in particular 1-naphthylmethyl, or quinolinyl unsubstituted, mono-, or disubstituted with R⁹ as defined.

Further subgroups of the compounds of formula I are those compounds of formula I, or any subgroup of compounds of formula I specified herein, wherein R⁸ is phenyl, naphthyl, pyridyl, pyridazinyl, triazolyl, tetrazolyl, quinolinyl, isoquinolinyl, quinazolinyl, pyrimidinyl, [1,8]naphthyridinyl, indolinyl, 1,2,3,4-tetrahydroquinolinyl, 1,2,3,4-tetrahydroisoquinolinyl; all optionally substituted with one, two or three R⁹ substituents as mentioned in the definitions of the compounds of formula I or any of the subgroups of compounds of formula I.

Other subgroups of the compounds of formula I are those compounds of formula I, or any subgroup of compounds of formula I specified herein, wherein
(a) R⁸ is phenyl, naphthyl (such as naphth-1-yl or naphth-2-yl), quinolinyl (in particular quinolin-4-yl), isoquinolinyl (in particular isoquinolin-1-yl), quinazolinyl (in particular quinazolin-4-yl), pyridyl (in particular 3-pyridyl), pyrimidinyl (in particular pyrimidin-4-yl), pyridazinyl (in particular pyridazin-3-yl and pyridazin-2-yl), [1,8]naphtyridinyl (in particular [1,8]naphthyridin-4-yl);
(b) R⁸ is triazolyl (in particular triazol-1-yl, triazol-2-yl), tetrazolyl (in particular tetrazol-1-yl, tetrazol-2-yl), 6-oxo-pyridazin-1-yl, pyrazolyl (in particular pyrazol-1-yl), or imidazolyl (in particular imidazol-1-yl, imidazol-2-yl);
(c) R⁸ is a heterocycle selected from 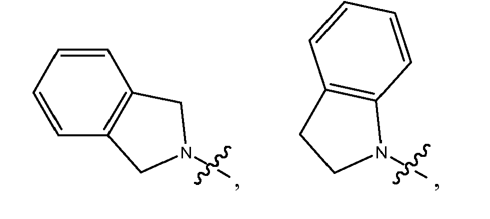 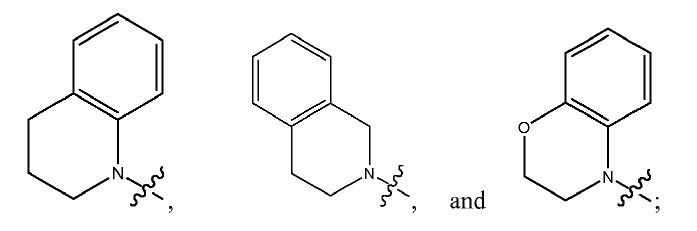
and wherein each of the above mentioned R⁸ radicals may be optionally substituted with one, two or three R⁹ substituents selected from those mentioned in the definitions of the compounds of formula I or any of the subgroups of compounds of formula I.

Embodiments of the invention are compounds of formula I or any of the subgroups of compounds of formula I wherein W is a direct bond, -O-, -O-C(=O)-, or -OC(=O)NH-, or in particular wherein W is -OC(=O)NH- or -O-, or more in particular wherein W is -O-. Preferably W is -O-, and R⁸ is as specified above in (a); or W is a direct bond, and R⁸ is as specified above in (b); or W is a bivalent radical -O-C(=O)-, and R⁸ is as specified above in (c).

Embodiments of the invention are compounds of formula I or any of the subgroups of compounds of formula I wherein W is -O- and R⁸ is quinolinyl (in particular quinolin-4-yl), isoquinolinyl (in particular isoquinolin-1-yl), quinazolinyl (in particular quinazolin-4-yl), or pyrimidinyl (in particular pyrimidin-4-yl), either of which is, independently, optionally mono, di, or tri substituted with C₁₋₆alkyl, C₁₋₆alkoxy, nitro, hydroxy, halo, trifluoromethyl, -NR²R³, -C(=O)NR²R³, C₃₋₇cycloalkyl, aryl, Het, or -C(=O)OR³; wherein aryl or Het are each, independently, optionally substituted with halo, C₁₋₆alkyl, C₁₋₆alkoxy, amino, mono- or diC₁₋₆alkylamino, pyrrolidinyl, piperidinyl, piperazinyl, 4-C₁₋₆alkylpiperazinyl (e.g.4-methylpiperazinyl) or morpholinyl; and wherein the morpholinyl and piperidinyl groups may optionally substituted with one or two C₁₋₆alkyl radicals.

Embodiments of the invention are compounds of formula I or any of the subgroups of compounds of formula I wherein W is -O- and R⁸ is quinolinyl (in particular quinolin-4-yl), isoquinolinyl (in particular isoquinolin-1-yl), quinazolinyl (in particular quinazolin-4-yl), or pyrimidinyl (in particular pyrimidin-4-yl), either of which is, independently, optionally mono, di, or tri substituted with methyl, ethyl, isopropyl, *tert*-butyl, methoxy, trifluoromethyl, trifluoromethoxy, fluoro, chloro, bromo, -NR²R³, -C(=O)NR²R³, phenyl, methoxyphenyl, cyanophenyl, halophenyl, pyridyl, C₁₋₄alkyl-pyridyl, pyrimidinyl, piperidinyl, morpholinyl, piperazinyl, C₁₋₄alkylpiperazinyl, pyrrolidinyl, pyrazolyl, C₁₋₄alkyl-pyrazolyl, thiazolyl, C₁₋₄alkylthiazolyl, cyclopropyl-thiazolyl, or mono- or diC₁₋₄alkyl-aminothiazolyl; and wherein the morpholinyl and piperidinyl groups may optionally substituted with one or two C₁₋₆alkyl (in particular one or two methyl) radicals.

Embodiments of the invention are compounds of formula I or any of the subgroups of compounds of formula I wherein R⁸ is quinolinyl, optionally substituted with 1, 2, 3 or 4 (or with 1, 2 or 3) substituents selected from those mentioned as possible substituents on the monocyclic or bicyclic ring systems of R⁸, as specified in the definitions of R⁹ of the compounds of formula I or of any of the subgroups of compounds of formula I.

Specific embodiments of the invention are those compounds of formula I or any of the subgroups of compounds of formula I wherein R⁸ is
(d-1) a radical of formula 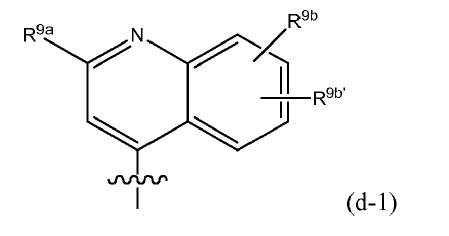
(d-2) a radical of formula 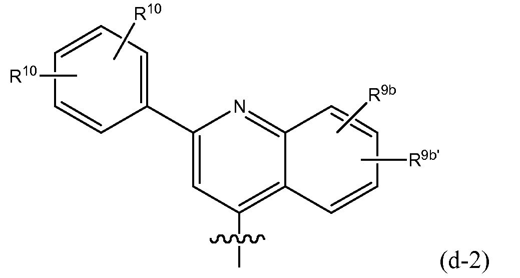
(d-3) a radical of formula 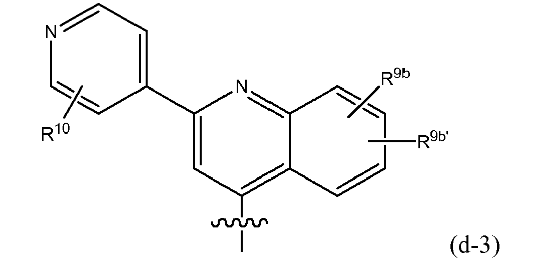
(d-4) a radical of formula 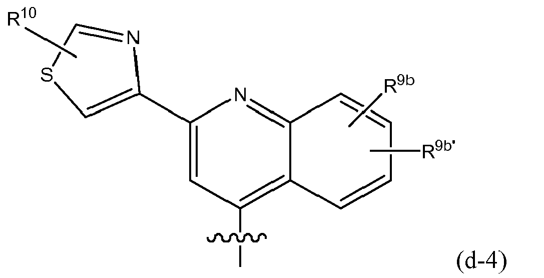 or in particular, (d-4-a) a radical of formula 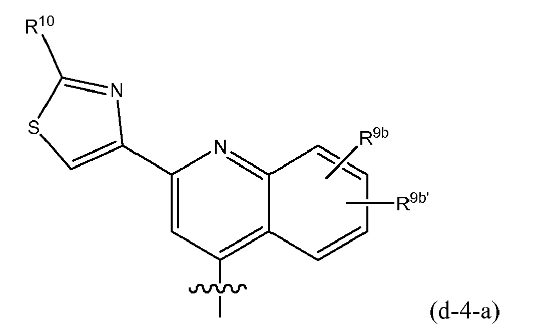
(d-5) a radical of formula 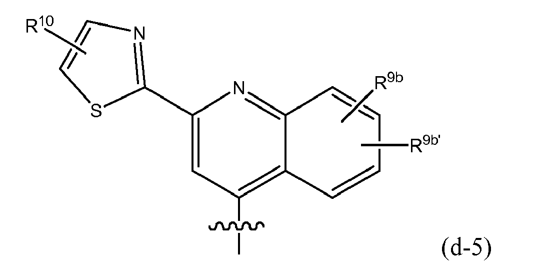 or in particular, (d-5-a) a radical of formula 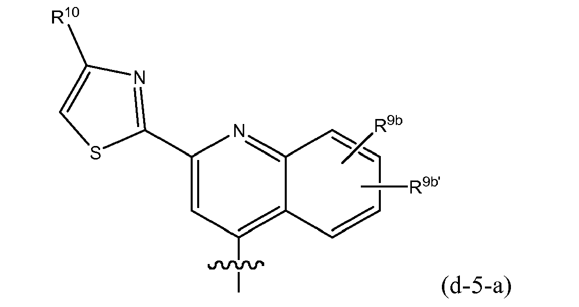
wherein in radicals (d-1) - (d-5), as well as in (d-4-a) and (d-5-a):
each R^{9a}, R^{9b}, R^{9b}' are independently any of the substituents selected from those mentioned as possible substituents on the monocyclic or bicyclic ring systems of R⁸, as specified in the definitions of R⁹ of the compounds of formula I or of any of the subgroups of compounds of formula I; and, R¹⁰ is each independently any of the substituents selected from those mentioned as possible substituents of R⁹, as specified in the definitions of R¹⁰ of the compounds of formula I or of any of the subgroups of compounds of formula I; or, in particular, wherein in radicals (d-1) - (d-5), as well as in (d-4-a) and (d-5-a):
R^{9b} and R^{9b'} may, independently, be hydrogen, C₁₋₆alkyl, C₁₋₆alkoxy, -NR²R³ (in particular amino or mono- or diC₁₋₆alkylamino), -C(=O)NR²R³, (in particular aminocarbonyl or mono- or diC₁₋₆alkylaminocarbonyl), nitro, hydroxy, halo, trifluoromethyl, or -C(=O)OR³ (in particular wherein R³ is C₁₋₆alkyl);
wherein each R², R³ mentioned above or hereinafter independently is as defined in the definitions of the compounds of formula I or of any of the subgroups of compounds of formula I;
or, in particular, wherein in radicals (d-1) - (d-5), as well as in (d-4-a) and (d-5-a):
R^{9a} is hydrogen, C₁₋₆alkyl, C₁₋₆alkoxy, C₁₋₆alkylthio, monoC₁₋₆alkylamino, amino, C₃₋₇cycloalkyl, aryl, or Het;
more specifically R^{9a} is aryl or Het; of interest are embodiments wherein R^{9a} is phenyl, pyridyl, thiazolyl, pyrazolyl, each substituted with R¹⁰ as specified in the definitions of the compounds of formula I or of any of the subgroups of the compounds of formula I;
in specific embodiments said aryl or Het may each, independently, be optionally substituted with C₁₋₆alkyl, C₁₋₆alkoxy, amino, mono- or diC₁₋₆alkylamino, pyrrolidinyl, piperidinyl, morpholinyl, piperazinyl, 4-C₁₋₆alkylpiperazinyl; and wherein the morpholinyl, and piperidinyl groups may optionally be substituted with one or two C₁₋₆alkyl radicals; and in particular
R^{9a} can be a radical Het; wherein Het may inculde pyrrolidinyl, piperidinyl, morpholinyl, piperazinyl, 4-C₁₋₆alkylpiperazinyl; and wherein the morpholinyl and piperidinyl groups may optionally be substituted with one or two C₁₋₆alkyl radicals.

Embodiments of the invention are compounds of formula I or any of the subgroups of compounds of formula I wherein R^{9a} is a radical 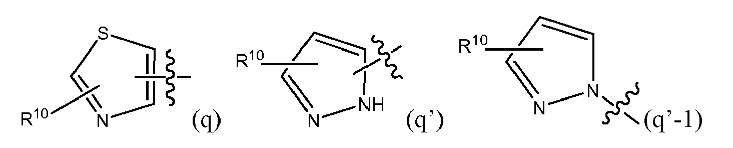 or, in particular, wherein R^{9a} is selected from the group consisting of: 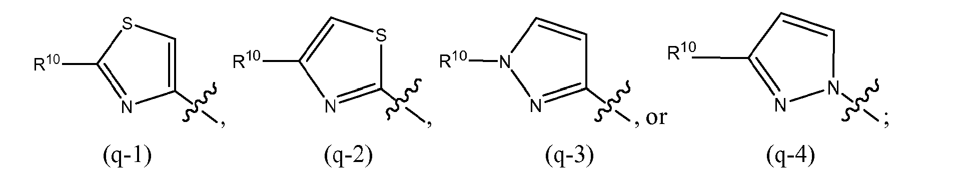 wherein, where possible a nitrogen may bear an R¹⁰ substituent or a link to the remainder of the molecule; each R¹⁰ in any of the R⁹ substituents may be selected from those mentioned as possible substituents on R⁹, as specified in the definitions of R¹⁰ for the compounds of formula I or of any of the subgroups of compounds of formula I; specifically each R¹⁰ may be hydrogen, halo, C₁₋₆alkyl, C₁₋₆alkoxy, polyhaloC₁₋₆alkyl (in particular trifluoromethyl), amino, or mono- or diC₁₋₆alkylamino, aminocarbonyl or mono- or diC₁₋₆alkylaminocarbonyl, nitro, hydroxy, carboxyl, C(=O)OR^{6a} (in particular wherein R^{6a} is C₁₋₆alkyl), phenyl, pyridyl, thiazolyl, pyrazolyl, pyrrolidinyl, piperidinyl, morpholinyl, piperazinyl, 4-C₁₋₆alkylpiperazinyl (in particular 4-methylpiperazinyl); and wherein the morpholinyl and piperidinyl groups may be optionally substituted with one or two C₁₋₆alkyl radicals; and the phenyl, pyridyl, thiazolyl, pyrazolyl groups may be optionally substituted with 1, 2 or 3 (in particular with 1 or 2) substituents each independently selected from C₁₋₆alkyl, C₁₋₆alkoxy, halo, amino, mono- or diC₁₋₆alkylamino;
more specifically each R¹⁰ may be hydrogen, halo, C₁₋₆alkyl, amino, or mono- or di-C₁₋₆alkylamino, pyrrolidinyl, piperidinyl, morpholinyl, piperazinyl, 4-C₁₋₆alkyl-piperazinyl; and wherein the morpholinyl and piperidinyl groups may optionally be substituted with one or two C₁₋₆alkyl radicals;
and where R¹⁰ is substituted on a nitrogen atom, it preferably is a carbon containing substituent that is connected to the nitrogen via a carbon atom or one of its carbon atoms;
specifically each R¹⁰ independently may be hydrogen, C₁₋₆alkyl, C₁₋₆alkoxy, or halo; or more specifically each R¹⁰ in (d-3) may be hydrogen, C₁₋₆alkyl, C₁₋₆alkoxy or halo; specifically R¹⁰ may be hydrogen, C₁₋₆alkyl, amino, mono- or diC₁₋₆alkylamino, pyrrolidinyl, piperidinyl, morpholinyl, piperazinyl, 4-C₁₋₆alkyl-piperazinyl (in particular 4-methylpiperazinyl); and wherein the morpholinyl and piperidinyl groups may optionally be substituted with one or two C₁₋₆alkyl radicals;
preferably each R¹⁰ is C₁₋₆alkoxy, more preferably methoxy;
specifically R¹⁰ may be hydrogen, C₁₋₆alkyl, amino, mono- or diC₁₋₆alkylamino, pyrrolidinyl, piperidinyl, piperazinyl, 4-C₁₋₆alkylpiperazinyl (in particular 4-methylpiperazinyl), or morpholinyl.

Specific embodiments of the invention are compounds of formula I or any of the subgroups of compounds of formula I wherein R⁸ is 7-methoxy-2-phenyl-quinolin-4-yl and W is -O-.

Embodiments of the invention are compounds of formula I or any of the subgroups of compounds of formula I wherein R⁸ is
(e) isoquinolinyl (in particular 1-isoquinolinyl), optionally substituted with 1, 2, 3 or 4 (or with 1, 2 or 3) R⁹ substituents selected from those mentioned as possible substituents on the monocyclic or bicyclic ring systems of R⁸, as specified in the definitions of the compounds of formula I or of any of the subgroups of compounds of formula I.

Specific such embodiments are those wherein R⁸ is
(e-1) a radical of formula: 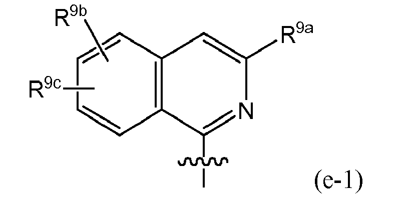 or in particular (e-1-a) a radical of formula: 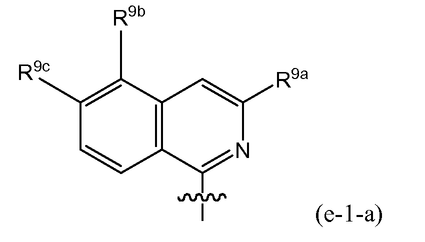 wherein R^{9a}, R^{9b}, R^{9c} independently from one another are any of the substituents selected from those mentioned as possible substituents on the monocyclic or bicyclic ring systems of R⁸, as specified in the definitions of the compounds of formula I or of any of the subgroups of compounds of formula I;
in particular R^{9a} is aryl or Het, either of which is optionally substituted with any of the radicals mentioned as substituents of aryl or of Het as specified in definitions of R¹⁰ for the compounds of formula I or of any of the subgroups of compounds of formula I (including the number of substituents); specifically said aryl or Het may be substituted with 1, 2 or 3 (in particular with one) radical or radicals R¹⁰;
wherein R¹⁰ is any of the radicals mentioned as substituents of aryl or Het as specified in definitions of the compounds of formula I or of any of the subgroups of compounds of formula I as defined above; or in particular R¹⁰ is hydrogen, C₁₋₆alkyl, C₃₋₇cycloalkyl, phenyl, pyridyl, thiazolyl, pyrazolyl, amino optionally mono or disubstituted with C₁₋₆alkyl, or aminocarbonyl or mono- or diC₁₋₆alkylaminocarbonyl, pyrrolidinyl, piperidinyl, piperazinyl, 4-C₁₋₆alkylpiperazinyl (e.g. 4-methylpiperazinyl) or morpholinyl; and wherein the morpholinyl or piperidinyl groups may optionally be substituted with one or two C₁₋₆alkyl radicals; and the phenyl, pyridyl, thiazolyl, pyrazolyl groups may be optionally substituted with 1,2 or 3 (in particular with 1 or 2) substituents each independently selected from C₁₋₆alkyl, C₁₋₆alkoxy, halo, amino, mono- or diC₁₋₆alkylamino; R^{9b} is preferably hydrogen, C₁₋₆alkyl, C₃₋₇cycloalkyl, aryl, Het, halo (e.g. bromo, chloro or fluoro); R^{9c} is preferably hydrogen or C₁₋₆alkoxy.

In particular R^{9a} in the isoquinolinyl radical specified under (e-1) or (e-1-a) includes phenyl, pyridyl, thiazolyl, oxazolyl or pyrazolyl either of which is optionally substituted with R¹⁰ as defined above, in particular optionally substituted with an R¹⁰ which may be hydrogen, C₁₋₆alkyl (e.g. methyl, ethyl, isopropyl, tert-butyl), amino, pyrrolidinyl, piperidinyl, piperazinyl, 4-C₁₋₆alkylpiperazinyl (e.g. 4-methylpiperazinyl) or morpholinyl, C₁₋₆alkylamino, (C₁₋₆alkyl)₂amino , aminocarbonyl, or mono- or diC₁₋₆alkylaminocarbonyl; and wherein the morpholinyl and piperidinyl groups may optionally be substituted with one or two C₁₋₆alkyl radicals.

Preferably R^{9a} in the isoquinolinyl radical specified under (e-1) or (e-1-a) includes any of the radicals (q), (q'), (q'-1), (q-1), (q-2), (q-3), (q-4) specified above as well as: 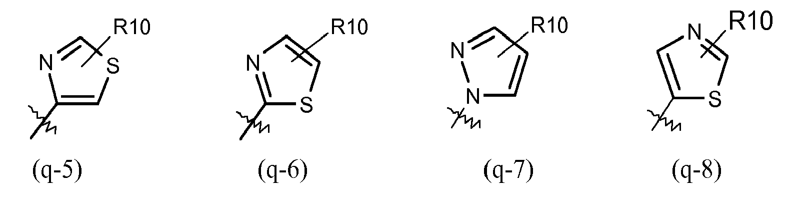 wherein each R¹⁰ is any of the radicals mentioned as substituents of Het as specified in the definitions of the compounds of formula I or of any of the subgroups of compounds of formula I; or in particular R¹⁰ is as defined above; especially R¹⁰ is hydrogen, C₁₋₆alkyl (e.g. methyl, ethyl, isopropyl, tert-butyl), amino, pyrrolidinyl, piperidinyl, piperazinyl, 4-C₁₋₆alkylpiperazinyl (e.g. 4-methylpiperazinyl), or morpholinyl; C₁₋₆alkylamino, (C₁₋₆alkyl)₂amino, aminocarbonyl, or mono- or diC₁₋₆alkylaminocarbonyl; and wherein the morpholinyl and piperidinyl may optionally be substituted with one or two C₁₋₆alkyl radicals.

Also preferably R^{9a} in the isoquinolinyl radical specified under (e-1) or (e-1-a) includes: 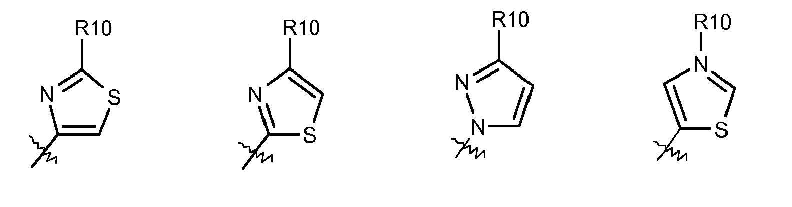 wherein each R¹⁰ is as defined above, and especially is hydrogen, halo, C₁-₆alkyl (e.g. methyl, ethyl, isopropyl, tert-butyl), amino, pyrrolidinyl, piperidinyl, piperazinyl, 4-C₁₋₆alkylpiperazinyl (e.g. 4-methylpiperazinyl) or morpholinyl; C₁₋₆alkylamino, (C₁₋₆alkyl)₂amino, aminocarbonyl, or mono- or diC₁₋₆alkylamino-carbonyl;
and wherein the morpholinyl and piperidinyl groups may optionally be substituted with one or two C₁₋₆alkyl radicals.

R^{9b} in the isoquinolinyl radical specified under (e-2) may be hydrogen, C₁₋₆alkyl, halo (e.g. bromo, chloro or fluoro), especially hydrogen or bromo. R^{9b} in the isoquinolinyl radical specified under (e-2) may be hydrogen or C₁₋₆alkoxy (e.g. methoxy).

Embodiments of the invention are compounds of formula I or any of the subgroups of compounds of formula I wherein R⁸ is 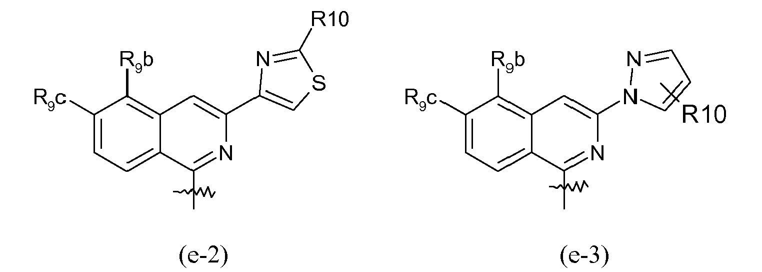 wherein R^{9b} is hydrogen or halo (e.g. bromo) and R^{9c} is hydrogen or C₁₋₆alkoxy (e.g. methoxy).

Embodiments of the invention are compounds of formula I or any of the subgroups of compounds of formula I wherein R⁸ is
(f) quinazolinyl (in particular quinazolin-4-yl), optionally substituted with 1, 2, 3 or 4 (or with 1, 2 or 3) substituents selected from those mentioned as possible substituents on the monocyclic or bicyclic ring systems of R⁸, as specified in the definitions of R⁹ of the compounds of formula I or of any of the subgroups of compounds of formula I. Quinazoline embodiments of R⁸ include
(f-1) a radical : 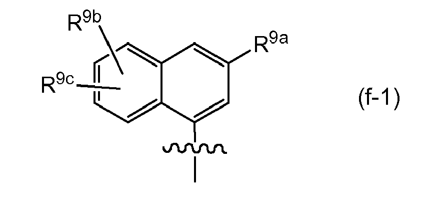 or in particular (f-1-a) a radical : 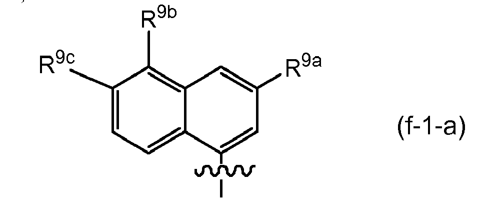 wherein R^{9a}, R^{9b} and R^{9c} have the meanings stated above in relation to R⁸ being isoquinolinyl (such as in radicals (e-1), (e-1-a), etc ); and wherein specifically R^{9a} is C₃₋₇cycloalkyl, aryl or Het, any of which is optionally substituted with one, two or three (in particular with one) R¹⁰;
wherein R¹⁰ is hydrogen, C₁₋₆alkyl, C₃₋₇cycloalkyl, phenyl, pyridyl, thiazolyl, pyrazolyl, pyrrolidinyl, piperidinyl, piperazinyl, 4-methylpiperazinyl or morpholinyl, aminocarbonyl, mono or di C₁₋₆alkylaminocarbonyl; wherein the piperidinyl, morpholinyl may be optionally substituted with one or two C₁₋₆alkyl radicals; and the phenyl, pyridyl, thiazolyl, pyrazolyl groups may be optionally substituted with 1, 2 or 3 (or with 1 or 2) substituents each independently selected from C₁₋₆alkyl, C₁₋₆alkoxy, halo, amino, mono- or diC₁₋₆alkylamino (in particular selected from C₁₋₆alkyl);
R^{9b} is hydrogen, halogen, C₁₋₆alkyl, C₃₋₇cycloalkyl, aryl, Het, halo (in particular bromo, chloro or fluoro); R^{9c} is hydrogen or C₁₋₆alkoxy;

Embodiments of R^{9a} for quinazolines include aryl or Het, especially wherein R^{9a} is phenyl, pyridyl, thiazolyl, oxazolyl or pyrazolyl either of which is optionally substituted with one, two or three (in particular with one) R¹⁰ as defined.

Embodiments of R¹⁰ for quinazoline include is hydrogen, methyl, ethyl, isopropyl, tert-butyl, halo (including dihalo, such as difluoro), pyrrolidinyl, piperidinyl, piperazinyl, 4-C₁₋₆alkylpiperazinyl (e.g. 4-methylpiperazinyl) or morpholinyl, C₁₋₆alkylamino, (C₁₋₆alkyl)₂amino, amino carbonyl, mono or diC₁₋₆alkylaminocarbonyl, or C₃₋₇cycloalkyl (in particular cyclopropyl).

Preferably R^{9a} in the quinazolyl radical specified under (f-1) or (f-1-a) includes any of radicals (q), (q'), (q'-1), (q-1), (q-2), (q-3), (q-4), (q-5), (q-6), (q-7), (q-8) specified above; wherein in these radicals R¹⁰ is as defined above or in particular is hydrogen, C₁₋₆alkyl (such as methyl, ethyl, isopropyl, tert-butyl), pyrrolidinyl, piperidinyl, piperazinyl, 4-C₁₋₆alkyl-piperazinyl, N-methylpiperazinyl or morpholinyl, C₁₋₆alkylamino, (C₁₋₆alkyl)₂amino or amino carbonyl, mono or diC₁₋₆alkylaminocarbonyl.

R^{9a} for quinazolines may include 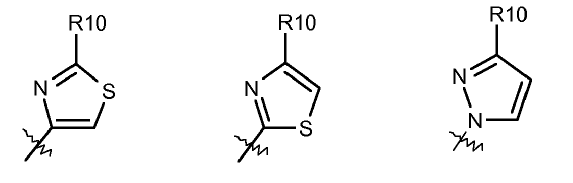 wherein R¹⁰ is hydrogen, halogen, C₁₋₆alkyl (such as methyl, ethyl, isopropyl, tert-butyl), C₁₋₆alkylamino, (C₁₋₆alkyl)₂amino, C₁₋₆alkylamido, morpholinyl or piperidin-1-yl, the morpholinyl and piperidinyl being optionally substituted with one or two C₁₋₆alkyl groups.

Additional R^{9a} embodiments for quinazolines include phenyl substituted with one or two R¹⁰ groups such as is hydrogen, methyl, ethyl, isopropyl, tert-butyl, pyrrolidinyl, piperidinyl, piperazinyl, C₁₋₆alkylamino, (C₁₋₆alkyl)₂amino or aminocarbonyl, mono- and diC₁₋₆alkylaminocarbonyl or halo (in particular fluoro).

Embodiments of R^{9b} for quinazolines include hydrogen, C₁₋₆alkyl, halo (e.g. bromo, chloro or fluoro) especially wherein R^{9b} is hydrogen or bromo. Embodiments of R^{9c} for quinazolines include hydrogen or C₁₋₆alkoxy (in particular methoxy).

Specific embodiments of the compounds of formula I or any of the subgroups of compounds of formula I are those wherein R⁸ is: 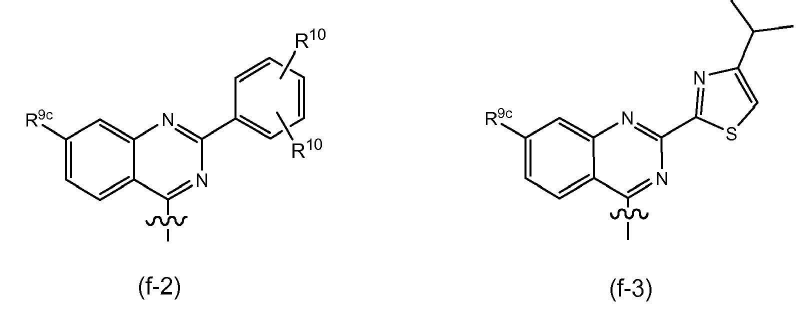 wherein each R¹⁰ and R^{9c} are as specified above and in particular R^{9c} is hydrogen or C₁₋₆alkoxy (e.g. methoxy).

Preferred amongst the subgroups of compounds of the embodiments wherein R⁸ is a radical (d-1) - (d-5), (e-1) - (e-3), (f-1) - (f-3) as specified above, are those compounds within these subgroups wherein is W is -O-.

Embodiments of the invention are compounds of formula I or any of the subgroups of compounds of formula I wherein W is a direct bond and R⁸ is selected from the group consisting of 1*H*-pyrrole, 1*H*-imidazole, 1*H*-pyrazole, furan, thiophene, oxazole, thiazole, isoxazole, isothiazole, pyridine, pyridazine, pyrimidine, pyrazine, phthalazine, quinoxaline, quinazoline, quinoline, cinnoline, 1*H*-pyrrolo[2,3-*b*]pyridine, 1*H*-indole, 1*H*-benzoimidazole, 1*H*-indazole, 7*H*-purine, benzothiazole, benzoxazole, 1*H*-imidazo[4,5-*c*]pyridine, 1*H*-imidazo[4,5-*b*]pyridine, 1, 3-dihydro-benzimidazol-2-one, 1,3-dihydro-benzimidazol-2-thione, 2,3-dihydro-1*H*-indole, 1,3-dihydro-indol-2-one, 1*H*-indole-2,3-dione, 1*H*-pyrrolo[2,3-*c*]pyridine, benzofuran, benzo[*b*]thiophene, benzo[*d*]isoxazole, benzo[*d*]isothiazole, 1H-quinotin-2-one, 1*H*-quinolin-4-one, 1*H*-quinazolin-4-one, 9*H*-carbazole, and 1*H*-quinazolin-2-one, each optionally substituted with the R⁸ substituents specified in the definitions of the compounds of formula I or any of the subgroups of compounds of formula I.

Further embodiments of the invention are compounds of formula I or any of the subgroups of compounds of formula I wherein W is a direct bond and R⁸ is selected from the group consisting of pyrrolidine, 4,5-dihydro-1*H*-pyrazole, pyrazolidine, imidazolidin-2-one, pyrrolidin-2-one, pyrrolidine-2,5-dione, piperidine-2,6-dione, piperidin-2-one, piperazine-2,6-dione, piperazin-2-one, piperazine, morpholine, pyrazolidin-3-one, imidazolidine-2,4-dione, piperidine, tetrahydrofuran, tetrahydropyran, 1,4-dioxane, and 1,2,3,6-tetrahydropyridine, each optionally substituted with the R⁸ substituents specified in the definitions of the compounds of formula I or any of the subgroups of compounds of formula I.

Embodiments of the invention are compounds of formula I or any of the subgroups of compounds of formula I wherein W is a direct bond and R⁸ is an optionally substituted tetrazolyl as depicted below: 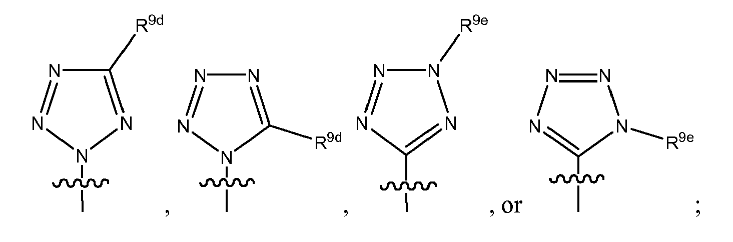 wherein R^{9d} is hydrogen, C₁₋₆alkoxy, hydroxy, -NR²R³, -C(=O)R³, -S(=O)ₚR³, C₃₋₇cycloalkyl, aryl, Het, or C₁₋₆alkyl optionally substituted with C₃₋₇cycloalkyl, aryl, or Het; R^{9e} is hydrogen, -NR²R³, C₃₋₇cycloalkyl, aryl, Het, or C₁₋₆alkyl optionally substituted with C₃₋₇cycloalkyl, aryl, or Het; and R² and R³ are as defined above.

Embodiments of the invention are compounds of formula I or any of the subgroups of compounds of formula I wherein W is a direct bond and R⁸ is optionally substituted triazolyl as depicted below: 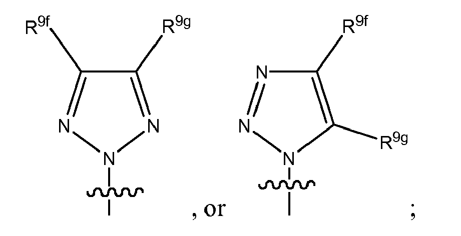 wherein R^{9f} and R^{9g} are each, independently, selected from the group consisting of hydrogen, halo, -C(=O)NR²R³, -C(=O)R³, C₃₋₇cycloalkyl, aryl, Het, and C₁₋₆alkyl optionally substituted with -NR²R³, or aryl; or alternatively, R^{9f} and R^{9g} taken together with the carbon atoms to which they are attached, may form a cyclic moiety selected from the group consisting of aryl and Het.

Further preferred substituents for R⁸ when W is a direct bond, include pyridazinone and derivatives thereof as shown below: 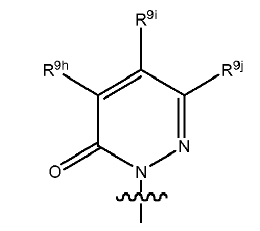 wherein R^{9h}, R⁹ⁱ and R^{9j} are independently selected from the group consisting of hydrogen, azido, halo, C₁₋₆alkyl, -NR²R³, C₃₋₇cycloalkyl, aryl, and Het; or alternatively, R^{9h} and R⁹ⁱ or R⁹ⁱ and R^{9j} taken together with the carbon atoms to which they are attached, may form a phenyl moiety, which in turn may be optionally substituted with azido, halo, C₁₋₆alkyl, -NR²R³, C₃₋₇cycloalkyl, aryl or Het.

Embodiments of the invention are compounds of formula I or any of the subgroups of compounds of formula I wherein W is -O-(C=O)-NH- or in particular wherein W is -O-(C=O)-NH- and R⁸ is aryl as defined above; or R⁸ is phenyl optionally substituted with 1, 2 or three substituents selected from those mentioned as possible substituents of the radical aryl as in the definitions of R⁹ of the compounds of formula I or of any of the subgroups of compounds of formula I; specifically R⁸ is a radical of formula: 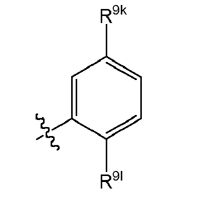 wherein
R^{9k} is hydrogen, C₁₋₆alkyl, polyhaloC₁₋₆alkyl or halo;
R⁹¹ is -C(=O)OR³, halo, Het or aryl; wherein Het and aryl are as defined herein and R³ is H or C₁₋₆alkyl, preferably R³ is methyl or ethyl.

In particular, R^{9k} may be hydrogen, fluoro or trifluoromethyl. In particular, R⁹¹ may be -COOC₁₋₆alkyl (e.g. -C(=O)OEt), phenyl, thiazolyl, 1-piperidinyl or 1-pyrazolyl, the phenyl, piperidinyl and pyrazolyl groups being optionally substituted with C₁₋₆alkyl, in particular with methyl.

Other embodiments of the invention are compounds of formula I or any of the subgroups of compounds of formula I wherein W is -O-(C=O)-NH- or, in particular, wherein W is -O-(C=O)-NH- and R⁸ is a radical of formula: 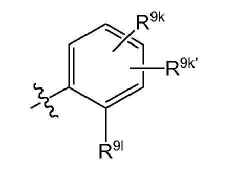 wherein R^{9k} and each R^{9k'} are, each independently from one another, hydrogen, halo, hydroxy, nitro, cyano, carboxyl, C₁₋₆alkyl, C₁₋₆alkoxy, C₁₋₆alkoxyC₁₋₆alkyl, C₁₋₆alkylcarbonyl, C₁₋₆alkoxycarbonyl, amino, azido, mercapto, C₁₋₆alkylthio, polyhaloC₁₋₆alkyl, aryl or Het; especially R⁹¹ and each R^{9k} are independently from one another hydrogen, halo, nitro, carboxyl, methyl, ethyl, isopropyl, t-butyl, methoxy, ethoxy, isopropoxy, t-butoxy, methylcarbonyl, ethylcarbonyl, isopropylcarbonyl, t-butyl-carbonyl, methoxycarbonyl, ethoxycarbonyl, isopropoxycarbonyl, t-butoxycarbonyl, methylthio, ethylthio, isopropylthio, t-butylthio, trifluoromethyl, or cyano;
R⁹¹ is aryl or Het, or R⁹¹ is -C(=O)OR³, wherein R³ is H or C₁₋₆alkyl, preferably methyl or ethyl.

In particular R⁹¹ may be phenyl, naphth-1-yl, naphth-2-yl, pyrrol-1-yl, 3-pyridyl, pyrimidin-4-yl, pyridazin-3-yl, pyridazin-2-yl, 6-oxo-pyridazin-1-yl, 1,2,3-triazol-2-yl, 1,2,4-triazol-3-yl, tetrazol-1-yl, tetrazol-2-yl, pyrazol-1-yl, pyrazol-3-yl, imidazol-1-yl, imidazol-2-yl, thiazol-2-yl, pyrrolidin-1-yl, piperidin-1-yl, furan-2-yl, thien-3-yl, morpholin-4-yl; all optionally substituted with one or two substituents selected from C₁₋₆alkyl, polyhaloC₁₋₆alkyl (such as trifluoromethyl) and C₁₋₆alkoxycarbonyl.

Preferred P3 groups, i.e. when m is 1, resemble natural or unnatural amino acids, especially aliphatic amino acids, such as L-valyl, L-leucyl, L-isoleucyl or L-*t*-leucyl.

Further preferred P3 groups, as shown in WO02/01898, include C₃₋₇cycloalkylalanine, or C₁₋₃alkyl substituted with C₃₋₇cycloalkylalanine, especially cyclohexylalanine optionally substituted with -C(=O)ORg, where Rg is H, amine, C₃₋₇cycloalkyl, aryl, Het, C₁₋₆alkyl optionally substituted with C₃₋₇cycloalkyl, aryl, or with Het; or N-acetyl-piperidine or tetrahydropyran. Preferred R¹¹ groups thus include C₃₋₇cycloalkyl, aryl, Het, C₁₋₆alkyl optionally substituted with C₃₋₇cycloalkyl, aryl, or with Het, any of which is optionally substituted with hydroxy, halo, amino, C₁₋₆alkoxy, C₁₋₆alkylthio, -C(=O)OR³, carboxyl, C₁₋₆alkoxycarbonyl, aryl, Het, especially where the substituent is hydroxy or -C(=O)OR³.

Preferred R¹¹ include *tert*-butyl, iso-butyl, cyclohexyl, phenylethyl, 2,2-dimethylpropyl, cyclohexylmethyl, phenylmethyl, 2-pyridylmethyl, 4-hydroxy-phenylmethyl, or carboxylpropyl. The most preferred R¹¹ values are *tert*-butyl, isobutyl, or cyclohexyl.

An embodiment of the invention includes compounds wherein P4 is absent (i.e. n is 0) and wherein the P3 function lacks a carbonyl, i.e. U is absent. Representative substructures include those of formula Ii below: 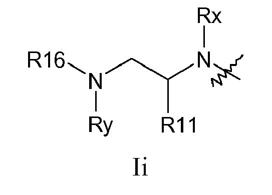 wherein Rx and Ry are as defined above, preferably H;
R¹¹ is C₁₋₆ alkyl, preferably branched C₃₋₅alkyl such as the side chains of L-valyl, L-leucyl, L-isoleucyl, L-*t*-leucyl; or C₃₋₇cycloalkyl, or C₁₋₂alkyl substituted with C₃₋₇cycloalkyl such as cyclohexyl or cyclohexylmethyl;
R¹⁶ is -Y-C(=O)R³, or -Y-S(=O)ₚR³, wherein R³ is C₃₋₇cycloalkyl, aryl or Het, or C₁₋₃alkyl optionally substituted with C₃₋₇cycloalkyl, aryl or with Het.

Alternatively, compounds of partial structure Ii may be macrocyclised between an appropriate value of R⁷ and one of Rx, Ry or R¹¹.

Representative embodiments of P3 groups that lack a carbonyl function (i.e. variable U is absent) include those of formula Iia-Iid below: 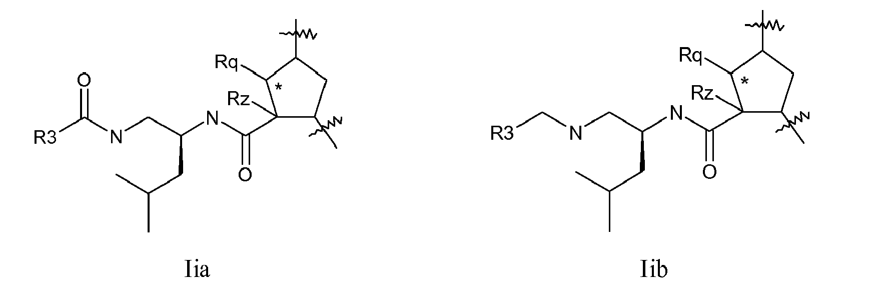 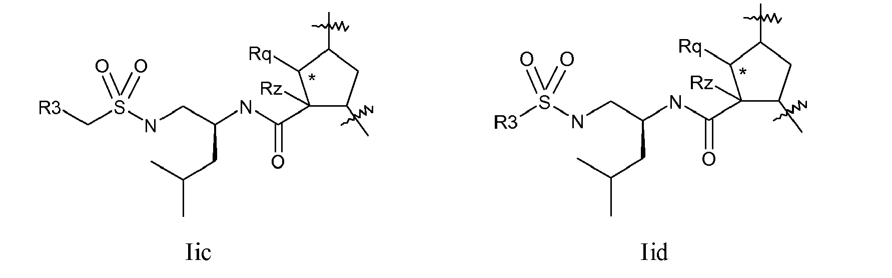 wherein R³ is C₃₋₇cycloalkyl, aryl or Het, especially aryl or Het, any of which is optionally substituted with halo, oxo, cyano, azido, nitro, C₁₋₆alkyl, amino, mono- or diC₁₋₆alkylamino, C₁₋₆alkoxy, C₁₋₆alkylcabonyl, aminocarbonyl, mono- or diC₁₋₆alkylaminocarbonyl, C₁₋₆alkylthio, mercapto, carboxyl, C₁₋₆alkoxycarbonyl. Although the partial structures of Formulae Iia - Iid have been illustrated in the context of a compound within Formula I, it will be apparent that such configurations of Formula Ii also apply to other values of q and k. Similarly, although the partial structures of formulae Iic and Iid show an R¹¹ group corresponding to leucine, these configurations may be applicable to other R¹¹ groups, especially those resembling the side chains of natural or unnatural L-amino acids, for example t-butyl alanine/t-leucine.

R¹⁵ in the compounds of the invention wherein n is 1, is preferably C₁₋₆alkyl optionally substituted with C₃₋₇cycloalkyl, or with aryl, for example C₁₋₃alkyl substituted with C₃₋₇cycloalkyl, any of which may be optionally substituted. Preferred P4 groups are typically analogues of natural or unnatural amino acids, especially aliphatic amino acids such as L-valyl, L-leucyl, L-isoleucyl, L-*t*-leucyl or L-cyclohexylalanine and thus preferred R¹⁵ groups include cyclohexyl, cyclohexylmethyl, *tert*-butyl, iso-propyl, or iso-butyl.

Preferred G values include -NRy-, especially wherein Ry is methyl or preferably H, or hydrazine. Another preferred G value is -O- thereby defining an ester with the carbonyl of P4 (if present) or the carbonyl of P3 (if present) or an ether in the case of variants wherein group U is absent. Conventional pharmaceutically acceptable ethers or esters capping groups for R¹⁶ include C₁₋₆alkyl (especially methyl or t-butyl), Het (especially pyridyl, benzimidazolyl, piperidyl, morpholinyl, piperazinyl), aryl, C₃₋₇cycloalkyl, or C₁₋₃alkyl substituted with aryl or C₃₋₇cycloalkyl (especially phenyl, benzyl, indanyl) any of which is optionally substituted with hydroxy, halo, amino, or C₁₋₆alkoxy.

Interesting compounds comprise a hydrazine group, for example where T is -NRd- and m is 1; with n being zero or 1. Alternatively, especially where m is zero, G can be -NRjNRj- such as -NHNH-, -NMeNH-, or -NMeNJ-. Compounds will generally not comprise a hydrazine at both G and T. Preferred hydrazines within Formula I, wherein m and n are zero include compounds of the partial structures Ija- Ijb below:

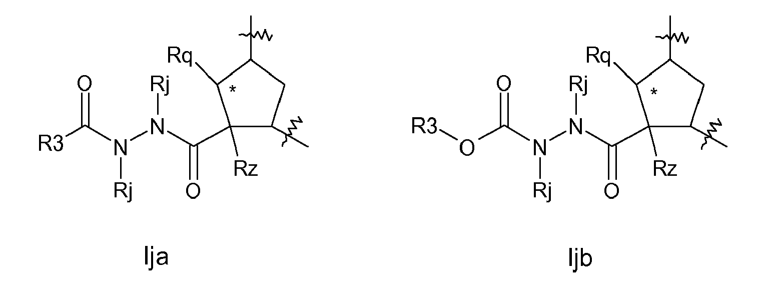

R³ in formulae Ija and Ijb can be C₃₋₇cycloalkyl, aryl, Het, or C₁₋₆alkyl optionally substituted with C₃₋₇cycloalkyl, aryl, or with Het, such as t-butyl, pyridyl, benzyl, phenyl, any of which is optionally substituted as defined above. Compounds of partial structures Ija and Ijb can be linear molecules as shown (both Rj are H), or preferably one of the depicted Rj groups can be macrocyclised via J to an appropriate R⁷ group.

Alternative hydrazines of Formula I where m is 1 include those of partial structures Ijc and Ijd below: 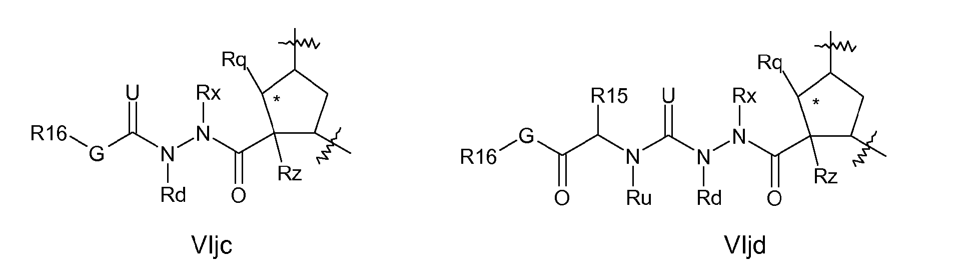 wherein G, R¹⁵, R¹⁶ ,Rx, Rd, Rq, Rz, and Ru are as defined for Formula I above. Compounds of partial structures Ijc and Ijd can be linear molecules as shown (both Rx and Rd are H), or preferably one of the depicted Rx or Rd groups can be macrocyclised via J to an appropriate R⁷ group. Although formulae Ija-Ijd are depicted with a five membered carbocyclic ring as P2 group, it will be apparent that this aspect of the invention is equally applies to other meanings of q' and k. Favored embodiments within formula Ija-Ijd include those wherein Rq and Rz are H, or those wherein Rz is a double bond and Rq is C₁₋₃alkyl.

Alternative hydrazine-like configurations are those wherein G is -NRy-, and m and n are 0, and R¹⁶ is an N-linked unsaturated heterocycle as defined below, for example pyridyl or pyrimidyl or a saturated heterocycle as defined below, such as piperazinyl, piperidinyl and especially morpholinyl. Examples of such embodiments include those of the formulae Ijc and Ijd:

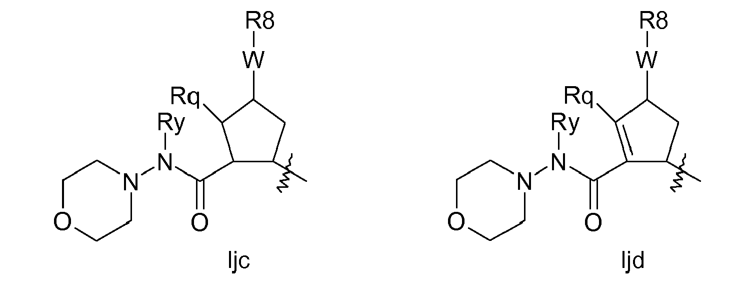

Compounds of partial structures Ijc and Ijd can be linear molecules as shown or preferably Rx can be macrocyclised via J to an appropriate R⁷ group. Although these partial structures are depicted with a five membered ring as the P2 scaffold, it will be readily apparent that this configuration extends to other values of q' and k. Similarly these configurations will be applicable to other N-linked heterocycles as R¹⁶.

Interesting R¹⁶ groups for the compounds of the invention include 2-indanol, indanyl, 2-hydroxy-1-phenyl-ethyl, 2-thiophenemethyl, cyclohexylmethyl, 2,3-ethylene-dioxybenzyl, cyclohexyl, phenyl, benzyl, 2-pyridylmethyl, cyclobutyl, iso-butyl, n-propyl, methyl, or 4-methoxyphenylethyl. Preferred R¹⁶ groups include H, methyl, pyrid-2-yl, pyrimid-2yl, 2-indanol, indan, 2-hydroxy-1-phenyl-ethyl, 2-thiophenemethyl, 2,3-methylenedioxybenzyl, or cyclohexylmethyl.

Unnatural amino acids include L-amino acids wherein the side chain is not one of the 20 naturally occurring amino acids. Examples of non-natural amino acids include L-beta-methylsulfonylmethylalanine, L-cyclohexylalanine, L-tertiary-leucine, L-norleucine, L-norvaline, L-ornithine, L-sarcosine, L-citurline, L-homophenylalanine, L-homoserine, L-beta-(1-napthyl)alanine, L-beta-(2-napthyl)alanine etc. Non-natural amino acids also include the D-amino acids corresponding to the 20 natural amino acids and D-amino acids bearing other side chains, such as those listed above.

### Synthesis

Synthesis of the compounds of the present invention can be performed by different chemical strategies in solution or solid phase or a combination of both. The suitably protected individual building blocks can first be prepared and subsequently coupled together i.e. P2+P1 → P2-P1. Alternatively, precursors of the building blocks can be coupled together and modified at a later stage of the synthesis of the inhibitor sequence. Further building blocks, precursors of building blocks or prefabricated bigger fragments of the desired structure, can then be coupled to the growing chain, e.g.
R¹⁶-G-P3 + C(=O)-P2-P1 → R¹⁶-G-P3-C(=O)-P2-P1 or
R¹⁶-G-P4-P3 + C(=O)-P2-P1 → R¹⁶-G-P4-P3-C(=O)-P2-P1.

Coupling between two amino acids, an amino acid and a peptide, or two peptide fragments can be carried out using standard coupling procedures such as the azide method, mixed carbonic-carboxylic acid anhydride (isobutyl chloroformate) method, carbodiimide (dicyclohexylcarbodiimide, diisopropylcarbodiimide, or water-soluble carbodiimide) method, active ester (p-nitrophenyl ester, N-hydroxysuccinic imido ester) method, Woodward reagent K-method, carbonyldiimidazole method, phosphorus reagents or oxidation-reduction methods. Some of these methods (especially the carbodiimide method) give better results when adding 1-hydroxybenzotriazole or 4-DMAP. These coupling reactions can be performed in either solution (liquid phase) or solid phase.

The coupling step involves the dehydrative coupling of a free carboxyl of one reactant with the free amino group of the other reactant in the present of a coupling agent to form a linking amide bond. Descriptions of such coupling agents are found in general textbooks on peptide chemistry, for example, M. Bodanszky, "Peptide Chemistry", 2nd rev ed., Springer-Verlag, Berlin, Germany, (1993) hereafter simply referred to as Bodanszky, the contents of which are incorporated herein by reference. Examples of suitable coupling agents are N,N'-dicyclohexylcarbodiimide, 1-hydroxybenzotriazole in the presence of N,N'- dicyclohexylcarbodiimide or N-ethyl-N'-[(3-dimethylamino)-propyl]carbodiimide. A practical and useful coupling agent is the commercially available (benzotriazol-1-yloxy) tris-(dimethylamino) phosphonium hexafluorophosphate, either by itself or in the presence of 1-hydroxybenzotriazole or 4-DMAP. Another practical and useful coupling agent is commercially available 2-(1H-benzotriazol-1-yl)-N, N, N',N'-tetramethyluronium tetrafluoroborate. Still another practical and useful coupling agent is commercially available O-(7-aza-benzotriazol-1-yl)-N,N,N',N'-tetramethyluronium hexafluorophosphate.

The coupling reaction is conducted in an inert solvent, e.g. dichloromethane, acetonitrile or dimethylformamide. An excess of a tertiary amine, e.g. diisopropylethylamine, N-methylmorpholine, N-methylpyrrolidine or 4-DMAP is added to maintain the reaction mixture at a pH of about 8. The reaction temperature usually ranges between 0°C and 50°C and the reaction time usually ranges between 15 min and 24 h.

The functional groups of the constituent amino acids generally has to be protected during the coupling reactions to avoid formation of undesired bonds. The protecting groups that can be used are listed in Greene, "Protective Groups in Organic Chemistry", John Wiley & Sons, New York (1981), and "The Peptides: Analysis, Synthesis, Biology", Vol. 3, Academic Press, New York (1981), hereafter referred to as Greene, incorporated herein by reference.

The α-carboxyl group of the C-terminal residue is usually protected as an ester that can be cleaved to give the carboxylic acid. Protecting groups that can be used include 1) alkyl esters such as methyl, trimethylsilyl and t-butyl, 2) arylalkyl esters such as benzyl and substituted benzyl, or 3) esters that can be cleaved by mild base or mild reductive means such as trichloroethyl and phenacyl esters. The α-amino group of each amino acid to be coupled is typically protected. Any protecting group known in the art can be used. Examples of such groups include: 1) acyl groups such as formyl, trifluoroacetyl, phthalyl, and p-toluenesulfonyl; 2) aromatic carbamate groups such as benzyloxycarbonyl (Cbz or Z) and substituted benzyloxycarbonyls, and 9-fluorenylmethyloxycarbonyl (Fmoc); 3) aliphatic carbamate groups such as *tert*-butyloxycarbonyl (Boc), ethoxycarbonyl, diisopropylmethoxy-carbonyl, and allyloxycarbonyl; 4) cyclic alkyl carbamate groups such as cyclopentyloxycarbonyl and adamantyloxycarbonyl; 5) alkyl groups such as triphenylmethyl and benzyl; 6) trialkylsilyl such as trimethylsilyl; and 7) thiol containing groups such asphenylthiocarbonyl and dithiasuccinoyl. The preferred α-amino protecting group is either Boc or Fmoc. Many amino acid derivatives suitably protected for peptide synthesis are commercially available.

The α-amino protecting group is cleaved prior to the next coupling step. When the Boc group is used, the methods of choice are trifluoroacetic acid, neat or in dichloromethane, or HCl in dioxane or in ethyl acetate. The resulting ammonium salt is then neutralized either prior to the coupling or in situ with basic solutions such as aqueous buffers, or tertiary amines in dichloromethane or acetonitrile or dimethylformamide. When the Fmoc group is used, the reagents of choice are piperidine or substituted piperidine in dimethylformamide, but any secondary amine can be used. The deprotection is carried out at a temperature between 0°C and room temperature usually 20-22°C.

Any of the natural or non-natural amino acids having side chain functionalities will typically be protected during the preparation of the peptide using any of the above described groups. Those skilled in the art will appreciate that the selection and use of appropriate protecting groups for these side chain functionalities depend upon the amino acid and presence of other protecting groups in the peptide. In the selection of such protecting groups it is desirable that the group is not removed during the deprotection and coupling of the α-amino group.

For example, when Boc is used as the α-amino protecting group, the following side chain protecting groups are suitable: p-toluenesulfonyl (tosyl) moieties can be used to protect the amino side chain of amino acids such as Lys and Arg; acetamidomethyl, benzyl (Bn), or *tert*-butylsulfonyl moities can be used to protect the sulfide containing side chain of cysteine; benzyl (Bn) ethers can be used to protect the hydroxy containing side chains of serine, threonine or hydroxyproline; and benzyl esters can be used to protect the carboxy containing side chains of aspartic acid and glutamic acid. When Fmoc is chosen for the α-amine protection, usually *tert*-butyl based protecting groups are selected. For instance, Boc can be used for lysine and arginine, *tert*-butyl ether for serine, threonine and hydroxyproline, and *tert-*butyl ester for aspartic acid and glutamic acid. Triphenylmethyl (Trityl) moiety can be used to protect the sulfide containing side chain of cysteine.

Once the couplings are completed, the protecting groups are removed using art-known procedures.

### Introduction of the P2 substituent

The R⁸ group can be coupled to the P2 group at any convenient stage of the synthesis of compounds according to the present invention. One approach is to first couple the R⁸ group to the P2 scaffold and subsequently adding the other desired building blocks, i.e. P1 and optionally P3 and P4. Another approach is to couple the P1, P2 and if present P3 and P4 moieties using an unsubstituted P2 group and add the R⁸ group afterwards.

Compounds wherein W is O and R⁸ is C₁₋₆alkyl or a ring system as specified above can be prepared according to the procedure described by E. M. Smith et al. (J. Med. Chem. (1988), 31, 875-885), as depicted in Scheme 1, which illustrates the technique with a saturated P2 scaffold wherein q' is 0 and k is 1.

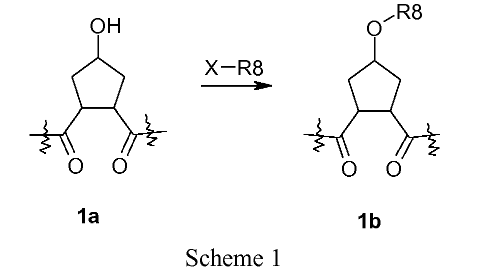

Treatment of a compound containing an unsubstituted P2 moiety (1a), which can be prepared as described herein below with a base such as sodium hydride or potassium t-butoxide in a solvent like dimethylformamide followed by reaction of the resulting alkoxide with an alkylating agent, R⁸-X, wherein X is a suitable leaving group such as a halide, mesylate, triflate or tosylate, provides the desired substituted derivative (1b). Alternatively, if X is OH or SH, the P2 substituent can be introduced via a Mitsunobu reaction by reacting the hydroxy group of compound la with the desired alcohol or thiol in the presence of triphenylphosphine and an activating agent like diethyl azodicarboxylate (DEAD), diisopropyl azodicarboxylate (DIAD) or the like. (Mitsunobu, 1981, Synthesis, January, 1-28; Rano et al., Tetrahedron Lett., 1995, 36, 22, 3779-3792; Krchnak et al., Tetrahedron Lett., 1995, 36, 5, 6193-6196; Richter et al., Tetrahedron Lett., 1994, 35, 27, 4705-4706).

Alcohol (1a) can alternatively be treated with phosgene thus providing the corresponding chloroformate which upon reaction with an amine, R⁸NH₂, in the presence of a base like sodium hydrogencarbonate or triethylamine, provides carbamates i.e. W is -OC(=O)NH-, whereas reaction of alcohol (1a) with an acylating agent, R⁸-CO-X, like an acid anhydride or acid halide for instance the acid chloride, to provide esters, i.e. W is -OC(=O)-. Another route to compounds wherein the P2-substituent is linked to the cyclic scaffold via a carbamate is to react alcohol 1a with the isocyanate of the P2-substituent in the presence of a base such as potassium t-butoxide.

Various alcohols R⁸-OH, and alkylating agents R⁸-X are described in WO00/09543 and WO00/59929. An example of the synthesis wherein R⁸ is a substituted quinoline derivative is shown in Scheme 2.

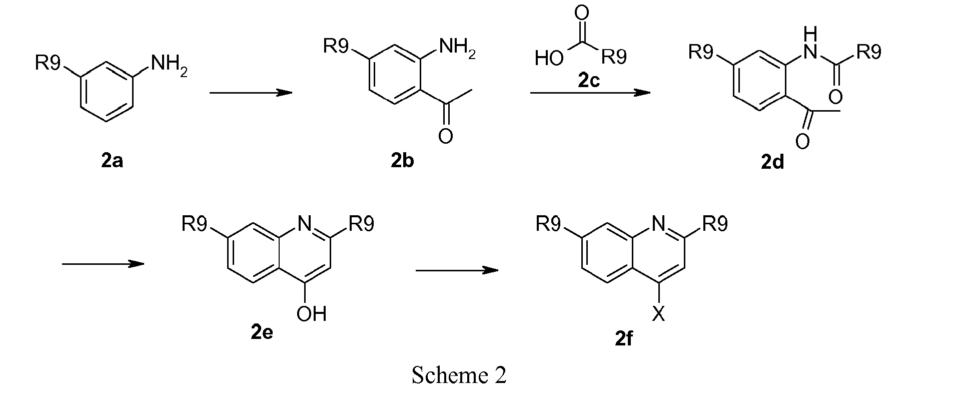

Friedel-Craft acylation of a suitable substituted aniline (2a), available either commercially or in the literature, using an acylating agent like acetyl chloride or the like in the presence of boron trichloride and aluminium trichloride in a solvent like dichloromethane provides (2b). Coupling of (2b) to a heterocyclic carboxylic acid (2c) under basic conditions, such as in pyridine, in the presence of an activating agent for the carboxylate group, for instance POCl₃, followed by ring closure and dehydration under basic conditions like potassium *tert*-butoxide in *tert*-butanol provides quinoline derivative (2e). Quinoline derivative (2e) can be coupled in a Mitsunobu reaction to an alcohol as described above, or the hydroxy group can be displaced by a suitable leaving group such as a halide like chloride, bromide or iodide, by treatment of quinoline (2e) with an appropriate halogenating agent for example phosphoryl chloride or the like.

A variety of carboxylic acids with the general structure (2c) can be used in Scheme 2. These acids are available either commercially or in the literature. Scheme 3 shows an example of the preparation of 2-(substituted)-amino-carboxy-aminothiazole derivatives, following the procedure by Berdikhina et al. Chem. Heterocycl. Compd. (Engl. Transl.) (1991), 427-433.

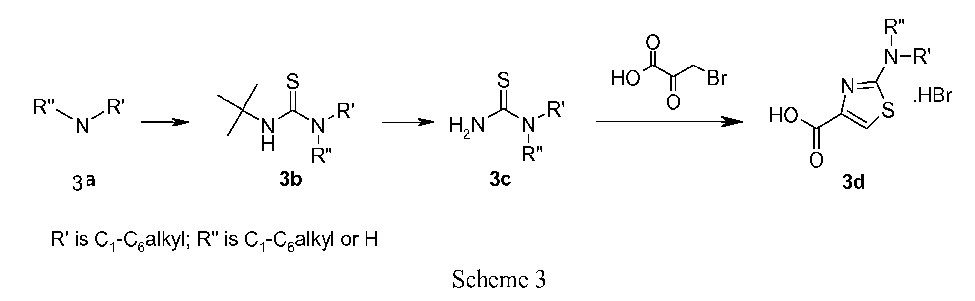

Thiourea (3c) with different alkyl substituents R' and R" can be formed by reaction of the appropriate amine (3a) with *tert*-butylisothiocyanate in the presence of a base like diisopropylethylamine in a solvent like dichloromethane followed by removal of the *tert*-butyl group under acidic conditions. Subsequent condensation of thiourea derivative (3c) with 3-bromopyruvic acid provides the acid (3d).

4-Substituted thiazole-2-carboxylic acids to be used in the reaction with the amine 2b in scheme 2 can be prepared as illustrated in scheme 3A.

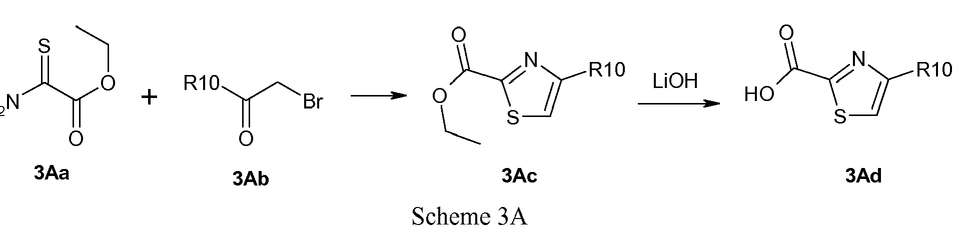

Condensation of ethyl thiooxamate (3Aa) with a desired α-bromoketone (3Ab) followed by ester hydrolysis performed by treatment with a base such as lithium hydroxide provides the thiazole caboxylic acid (3Ad). α-Bromoketones (3Ab) are commercially available or can be prepared by α-bromination of the corresponding keton.

An example of the synthesis of substituted quinazoline derivatives to be used as P2-substituents is shown in Scheme 3B.

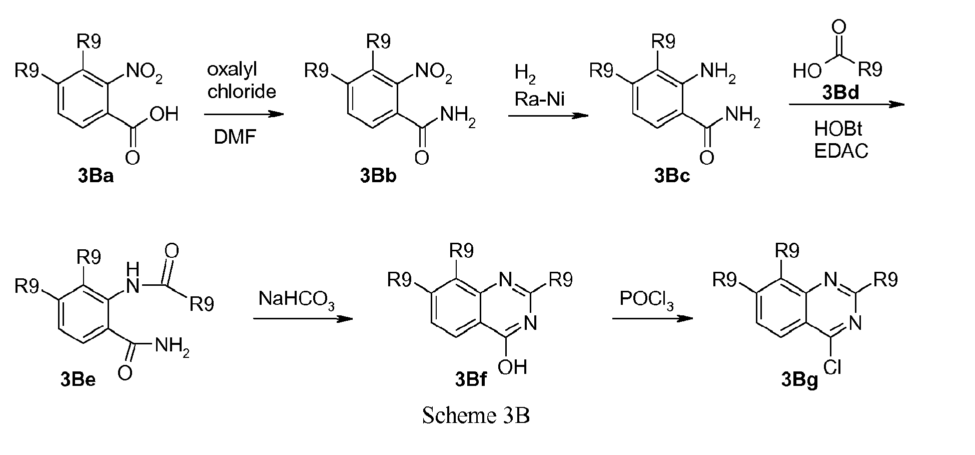

Transformation of a nitro substituted benzoic acid derivative (3Ba) to the corresponding benzamide for example by subjection of the acid to Vilsmeyer conditions followed by reduction of the nitro group using conditions like catalytic hydrogenation over Raney-nickel gives the corresponding amine (3Bc). The afforded amine can subsequently be coupled to a heterocyclic carboxylic acid (2d) under peptide coupling conditions, such as with HOBt and EDAC or any other suitable coupling agents well known in the art. Ring closure and dehydration can thereafter be performed by treatment with a base such as sodium hydrogencarbonate, which provides the quinazoline derivative (3Bf). The quinazoline derivative (3Bf) can then be coupled to the hydroxy group of a P2 scaffold in a Mitsunobu reaction as described above, or the hydroxy group of the quinazoline can be replaced by a suitable leaving group such as a halide like chloride, bromide or iodide, by treatment of quinazolinol (3Bf) with an appropriate halogenating agent for example phosphoryl chloride or the like.

P2 building blocks wherein the R⁸ substituent is attached via an amine, amide, urea or sulphonamide, can be prepared from amino substituted carbocycles achieved for example by transforming the hydroxy group of the corresponding hydroxy derivative into an azide group for example by transforming the hydroxy group into a suitable leaving group such as a mesylate or halogen like chloride, followed by substitution of the leaving group with azide or by the use of an azide transfer agent like diphenylphosphoryl azide (DPPA). Reduction of the azide by catalytic hydrogenation or any other suitable reduction method provides the amine. The amino derivative can be reacted in a displacement reaction with an arylating agent of the general formula R⁸-X wherein R⁸ and X are as described for scheme 1, to form P2 building blocks for use in the preparation of compounds of general formula I, wherein W is -NH-. Reaction of the amino substituted carbocycle with an acid of the general formula R⁸-COOH under standard amide coupling conditions provides compounds wherein the R⁸ substituent is linked via an amide bond, whereas reaction of the amino substituted carbocycle with an appropriate derivative of sulphonic acid, R⁸-S(O)₂-X where X is a leaving group for example chloride, in the presence of a base, provides sulphonamides. Compounds wherein the linkage between the cyclic scaffold and the R⁸ substituent is constituted of a urea group can for example be achieved by treatment of an amino substituted carbocycle with phosgene to afford the corresponding chlorocarbamate, followed by a reaction with the desired amine. Alternatively, the amino substituted carbocycle can be reacted with the carbamoyl chloride or isocyanate of the desired R⁸ substituent for the formation of the urea linkage. It will be apparent that corresponding reactions will be available for P2 groups with other ring sizes and substitution pattern.

Compounds wherein a heterocyclic R⁸ group is attached directly to the cyclic P2 scaffold i.e. W is a bond in general formula I, can be prepared for example by using a replacement reaction wherein a suitable leaving group such as a halide or a mesylate or the like on the P2 scaffold is replaced by the desired R⁸ group such as a heterocyclic group. Alternatively the R⁸ group can be introduced by way of a Mitsunobu reaction wherein the hydroxy group of the P2 precursor is reacted with a nitrogen atom in the heterocyclic R⁸ group.

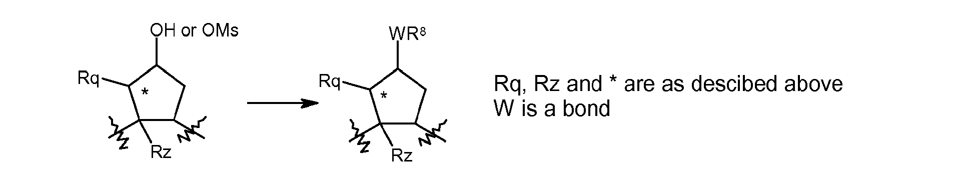

Compounds wherein a tetrazole derivative is attached to one of its ring carbons are conveniently prepared by building up the tetrazole moiety directly on the P2 precursor. This can be achieved for instance by transforming the hydroxy group of the P2 precursor into a cyano group followed by reaction with an azide reagent like sodium azide. Triazole derivatives can also be built up directly on the P2 precursor, for example by transforming the hydroxy group of the P2 precursor into an azide group followed by a 3+2 cycloaddition reaction of the afforded azide and a suitable alkyne derivative.

Structurally diverse tetrazoles for use in the above described substitution or Mitsunobu reactions can be prepared by reacting commercially available cyano compounds with sodium azide. Triazole derivatives can be prepared by reaction of an alkyne compound and trimethylsilyl azide. Useful alkyne compounds are available either commercially or they can be prepared for instance according to the Sonogashira reaction i.e. reaction of a primary alkyne, an aryl halide and triethylamine in the presence of PdCl₂(PPh)₃ and CuI as described for example in A. Elangovan, Y.-H. Wang, T.-I. Ho, Org. Lett., 2003, 5, 1841-1844. The heterocyclic substituent can also be modified when attached to the P2 building block either before or after coupling of the P2 building block to the other building blocks.

These methods and further alternatives for the preparation of compounds wherein W is a bond and R⁸ is an optionally substituted heterocycle are extensively described in WO2004/072243. Compounds having another ring type and/or position of the W-R⁸ substituent of the carbocyclic derivative in scheme 1 may also be used in the preparation of compounds according to the present invention.

### Synthesis and introduction of P1 building blocks.

The amino acids used in the preparation of P1 fragments are available either commercially or in the literature, see for example WO02/018369.
Scheme 4' shows some examples of the preparation and coupling of A fragments.

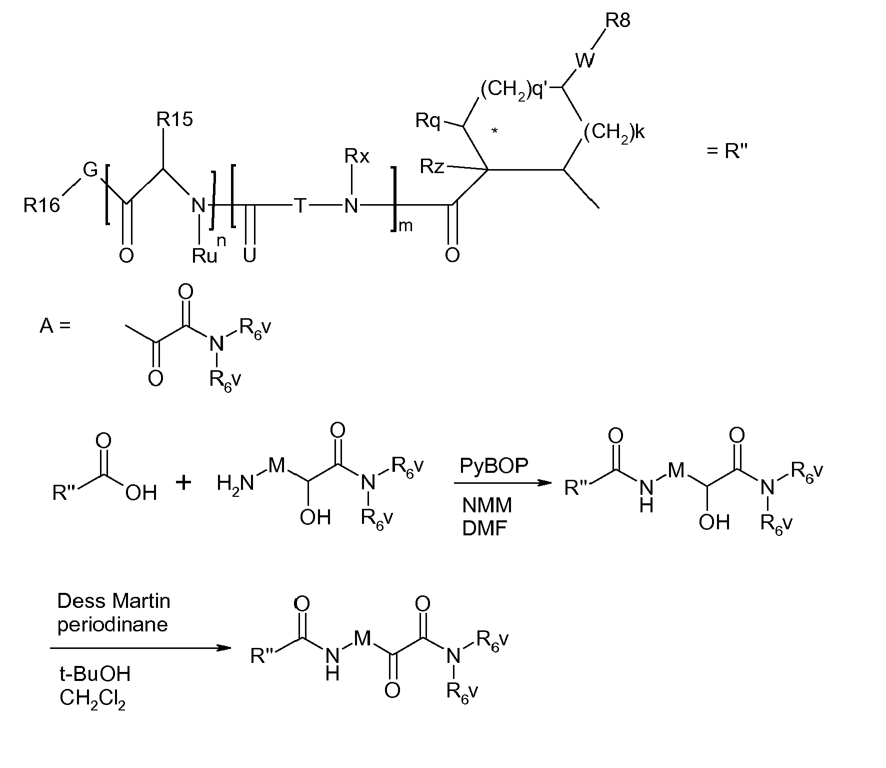

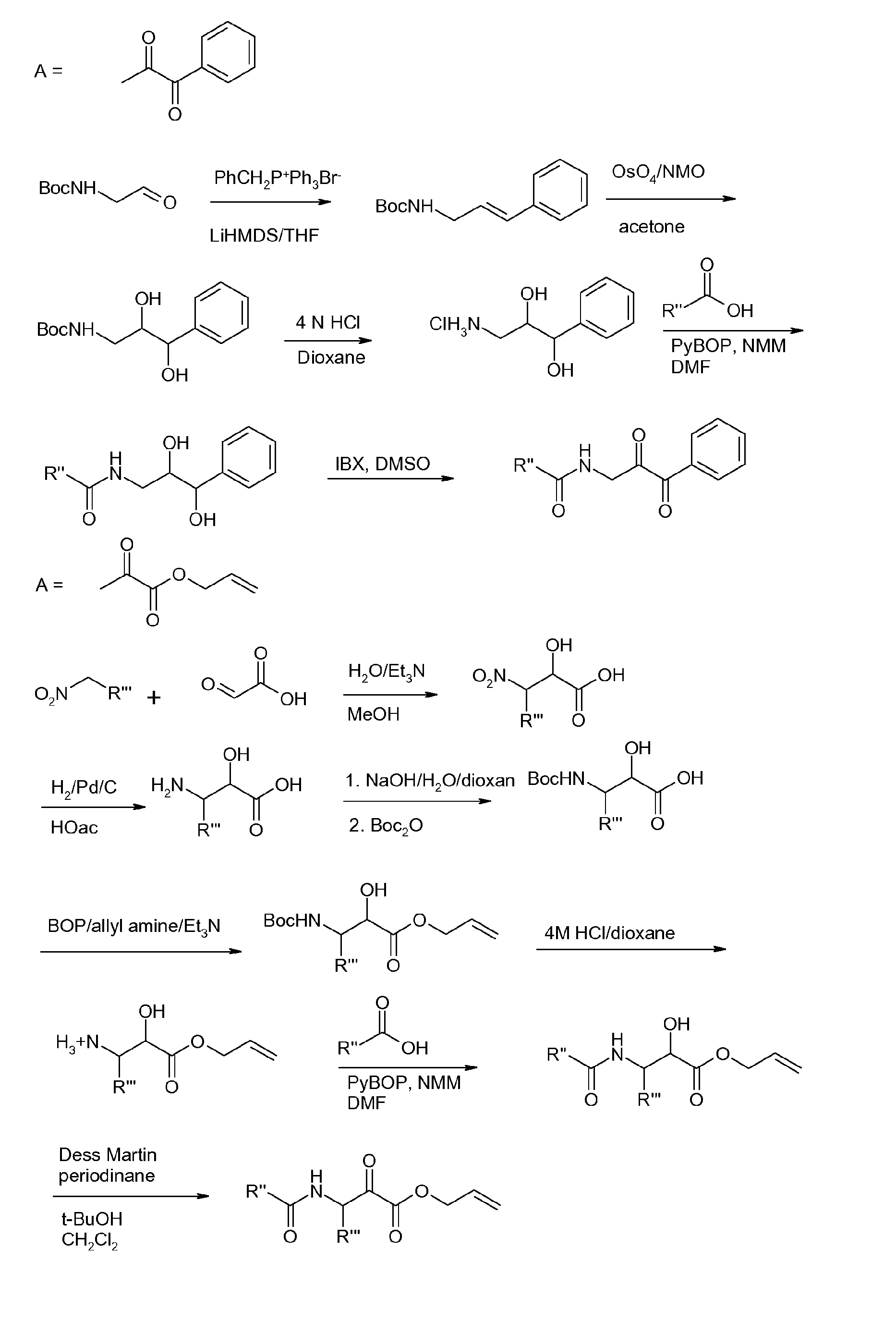

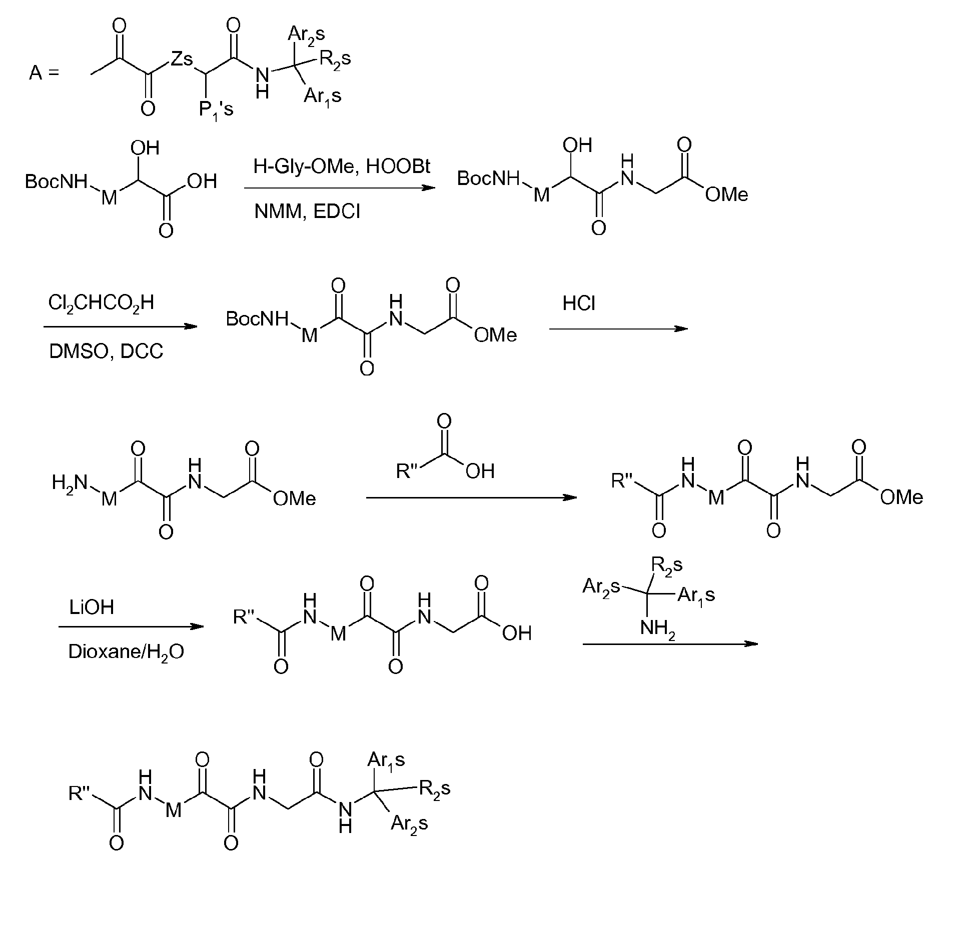

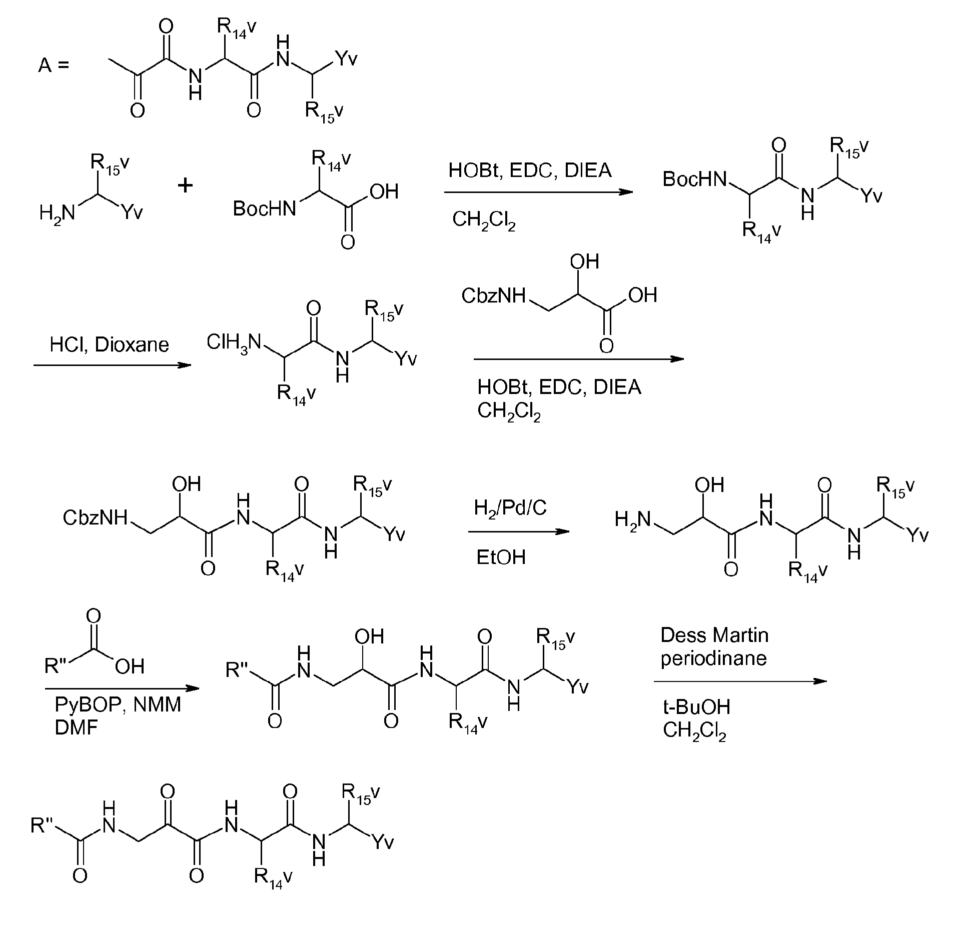

### Synthesis of capped P3 and P4-P3 building blocks

The building blocks R¹⁶-G-P3 and R¹⁶-G-P4-P3 can be prepared as generally depicted in scheme 6.

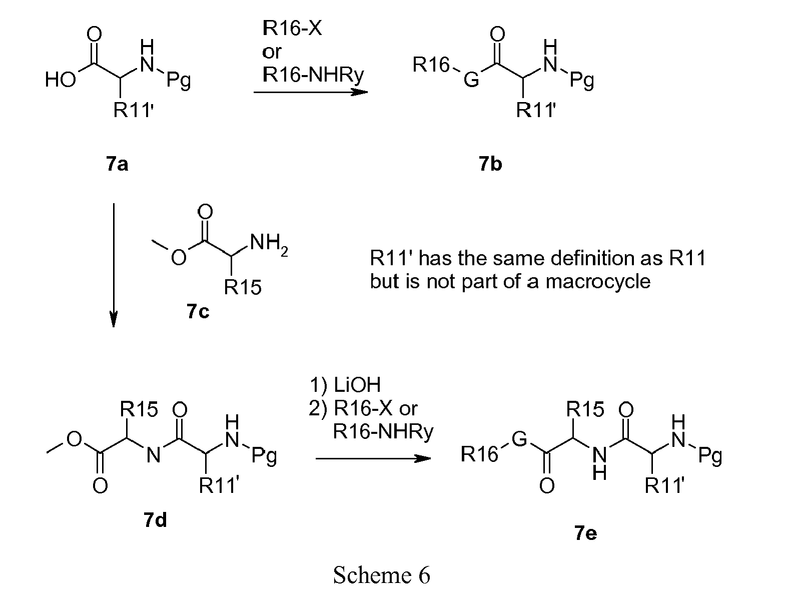

A suitable N-protected amino acid (6a) can be coupled with an amino capping group (R¹⁶-NHRy) using standard peptide coupling conditions like with coupling agents such as HATU, DCC, HOBt or the like in the presence of a base such as DIEA or DMAP in a solvent like dichloromethane, chloroform or dimethylformamide or a mixture thereof and ester formation conditions like providing amides i.e. G is NHRy (6b). Alternatively, reaction of amino acid (6a) with a compound of general formula R¹⁶-X where R¹⁶ is as defined above and X is a leaving group such as a halide, in the presence of a base like cesium carbonate or silver (I) oxide provides esters, i.e. G is -O- (6b). On the other hand, amino acid (6a) can be coupled to a second, suitably O-protected, amino acid (6d) using standard peptide coupling conditions as described above, providing (6e). Displacement of the ester group with a suitable capping group (6b) provides fragment (6f) useful for the preparation of compounds according to the present invention wherein m and n are 1.

When G is -N-Ry, the capped P3 or P2 building block can also be prepared on solid support as exemplified in Scheme 7. An appropriate N-protected, for example Boc protected, amino acid (7a) can be immobilized on a solid support, here exemplified by Agronaut resin PS-TFP, by reacting the amino acid with the desired solid support in the presence of coupling reagent like N,N'-diisopropylcarbodiimide and a base like DMAP in a solvent like dichloromethane and dimethylformamide. The immobilized amino acid (7b) can then be cleaved from the support with a suitable capping group (7c) thus giving fragments (7d) useful for the preparation of compounds according to the present invention wherein m or n is 1. Optionally the amino protecting group can be removed followed by coupling of an appropriate amino acid using standard methods thus providing fragments useful for the preparation of compounds according to the present invention wherein m and n are 1.

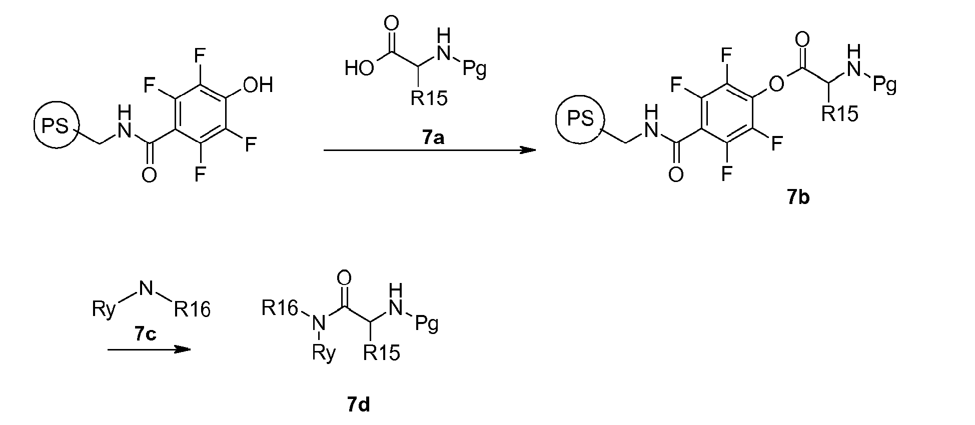

### Preparation and incorporation of P2 building blocks

A typical route to compounds containing a 5 membered saturated P2 scaffold is shown in Scheme 8.

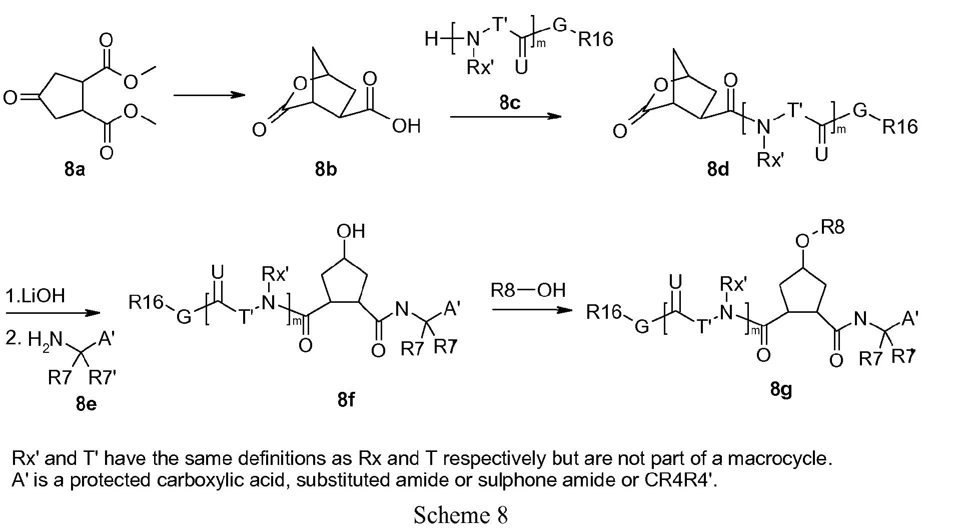

The cyclic lactone (8b) can be prepared, for example, from 3,4-bis(methoxycarbonyl)-cyclopentanone (8a), as described by Rosenquist et al. in Acta Chem. Scand. 46 (1992) 1127-1129 by reduction of the keto group with a reduction agent like sodium borohydride in a solvent like methanol, followed by hydrolysis of the esters and finally ring closure in acetic anhydride in the presence of pyridine. The resulting bicyclic acid (8b) can then be coupled to the amine function of the desired P3 fragment (8c), P3-P4 fragment or capping group R¹⁶-NHRy, using conventional peptide coupling conditions like with HATU and diisopropyl amine in a solvent like dimethyl formamide, giving (8d). Lactone opening of (8d) with e.g. LiOH provides the acid, which subsequently can be coupled to the amino group of a P1 building block or a precursor of a desired P1 fragment (8e), using conventional peptide coupling conditions. The R⁸-substituent of the carbocycle can be introduced for example by a Mitsunobu reaction with the appropriate alcohol as described above or by any other suitable method previously described. When R⁷, R^{7,} and A' contain functional groups, these are optionally suitably protected by art-known methods.

Scheme 9 shows an alternative route towards compounds of Formula I with a saturated P2 group, where the building blocks are introduced in the reversed order, i.e. the P1 fragment is introduced before the capping group, P3 or P3-P4 building block.

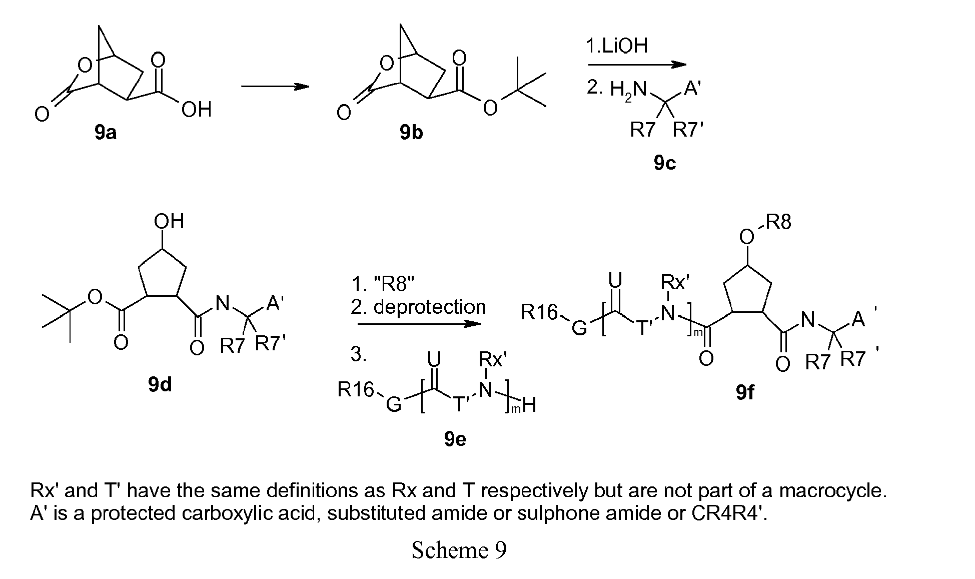

Protection of the acid group of (9a) for example as the *tert*-butyl ester by treatment with di-*tert*-butyl dicarbonate in the presence of a base like dimethylaminopyridine and triethylamine in a solvent like dichloromethane provides ester (9b). Lactone opening and coupling of a P1 building block (9c) as described in scheme 13 or directly by the amine group of the P1 fragment provides (9d). Introduction of an R⁸-substituent, as described above, followed by removal of the acid protection group by subjecting the ester to acidic conditions like trifluoroacetic acid and triethylsilane in a solvent like methylene chloride, and finally coupling of the P3 building block (9e), P3-P4 building block or capping group R¹⁶-NHRy as described above, provides (9f). When R⁷, R⁷' and A' contain functional groups, these are optionally suitably protected using art-known methods, see for example Bodanzky or Greene cited above.

An unsaturated P2 group to be used in the preparation of compounds of Formula I can be prepared as illustrated with cyclopentene below in scheme 10.

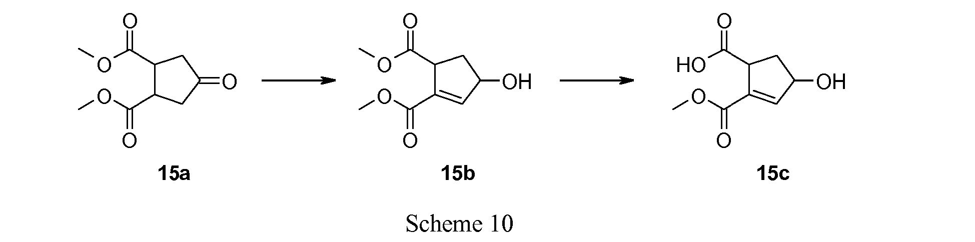

A bromination-elemination reaction of 3,4-bis(methoxycarbonyl)cyclopentanone (15a), as described by Dolby et al. in J. Org. Chem. 36 (1971) 1277-1285, followed by reduction of the keto functionality with a reduction agent like sodium borohydride, provides the unsaturated hydroxy compound (15b). Selective ester hydrolysis using for example lithium hydroxide in a solvent like a mixture of dioxane and water, provides hydroxy substituted monoester derivative (15c).

An unsaturated P2 group wherein Rq is different from hydrogen, such as a methylated cyclopentene moiety, can be prepared as shown in scheme 11.

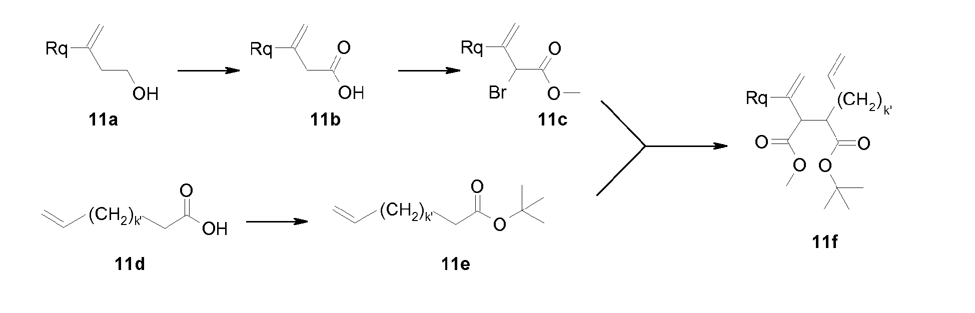

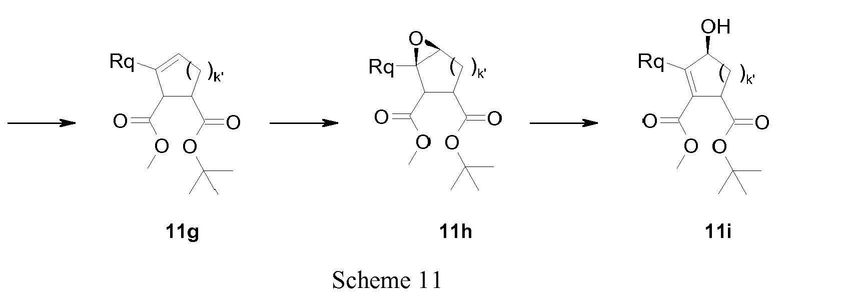

Oxidation of commercially available 3-methyl-3-buten-1-ol (16a) by the use of an oxidation agent like pyridinium chlorochromate followed by treatment with acetyl chloride, bromine and methanol, provides the α-bromo ester (16c). The afforded ester (16c) can then be reacted with the enolate of (16e), achieved for example by treatment of the corresponding *tert*-butyl ester with a base such as lithium diisopropyl amide in a solvent like tetrahydrofuran, to give the alkylated compound (16f). The *tert*-butyl ester (16e) can be prepared by treatment of the corresponding, commercially available acid (16d), where k' is 1 to 3, with di-*tert*-butyl dicarbonate in the presence of a base like dimethylaminopyridine. Cyclisation of (16f) by an olefin metathesis reaction performed as described above provides cyclopentene derivative (16g). Stereoselective epoxidation of (16g) can be carried out using the Jacobsen asymmetric epoxidation method to furnish the epoxide (16h). Finally, addition of a base like DBN (1,5-diazabicyclo-[4.3.0]non-5-ene) yields the alcohol (16i). Optionally the double bond of compound (16i) can be reduced, for example by catalytic hydrogenation, using a catalyst like palladium on carbon, which provides the corresponding saturated compound.

The resulting cyclic groups can then be used, as described above, to complete the molecular sequence. An example is shown in scheme 12.

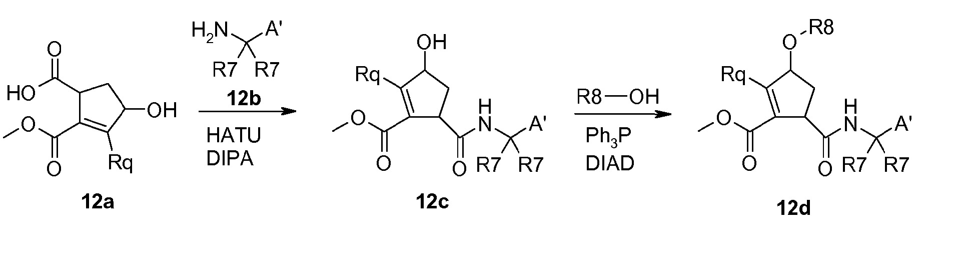

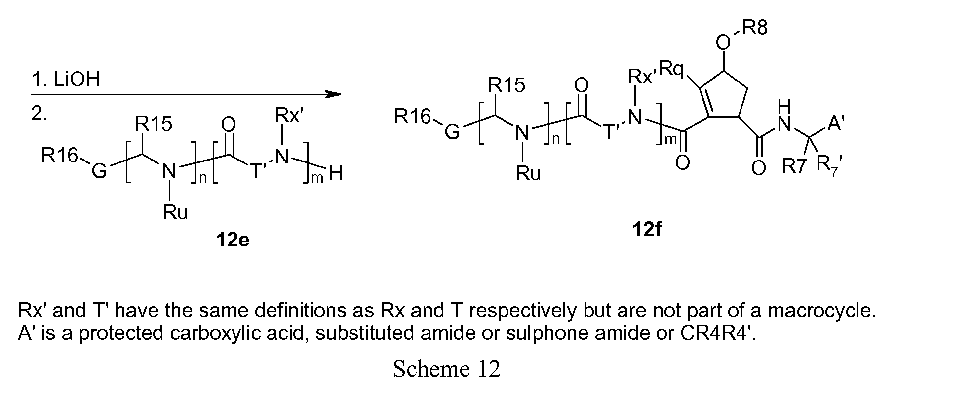

The amino group of a P1-building block or a suitable precursor thereof (12b) can be coupled to the acid of the cyclopentene derivative (12a) using standard amide coupling conditions such as using HATU in the presence of a base like diisopropyl phenylamine or the like, followed by introduction of the R⁸-substituent for example by Mitsunobu conditions as described above to provide (12d). Hydrolysis of the remaining ester and subsequent amide coupling of a desired P3 or P3-P4 building block (12e), optionally followed by manipulations of the P1 part, provides cyclopentene containing compounds (12f) according to general formula I. When R⁷, R⁷, and A' contain functional groups, these are optionally suitably protected by methods recognized by persons skilled in the art, see for example Bodanzky or Greene cited above.

Compounds having a hydrazine containing capping group attached directly to the P2 moiety, i.e. P3 and P4 are absent and G is NRjNRj, can be prepared as depicted in Scheme 13

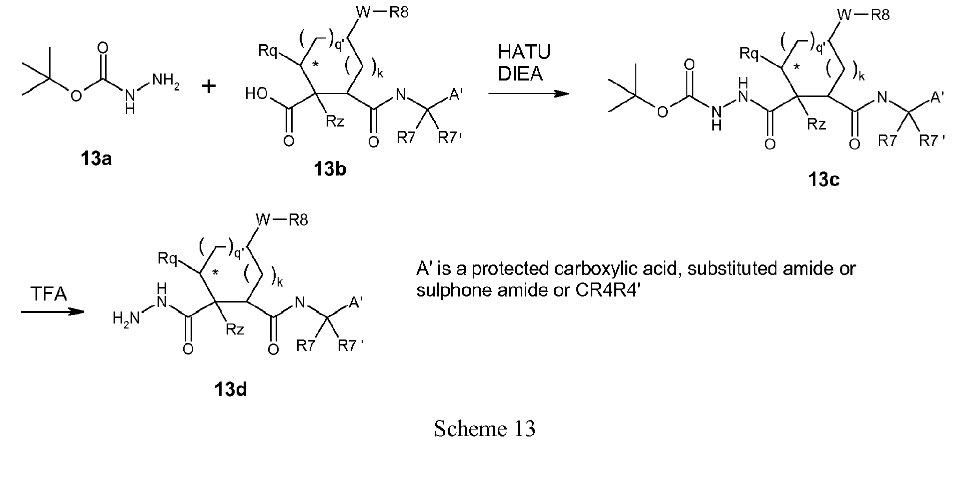

Reaction of *tert*-butyl carbazate (13a), optionally alkyl substituted on one or both nitrogens, with the acid (13b) under peptide coupling conditions like with HATU and DIEA in a solvent like DMF provides (13c). Optional removal of the boc group by standard procedures like acidic treatment with for example TFA in a suitable solvent such as dichloromethane, provides the hydrazine containing derivative (13d). Alternatively, any appropriate hydrazine derivative, such as morpholin-1-ylamine, piperidin-1-ylamine or the like can be linked to the acid (13b) instead of the *tert*-butyl carbazate derivative. The resulting compounds can then be further extended by coupling of a P3 or P4-P3 building block to the primary amine of compound 13d for example as shown in scheme 14. Treatment of the α-amino compound (14a) with sodium nitrite, potassium bromide and sulfuric acid (Yang et al. J. Org. Chem. (2001), 66, 7303-7312) provides the corresponding α-bromo compound (14b) which upon reaction with the above described derivative (13d) provides the hydrazine containing derivative (14c).

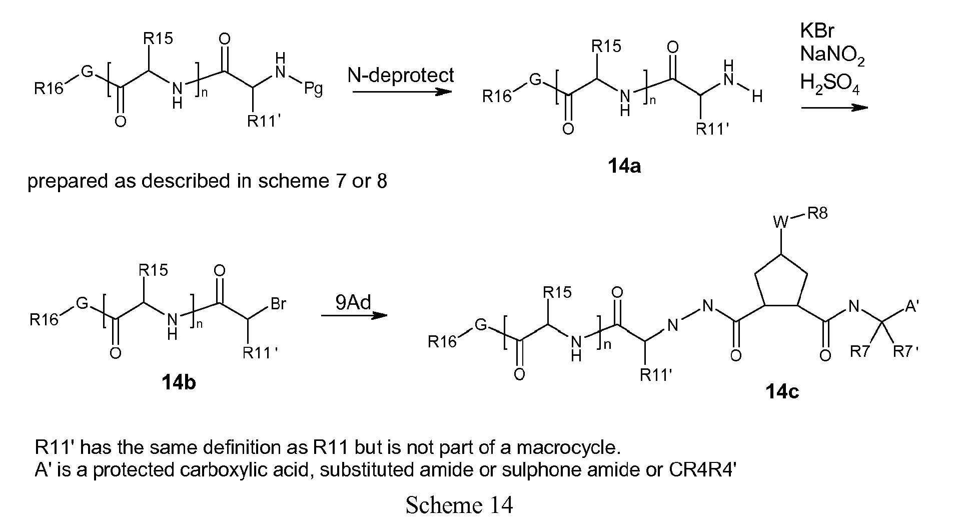

Compounds lacking a carbonyl group in the P3 unit can be prepared as illustrated in Scheme 15 exemplified with a cyclopentane derivative as P2 group.

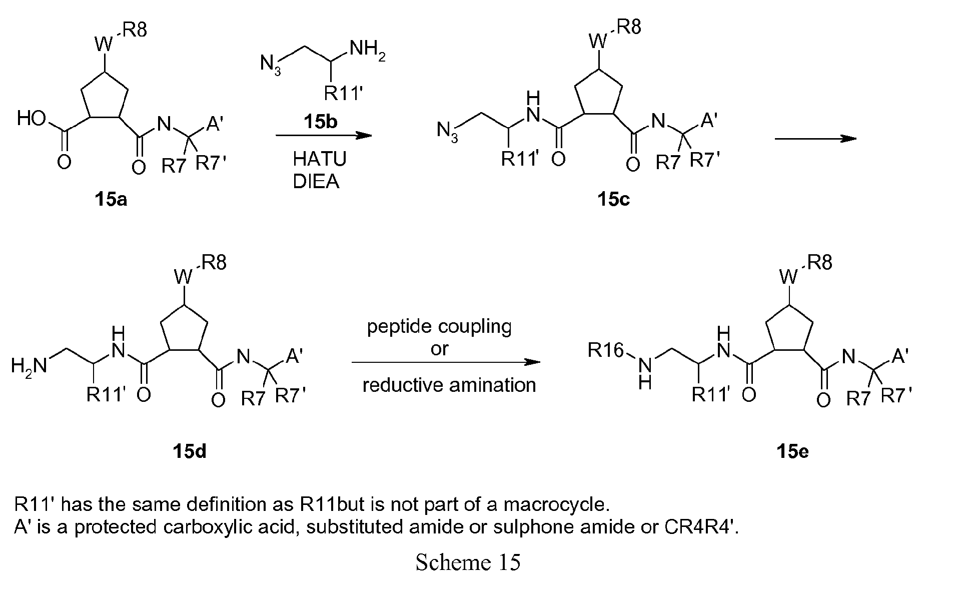

The acid (15a) can be coupled to an amino azide derivative (15b), prepared by methods known from the literature using standard peptide coupling conditions to give the amide derivative (15c). Reduction of the azide function for example by polymer bound triphenylphosphine in a solvent like methanol or any other suitable reduction method provides intermediate (15d) which subsequently can be reacted with an acid under peptide coupling conditions or with an amine in a reductive amination reaction providing amides and secondary amines respectively.

Scheme 16 shows an alternative route towards compounds lacking a carboxy group in the P3 group. Instead of using the azide derivative (15b) in scheme 15 the corresponding, optionally protected, hydroxy derivative (16b) can be used in the coupling with the acid (16a) and thus introducing a primary alcohol. The alcohol (16c) can then, after optional deprotection, be oxidized with a suitable oxidizing agent like for example Dess-Martin periodinane to form the corresponding aldehyde. Reaction of the aldehyde with a desired amine in a reductive amination reaction, using a reagent like for example polystyrene bound cyanoborohydride in a solvent like THF, provides amine derivatives (16e).

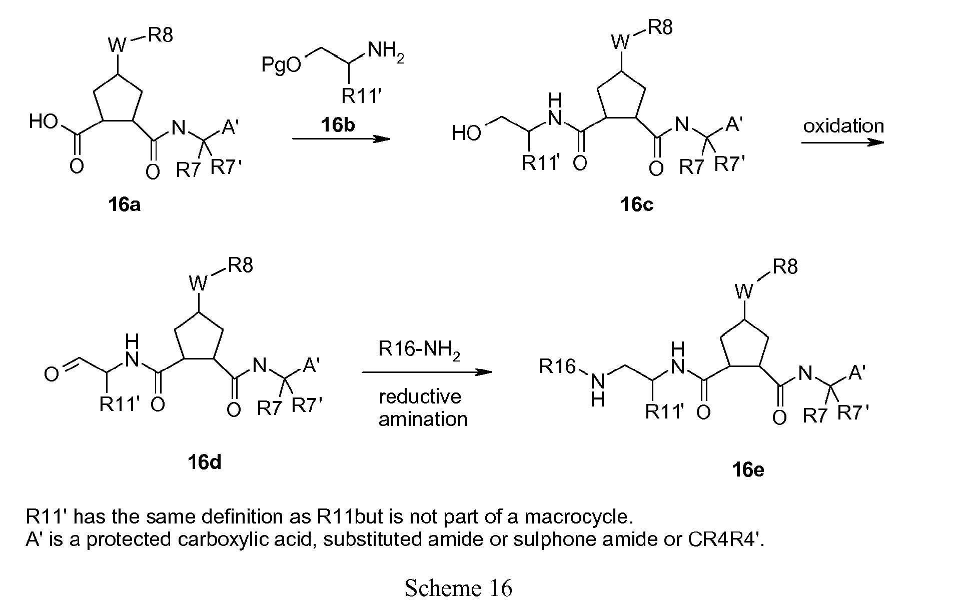

Alternatively, alcohol (16c) can be reacted with a suitable acylating or alkylating agent under the appropriate conditions to provide ester and ether compounds respectively, i.e. G is O in general formula I.

Although Scheme 15 and 16 have been described with reference to a cyclopentane derivative i.e. q' is 0 and k is 1 in Formula I, it will be readily apparent that the methodology is applicable to other compounds of the Formula I. When R⁷, R^{7'} and A' contain functional groups, these are suitably protected by art-kmown methods.

### Formation of macrocyclic compounds

Compounds according to the present invention wherein an alkylene chain extending from the R⁷/R^{7'} cycloalkyl to Rx, Rd or R¹¹, thus forming a macrocycle, can be prepared as described below. Suitable P1, P2 and P3 building blocks, or precursors thereof, are coupled together using the strategies described above, followed by a ring-closing reaction (macrocyclization). The substituent W-R⁸ of the P2 building block can be incorporated via a Mitsunobu reaction as described above, before or after formation of the macrocycle, or the assembly can be done with the required substituted proline analogue or carbocycle. For macrocyclic structures extending from the R⁷/R^{7'} cycloalkyl to R¹¹, P3 amino acids containing the appropriate side chain can be prepared as described in WO00/59929.

A typical route to macrocyclic compounds is shown in Scheme 17, which illustrates the method applied to a compound having a spiro-cyclopropyl P1, where the macrocycle incorporates the P3 side chain.

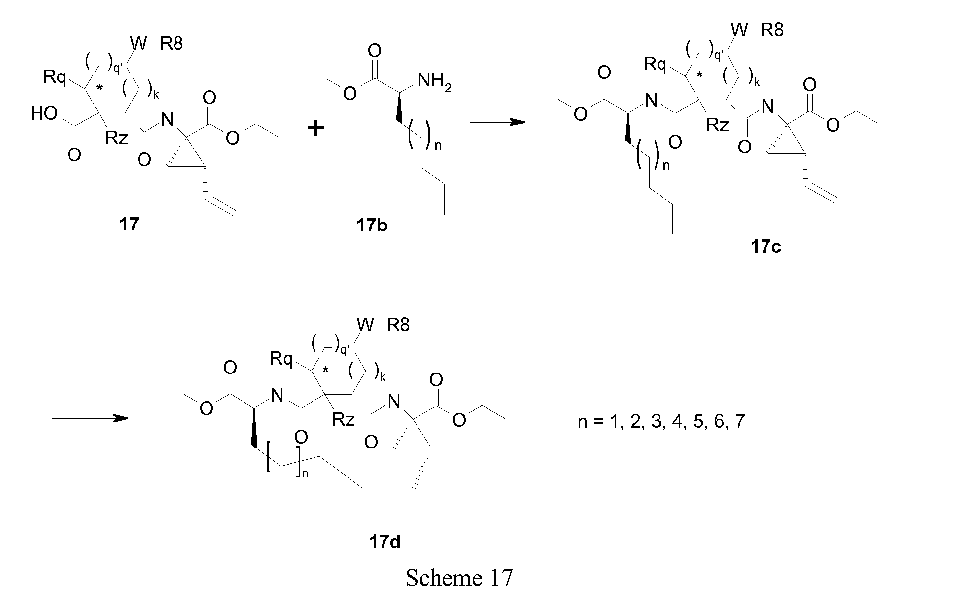

Coupling of acid derivative (17a) with the appropriate, acid protected, amino acid (17b) using standard peptide coupling conditions as described above provides (17c). Formation of the macrocycle can then be carried out via an olefin metathesis reaction using a Ru-based catalyst such as the one reported by Miller, S.J., Blackwell, H.E.; Grubbs, R.H. J. Am. Chem. Soc. 118, (1996), 9606-9614; Kingsbury, J. S., Harrity, J. P. A., Bonitatebus, P. J., Hoveyda, A. H., J. Am. Chem. Soc. 121, (1999), 791-799; and Huang et al., J. Am. Chem. Soc. 121, (1999), 2674-2678. It will also be recognized that catalysts containing other transition metals such as Mo can be used for this reaction. Optionally the double bond is reduced and/or the ethyl ester is hydrolyzed by standard hydrogenation and/or hydrolysis methods respectively well known in the art. Alternatively the methyl ester can be selectively hydrolyzed followed by coupling of a R¹⁶-G-P4 building block by standard peptide coupling conditions. The macrocyclisation step described in Scheme 17 can also be applied to the corresponding carbocyclic analogues described above. When the linker contains a nitrogen atom the ring closure can be carried out by reductive amination as described in WO00/59929.

Macrocyclic compounds without the cyclopropyl moiety in the P1 part, i.e. the macrocyclic ring extends directly from the peptidic backbone at the carbon adjacent R⁷, can be prepared using the methods described herein. An example wherein a 5 membered cycloalkyl derivative is used as the P2 scaffold is shown in scheme 18. 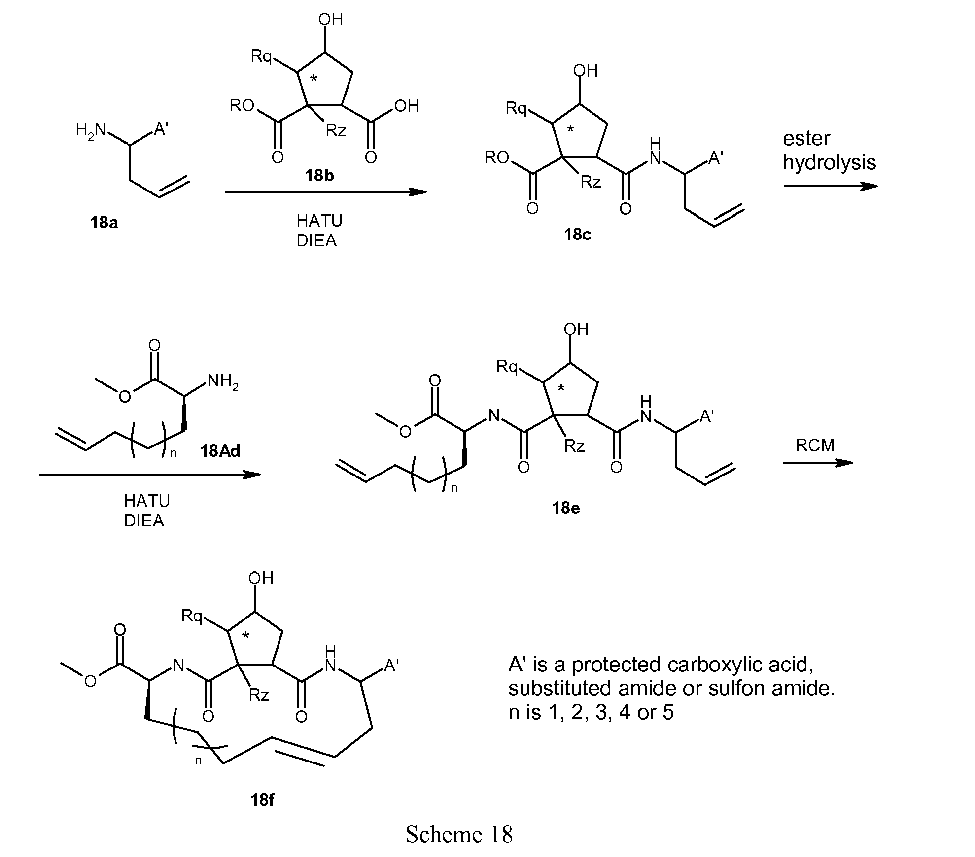 Coupling of a suitable allylglycine derivative (18a), to the acid function of the P2 scaffold (18b) using standard peptide coupling conditions yields the amide derivative (18c). Hydrolysis of the ester group followed by a peptide coupling reaction with the olefin-substituted amino acid (18Ad) provides the amide compound (18e). A ring closing metathesis reaction is then performed by using for example a Hoveyda-Grubbs catalyst, which gives the macrocyclic compound (18f). Even though scheme 18 shows the synthetic sequence using a P2 group with an unsubstituted hydroxy group, it will be apparent that the R8 substituent can be introduced at any convenient stage of the synthesis for example as described in scheme 9 and 10 or it can be introduced after the metathesis reaction, i.e. on compound 18f, using any of the methods described herein.

Building blocks to be used in the preparation of compounds wherein the macrocycle extends from the amide nitrogen in the P3 fragment i.e. Rx is J in general formula I, or in the preparation of compounds wherein the P3 and P4 fragments are absent, i.e. m and n are 0 and G is NRj in general formula I, can typically be prepared as outlined in scheme 18B.

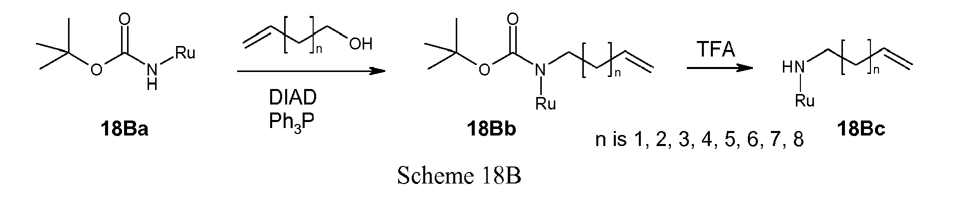

Carbamate 18Ba, which is commercially available or is readily prepared for instance by reaction of the desired alkyl amine with di-*tert*-butyl dicarbonate, can be reacted with an appropriate ω-unsaturated alcohol under Mitsunobu conditions to provide the alkylated carbamate (18Bb). Subjection of 18Bb to acidic conditions like for example treatment with trifluoroacetic acid in a solvent like dichloromethane gives the free amine (18Bc), which can be linked to a P2 fragment using any of the previously described strategies.

Macrocyclic structures containing a hydrazine group i.e. T is NRd or m and n are 0 and G is NRjNRj, in general formula I, can be prepared by linking a suitably N-alkylated carbazate derivative to the P2 fragment. Alkylated carbazate derivatives can be prepared, for example, as described in Scheme 19. 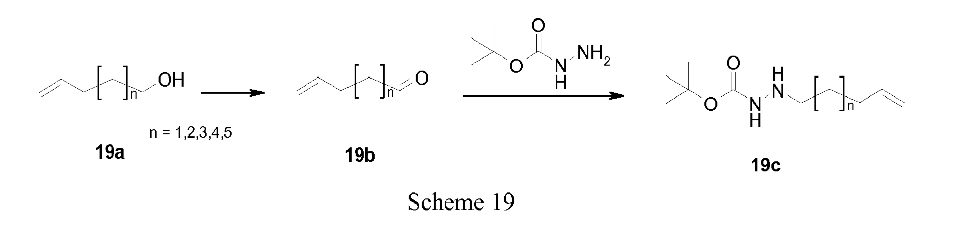 Oxidation of the appropriate alcohol (19a) effected by a suitable oxidation method like for example with N-methyl morpholine oxide and tetrapropylammonium perruthenate in a solvent like dichloromethane provides aldehyde (19b). Reductive alkylation of *tert-*butyl carbazate with the afforded aldehyde gives the desired N-alkylated building block (19c). Alternatively, any desired hydrazine derivative such as morpholin-1-ylamine, piperidin-1-ylamine or the like can be used instead of *tert*-butyl carbazate in the reaction with aldehyde 19b.

Scheme 20 illustrates synthetic sequences to building blocks suitable for the preparation of compounds wherein the "outer" nitrogen of the hydrazine group is alkylated, either with an ω-unsaturated alkyl chain appropriate for subsequent macrocycle formation, or with any other suitable alkyl group. 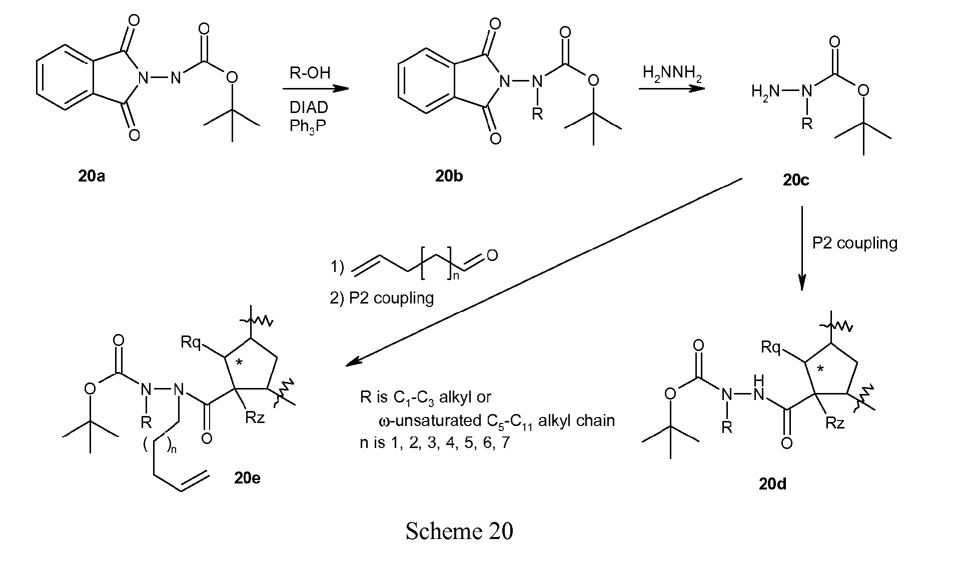 Reaction of a suitably protected hydrazine derivative, for example (1,3-dioxo-1,3-dihydro-isonidol-2-yl)-carbamic acid *tert*-butyl ester (20a), which can easily be prepared by a person skilled in the art, with a desired alcohol, R-OH, under Mitsunobu conditions provides N-alkylated hydrazine compound (20b). Removal of the phtalimido group performed by treatment with hydrazine or a derivative thereof like hydrazine hydrate or hydrazine acetate provides the carbazate (20c). The resulting primary amine can then either be coupled to any desired P2 fragment using any of the methods previously described to give (20d), or alternatively it can be further alkylated using for example the reductive amination method described in scheme 19 followed by coupling to a P2 fragment as previously described to give (20e).

Scheme 21 exemplifies the coupling of a hydrazine containing P3 building block to a cyclopentane scaffold followed by macrocyclisation. Coupling of the carbazate derivative (21b) to the P2-P1 building block (21a) using standard peptide coupling conditions provides intermediate (21c). Ring closure of (2 1 c) by an olefin metathesis reaction as described in scheme 18 gives the macrocyclic compound (21d). 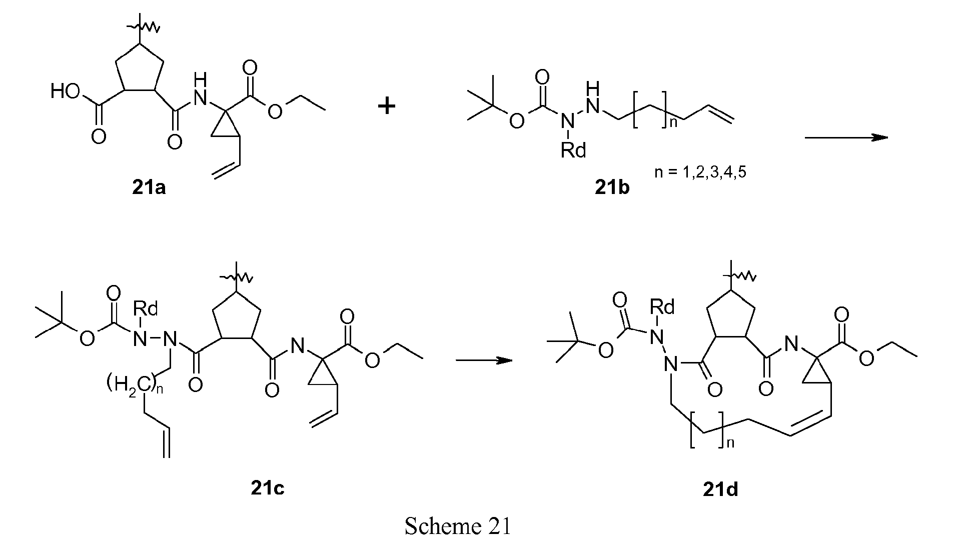 The term "N-protecting group" or "N-protected" as used herein refers to those groups intended to protect the N-terminus of an amino acid or peptide or to protect an amino group against undesirable reactions during synthetic procedures. Commonly used N-protecting groups are disclosed in Greene, "Protective Groups in Organic Synthesis" (John Wiley & Sons, New York, 1981), incorporated herein by reference. N-protecting groups include acyl groups such as formyl, acetyl, propionyl, pivaloyl, t-butylacetyl, 2-chloroacetyl, 2-bromoacetyl, trifluoracetyl, trichloroacetyl, phthalyl, o-nitrophenoxy-acetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl, and the like, carbamate forming groups such as benzyloxycarbonyl, p-chlorobenzyl-oxycarbonyl, p-methoxybenzyloxycarbonyl, p-nitrobenzyloxycarbonyl, 2-nitrobenzyl-oxycarbonyl, p-bromobenzyloxycarbonyl, 3,4-dimethoxybenzyloxy-carbonyl, 4-methoxybenzyloxycarbonyl, 2-nitro-4,5-dimethoxybenzyloxycarbonyl, 3,4,5-trimethoxybenzyloxy-carbonyl, 1-(p-biphenylyl)-1-methylethoxycarbonyl, α,α-dimethyl-3,5-dimethoxybenzyloxycarbonyl, benzhydryloxycarbonyl, t-butoxycarbonyl, diisopropylmethoxy-carbonyl, isopropyloxycarbonyl, ethoxycarbonyl, methoxycarbonyl, allyloxycarbonyl, 2,2,2-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl, and the like; alkyl gropus such as benzyl, triphenylmethyl, benzyloxymethyl and the like; and silyl groups such as trimethylsilyl and the like. Favored N-protecting groups include Fmoc, formyl, acetyl, benzoyl, pivaloyl, t-butylacetyl, phenylsulfonyl, benzyl, t-butoxycarbonyl (BOC) and benzyloxycarbonyl (Cbz).

Hydroxy protecting group as used herein refers to a substituent that protects hydoxygroups against undesirable reactions during synthetic procedures such as those O-protecting groups disclosed in Greene, "Protective Groups In Organic Synthesis," (John Wiley & Sons, New York (1981)). Hydroxy protecting groups comprise substituted methyl ethers, for example, methoxymethyl, benzyloxymethyl, 2-methoxyethoxy-methyl, 2-(trimethylsilyl)ethoxymethyl, t-butyl and other lower alkyl ethers, such as isopropyl, ethyl and especially methyl, benzyl and triphenylmethyl; tetrahydropyranyl ethers; substituted ethyl ethers, for example, 2,2,2-trichloroethyl; silyl ethers, for example, trimethylsilyl, t-butyldimethylsilyl and t-butyldiphenylsilyl; and esters prepared by reacting the hydoxygroup with a carboxylic acid, for example, acetate, propionate, benzoate and the like.

In treating conditions caused by flavivirus such as HCV, the compounds of Formula I are typically administered in an amount to achieve a plasma level of around 100 to 5000 nM, such as 300 to 2000 nM. This corresponds to a dosage rate, depending on the bioavailability of the formulation, of the order 0.01 to 10 mg/kg/day, preferably 0.1 to 2 mg/kg/day. A typical dosage rate for a normal adult will be around 0.05 to 5 g per day, preferably 0.1 to 2 g such as 500-750 mg, in one to four dosage units per day. As with all pharmaceuticals, dosage rates will vary with the size and metabolic condition of the patient as well as the severity of the infection and may need to be adjusted for concomitant medications.

As is good prescribing practice with antiviral therapy, the compounds of Formula I are typically coadministered with other HCV therapies to avoid the generation of drug escape mutants. Examples of such additional HCV antiviral therapies include ribavirin, interferons, including pegylated interferons. Additionally a number of nucleoside analogues and protease inhibitors are in clinical or preclinical development and will be amenable to co-administration with the compounds of the invention.

Accordingly a further aspect of the invention provides a composition comprising a compound of Formula I and at least one further HCV antiviral in a common dosage unit, such as any of the dosage forms described below, but especially an orally administered tablet, or capsule or a liquid suspension or solution for oral or injection use. A further aspect of the invention provides a method for the treatment or prophylaxis of flavivirus infection, such as HCV, comprising the sequential or simultaneous administration of a compound of Formula I and at least one further HCV antiviral. A related aspect of the invention provides a patient pack comprising a first pharmaceutical composition, preferably in unit dosage form, of the compound of Formula I and a second pharmaceutical composition of a second HCV antiviral, typically also in unit dosage form and generally in a separate container within the patient pack. A patient pack will conveniently also be provided with instructions printed on the package or a container therein, or on a package insert, for the simultaneous or sequential administration of the respective pharmaceutical compositions.

Many HCV patients are co-infected, or prone to superinfection, with other infectious diseases. Accordingly, a further aspect of the invention provides combination therapies comprising the compound of the invention co-formulated in the same dosage unit or co-packaged with at least one further anti-infective pharmaceutical. The compound of the invention and the at least one further anti-infective are administered simultaneously or sequentially, typically at doses corresponding to the monotherapy dose for the agent concerned. However, certain anti-infectives can induce a synergistic response, allowing one or both of the active ingredients to be administered at a lower dose than the corresponding monotherapy. For example in drugs prone to rapid metabolism by Cyp3A4, co-dosing with the HIV protease inhibitor ritonavir can allow lower dosage regimes to be administered. Typical coinfections or superinfections with HCV include hepatitis B virus or HIV. Accordingly the compound of the invention is advantageously co-administered (either in the same dosage unit, co-packaged or separately prescribed dosage unit) with at least one HIV antiviral and/or at least one HBV antiviral.

Representative HIV antivirals include NRTI such as alovudine (FLT), zidovudine (AZT, ZDV), stavudine (d4T, Zerit), zalcitabine (ddC), didanosine (ddI, Videx), abacavir, (ABC, Ziagen), lamivudine (3TC, Epivir), emtricitabine (FTC, Emtriva), racevir (racemic FTC), adefovir (ADV), entacavir (BMS 200475), alovudine (FLT), tenofovir disoproxil fumarate (TNF, Viread), amdoxavir (DAPD), D-d4FC (DPC-817), -dOTC (Shire SPD754), elvucitabine (Achillion ACH-126443), BCH 10681 (Shire) SPD-756, racivir, D-FDOC, GS7340, INK-20 (thioether phospholipid AZT, Kucera), 2'3'-dideoxy-3'-fluoroguanosine (FLG) and its prodrugs such as MIV-210, reverset (RVT, D-D4FC, Pharmasset DPC-817). Representative NNRTI include delavirdine (Rescriptor), efavirenz (DMP-266, Sustiva), nevirapine (BIRG-587, Viramune), (+)calanolide A and B (Advanced Life Sciences), capravirine (AG1549f S-1153; Pfizer), GW-695634 (GW-8248; GSK), MIV-150 (Medivir), MV026048 (R-1495; Medivir AB/Roche), NV-05 2 2 (Idenix Pharm.), RS-1588 (Idenix Pharm.), TMC120, TMC125, TMC278, UC-781 (Biosyn Inc.) and YM215389 (Yamanoushi). Representative HIV protease inhibitors include PA-457 (Panacos), KPC-2 (Kucera Pharm.), 5 HGTV-43 (Enzo Biochem), amprenavir (VX-478, Agenerase), atazanavir (Reyataz), indinavir sulfate (MK-639, Crixivan), Lexiva (fosamprenavir calcium, GW -433908 or 908, VX-175), ritonavir (Norvir), lopinavir + ritonavir (ABT-378, Kaletra), tipranavir, nelfinavir mesylate (Viracept), saquinavir (Invirase, Fortovase), AG1776 (JE-2147, KNI-764; Nippon Mining Holdings), AG-1859 (Pfizer), DPC-681/684 (BMS), GS224338 (Gilead Sciences), KNI-272 (Nippon Mining Holdings), Nar-DG-35 (Narhex), P(PL)-100 (P-1946; Procyon Biopharma), P-1946 (Procyon Biopharma), R-944 (Hoffmann-LaRoche), RO-0334649 (Hoffmann-LaRoche), TMC114, VX-385 (GW640385; GSK/Vertex), VX-478 (Vertex/GSK). Other HIV antivirals include entry inhibitors, including fusion inhibitors, inhibitors of the CD4 receptor, inhibitors of the CCR5 co-receptor and inhibitors of the CXCR4 coreceptor, or a pharmaceutically acceptable salt or prodrug thereof. Examples of entry inhibitors are AMD-070 (AMD11070; AnorMed), BlockAide/CR (ADVENTRX Pharm.), BMS 806 (BMS-378806; BMS), Enfurvirtide (T-20, R698, Fuzeon), KRH1636 (Kureha Pharmaceuticals), ONO-4128 (GW-873140, AK-602, E-913; ONO Pharmaceuticals), Pro-140 (Progenies Pharm), PRO542 (Progenics Pharm.), SCH-D (SCH-417690; Schering-Plough), T-1249 (R724; Roche/Trimeris), TAK-220 (Takeda Chem. Ind.), TNX-355 (Tanox) and UK-427,857 (Pfizer). Examples of integrase inhibitors are L-870810 (Merck & Co.), c-2507 (Merck & Co.) and S(RSC)-1838 (shionogi/GSK).

Examples of HBV antivirals include adefovir dipivoxil (Hepsera), and especially lamivudine and 2'3'-dideoxy-3'-fluoroguanosine (FLG) and its prodrugs such as MIV-210, the 5'-O-valyl-L-lactyl prodrug of FLG. These latter HBV antivirals are particularly convenient as they are also active against HIV.

While it is possible for the active agent to be administered alone, it is preferred to present it as part of a pharmaceutical formulation. Such a formulation will comprise the above-defined active agent together with one or more acceptable carriers or excipients and optionally other therapeutic ingredients. The carrier(s) should be acceptable in the sense of being compatible with the other ingredients of the formulation and not deleterious to the recipient.

The formulations include those suitable for rectal, nasal, topical (including buccal and sublingual), vaginal or parenteral (including subcutaneous, intramuscular, intravenous and intradermal) administration, but preferably the formulation is an orally administered formulation. The formulations may conveniently be presented in unit dosage form, e.g. tablets and sustained release capsules, and may be prepared by known methods.

Such methods include the step of bringing into association the above-defined active agent with the carrier. In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of Formula I or its pharmaceutically acceptable salt in conjunction or association with a pharmaceutically acceptable carrier or vehicle. If the manufacture of pharmaceutical formulations involves intimate mixing of pharmaceutical excipients and the active ingredient in salt form, then it is often preferred to use excipients that are non-basic in nature, i.e. either acidic or neutral. Formulations for oral administration in the present invention may be presented as discrete units such as capsules, cachets or tablets each containing a predetermined amount of the active agent; as a powder or granules; as a solution or a suspension of the active agent in an aqueous liquid or a nonaqueous liquid; or as an oil-in-water liquid emulsion or a water in oil liquid emulsion and as a bolus etc.

With regard to compositions for oral administration (e.g. tablets and capsules), the term suitable carrier includes vehicles such as common excipients e.g. binding agents, for example syrup, acacia, gelatine, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatine, lactose, sucrose, microcrystalline cellulose, kaolin, mannitol, dicalcium phosphate, sodium chloride and alginic acid; and lubricants such as magnesium stearate, sodium stearate and other metallic stearates, stearic acid, glycerol stearate, silicone fluid, talc waxes, oils and colloidal silica. Flavouring agents can be added. It may be desirable to add a colouring agent to make the dosage form readily identifiable. The tablets may be optionally be coated or scored and may be formulated so as to provide slow or controlled release of the active agent. Other formulations suitable for oral administration include lozenges comprising the active agent in a flavored base, usually sucrose and acacia or tragacanth; pastilles comprising the active agent in an inert base such as gelatin and glycerin, or sucrose and acacia; and mouthwashes comprising the active agent in a suitable liquid carrier.

### Examples

Various end products and intermediates towards the synthesis thereof are described by way of illustration and should not be interpreted as consisting of a limitation of the scope of this invention.

### Example 1: 7-Methoxy-2-phenyl-quinolin-4-ol (1)

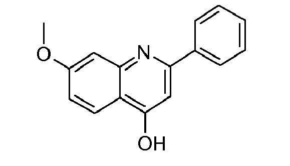

To a stirred mixture of toluene (100 mL), ethyl benzoyl acetate (18.7 g, 97 mmol) and *m*-anisidine (12 g, 97 mmol) were added. 4 M HCl in dioxane (0.5 mL) and the reaction mixture was refluxed for 6 h (140°C). The mixture was co-evaporated with toluene. Diphenyl ether (50 mL) was added to the crude mixture and the mixture was heated to 280°C for 2 h. When the theoretical amount of ethanol (6 mL) was collected in a Dean Stark trap, the heating was stopped and the mixture was cooled to RT. The crude mixture was dissolved in CH₂Cl₂ (100 mL) and stirred for 30 min. The formed precipitate was filtered off and dried which gave **1** (4.12 g, 16.4 mmol, 17 %): pale yellow powder.
¹H (300 MHz, DMSO-D₆): δ 3.8 (s, 3H), 6.24 (s, 1H), 6.88-6.96 (dd, 1H, *J* = 9.07 Hz, *J* = 2.47 Hz), 7.19 (d, 1H, *J*=2.19 Hz), 7.56 (t, 3H, *J* = 2.19 Hz), 7.8 (dd, 2H, *J* = 7.14 Hz, *J* = 2.19 Hz), 8.0 (d, 1H, *J* = 9.06 Hz); ¹³C (75.5 MHz, DMSO-D₆): δ 55.3, 99.6, 106.9, 113.1, 119.1, 126.4, 127.5, 128.8, 130.2, 134.1, 142.2, 149.4, 161.8, 176.4.

### Example 2: (Rac)-4-oxocyclopent-2-ene-1, 2-dicarboxylic acid dimethyl ester (2)

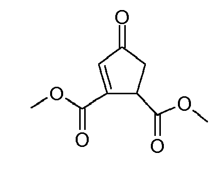

(1*R*, 2*S*)-4-oxo-cyclopentane-1, 2-dicarboxylic acid dimethyl ester (4.8 g, 23.8 mmol) and CuBr₂ (11.9 g, 53.2 mmol) were dissolved in dry THF (70 mL) and the mixture was refluxed for two hours at 90 °C. The formed CuBr was filtered off and the organic phase was concentrated. CaCO₃ (2.7 g, 27.2 mmol) and DMF (70 mL) were added and the mixture was held at 100 °C for one hour. The dark brown mixture was poured over ice (35 g) and the formed precipitate was filtered off. The aqueous layer was extracted with ethyl acetate (1 x 300mL + 3 x 150 mL). The organic phases were dried, filtrated and concentrated. Purification by flash chromatography (toluene/EtOAc 9:1) gave **2** (2.1 g, 45 %) as yellow crystals

### Example 3: ((1S,4R) and (1R,4S))-4-hydroxy-cyclopent-2-ene-1,2-dicarboxylic acid

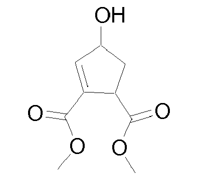

dimethyl ester (3)
To a cold solution (-30 °C) of 2 (3.18 g, 16.1 mmol) dissolved in MeOH (23 mL), NaBH₄ (0.66 g, 17.5 mmol) was added. After nine minutes the excess of NaBH₄ was destroyed by adding brine (80 mL). The mixture was concentrated and extracted with ethyl acetate (4 x 80 mL). The organic phases were dried, filtrated and concentrated and gave **3** (3.0 g, 92 %) as a yellow oil.

### Example 4: (1S,4R) and (1R,4S)-4-hydroxy-cyclopent-2-ene-1,2-dicarboxylic acid 2-methyl ester (4)

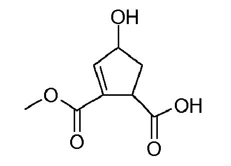

To an ice-cold solution of **3** (3.4 g, 22 mmol) dissolved in dioxane and water (1:1, 110mL), LiOH (0.52 g, 22 mmol) was added. After two and a half hours the mixture was co-evaporated with toluene and methanol. Purification by flash chromatography (toluene/Ethyl acetate 3:1 + 1 % HOAc) gave the title compound **4** (1.0 g, 27 %) as yellow-white crystals. ¹H-NMR (300 MHz, CD₃OD): δ 1.78-1.89 (m, 1H), 2.70-2.84 (m, 1H), 3.56-3.71 (m, 1H), 3.76 (s, 3H), 4.81-4.90 (m, 1H), 6.76-6.81 (m, 1H); ¹³C-NMR (75.5 MHz, CDCl₃): δ 38.0, 48.0, 52.4, 75.7, 137.0, 146.2, 165.0 178.4.

### Example 5: trans-(3R,4R)-Bis(methoxycarbonyl)cyclopentanol (5)

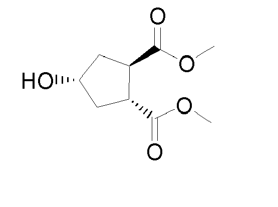

Sodium borohydride (1.11 g, 0.029 mol) was added to a stirred solution of (1R, 2S)-4-oxo-cyclopentane1,2-dicarboxylic acid dimethyl ester (4.88 g, 0.0244 mol) in methanol (300 mL) at 0 °C. After 1 h, the reaction was quenched with 90 mL brine, concentrated and extracted with ethyl acetate. The organic phases were pooled, dried, filtered and concentrated. The crude product was purified by flash column chromatography (toluene/ethyl acetate 1:1) to give **5** (3.73 g, 76%) as a yellow oil.

### Example 6: 3-Oxo-2-oxa-bicyclo[2.2.1]heptane-5-carboxylic acid (6)

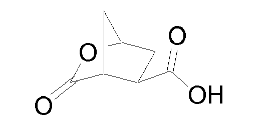

Sodium hydroxide (1M, 74 mL, 0.074 mol) was added to a stirred solution of **5** (3.73 g, 0.018 mol) in methanol (105 mL) at room temperature. After 4 h, the reaction mixture was neutralized with 3M HCl, evaporated and co-evaporated with toluene several times. Pyridine (75 mL) and Ac₂O (53 mL) were added and the reaction mixture was allowed to shake overnight at room temperature. The mixture was then co-evaporated with toluene and purified by flash column chromatography (ethyl acetate + 1% acetic acid) to give **6** (2.51 g, 88%) as a yellow oil.

### Example 7: 3-Oxo-2-oxa-bicyclo[2.2.1]heptane-5-carboxylic acid tert-butyl ester (7)

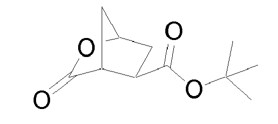

DMAP (14 mg, 0.115 mmol) and Boc₂O (252 mg, 1.44 mmol) was added to a stirred solution of **6** (180 mg, 1.15 mmol) in 2 mL CH₂Cl₂ under inert argon atmosphere at 0 °C. The reaction was allowed to warm to room temperature and was stirred overnight. The reaction mixture was concentrated and the crude product was purified by flash column chromatography (toluene/ethyl acetate gradient 15:1, 9:1, 6:1, 4:1, 2:1) to give **7** (124 mg, 51%) as white crystals. ¹H-NMR (300 MHz, CD₃OD) δ 1.45 (s, 9H), 1.90 (d, *J* = 11.0 Hz, 1H), 2.10-2.19 (m, 3H), 2.76-2.83 (m, 1H), 3.10 (s, 1H), 4.99 (s, 1H); ¹³C-NMR (75.5 MHz, CD₃OD) δ 27.1, 33.0, 37.7, 40.8, 46.1, 81.1, 81.6, 172.0, 177.7.

### Example 8: Boc-L-tert-leucine-OH (8)

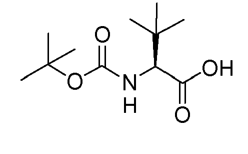

Triethylamine (890 uL, 6.40 mmol) was added dropwise to a stirred solution of L-*tert-*leucine (300 mg, 2.29 mmol) and di-*tert*-butyl dicarbonate (599 mg, 2.74 mmol) in dioxane/ water 1:1 (8 mL) and the solution was stirred overnight. The mixture was extracted with petroleum ether (2×) and the aqueous phase was cooled to 0°C and carefully acidified to pH 3 by slow addition of 4M NaHSO₄·H₂O. The acidified water phase was extracted with EtOAc (3×) and the combined organic phases were washed with brine (2×) and was then dried, filtered and concentrated to give the title compound **8** (522 mg, 99 %) as a colorless powder. No further purification was needed.
¹H-NMR (300 MHz, CD₃OD) δ 0.99 (s, 9H), 1.44 (s, 9H), 3.96 (s, 1H); ¹³C-NMR (75.5 MHz, CD₃OD) δ 27.1, 28.7, 34.9, 68.0, 80.5, 157.8, 174.7.

### Example 9: ((S)-Cyclohexyl-methylcarbamoyl-methyl)-carbamic acid tert-butyl ester (9)

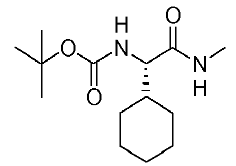

Boc-Chg-OH (387 mg, 1.50 mmol) was coupled to methylamine hydrochloride (111 mg, 1.65 mmol) using HATU coupling conditions, similar as described in example 14. The crude product was extracted with EtOAc, washed with brine and concentrated. Purification by flash column chromatography (EtOAc) provided the title compound 9 (307 mg, 76 %) as a colorless solid. ¹H-NMR (300 MHz, CDCl₃) δ 0.91-1.13 (m, 2H), 1.14-1.31 (m, 3H), 1.44 (s, 9H), 1.61-1.80 (m, 6H), 2.80 (d, *J* = 4.7 Hz, 3H), 3.91 (dd, *J* = 7.1, 9.1 Hz, 1H), 5.23 (b, 1H), 6.52 (bs, 1H); ¹³C-NMR (75.5 MHz, CDCl₃) δ 25.9, 26.0, 26.1, 28.3, 28.5, 29.6, 40.5, 59.5, 79.7, 155.9, 172.4.

### Example 10: {(S)-1-[((S)-Cyclohexyl-methylcarbamoyl-methyl)-carbamoyl]-2,2-dimethyl-propyl}-carbamic acid tert-butyl ester (10)

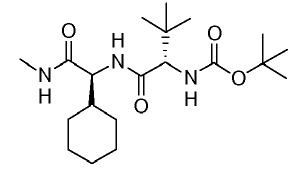

To a solution of compound **9** (98 mg, 0.362 mmol) in methylene chloride (3 mL) were added triethylsilane (115 mL, 0.742 mmol) and TFA (3 mL). The mixture was stirred for 2 h at room temperature and was then evaporated and coevaporated with toluene. The deprotected amine was dissolved in DMF (5 mL) and coupled to compound 36 (84 mg, 0.363 mmol) using HATU coupling conditions as in the synthesis of 14. The crude product was extracted with EtOAc, washed with brine, dried, filtered and concentrated. Purification by flash column chromatography (toluene/ EtOAc 1:1) provided the title compound **10** (128 mg, 92 %) as a colorless solid. ¹H-NMR (300 MHz, CDCl₃) δ 0.99 (s, 9H), 1.02-1.30 (m, 5H), 1.44 (s, 9H), 1.58-1.77 (m, 4H), 1.78-1.89 (m, 2H), 2.79 (d, *J* = 4.7 Hz, 3H), 4.11 (d, *J* = 9.3 Hz, 1H), 4.33 (app. t, *J* = 8.5 Hz, 1H), 5.65 (b, 1H), 7.25 (b, 1H), 7.39 (b, 1H); ¹³C-NMR (75.5 MHz, CDCl₃) δ 25.9, 25.9, 26.0, 26.2, 26.8, 28.4, 29.0, 29.7, 34.5, 39.7, 58.4, 62.4, 79.4, 156.0, 171.4, 171.8.

### Example 11: 3-Oxo-2-oxa-bicyclo[2.2.1]heptane-5-carboxylic acid methyl ester (11)

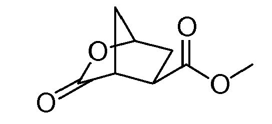

Compound **6** (1.014 g, 6.50 mmol) was dissolved in acetone (35 mL) before methyl iodide (13.68 g, 96.4 mmol) and silver(I)oxide (1.61 g, 6.95 mmol) were added. After stirring for 3 h, the mixture was filtered through celite and the filtrate was evaporated before purification by flash column chromatography (toluene/ethyl acetate 4:1) was performed yielding the methyl ester **11** (702 mg, 64 %) as white crystals.
¹H-NMR (300 MHz, CDCl₃): δ 1.96 (d, *J* = 10.7 Hz, 1H), 2.21-2.25 (m, 3H), 2.91-2.95 (m, 1H), 3.16 (s, 1H), 3.75 (s, 3H), 4.98 (app. s, 1H).

### Example 12: Hept-6-enal (12)

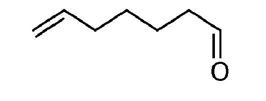

To a solution of hept-6-en-1-ol (1 mL, 7.44 mmol) and N-methylmorpholine N-oxide (1.308 g, 11.17 mmol) in DCM (17 mL) was added ground molecular sieves (3.5 g, 4 Å). The mixture was stirred for 10 min at room temperature under nitrogen atmosphere before tetrapropylammonium perruthenate (TPAP) (131 mg, 0.37 mmol) was added. After stirring for an additional 2.5 h, the solution was filtered through celite. The solvent was then carefully evaporated and the remaining liquid was purified by flash column chromatography (DCM) to give the volatile aldehyde **12** (620 mg, 74%) as an oil.

### Example 13: N'-Hept-6-en-(E)-ylidene-hydrazinecarboxylic acid tert-butyl ester (13)

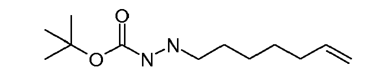

To a solution of **12** (68 mg, 0.610 mmol) and *tert*-butyl carbazate (81 mg, 0.613 mmol) in MeOH (5 mL) was added ground molecular sieves (115 mg, 31Å). The mixture was stirred for 3 h after which it was filtered through celite and evaporated. The residue was dissolved in dry THF (3 mL) and AcOH (3mL). NaBH₃CN (95 mg, 1.51 mmol) was added and the solution was stirred over night. The reaction mixture was diluted with saturated NaHCO₃ solution (6 mL) and EtOAc (6 mL). The organic phase was consecutively washed with brine, saturated NaHCO₃, brine, dried over MgSO₄ and evaporated. The cyanoborane adduct was hydrolyzed by treatment with MeOH (3 mL) and 2 M NaOH (1.9 mL). The mixture was stirred for 2 h and the MeOH was evaporated. H₂O (5 mL) and DCM (5 mL) were added and the water phase was extracted three times with DCM. The combined organic phases were dried and evaporated. Purification by flash column chromatography (toluene/ethyl acetate 9:1 with 1 % triethylamine and toluene/ethyl acetate 6:1 with 1 % triethylamine) provided the title compound **13** (85 mg, 61 %) as an oil.

### Example 14: ((S)-1-Cyclopentylcarbamoyl-2,2-dimethyl-propyl)-carbamic acid tert-butyl ester (14)

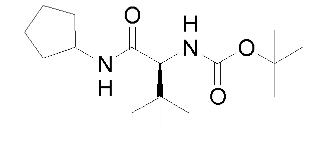

To a cold solution of **8** (133 mg, 0.575 mmol), cyclopentylamine (64 µL, 0.648 mmol) and DIEA (301 µL, 1.73 mmol) in DMF (3 mL), was added the coupling reagent HATU (240 mg, 0.631 mmol). The mixture was stirred cold for half an hour and for an additional two hours at room temperature. The solvent was removed by heating the reaction flask in a water bath under diminished pressure and the residue was dissolved in ethyl acetate, after which the organic phase was washed three times with brine, dried, filtered and evaporated. Purification by flash column chromatography (toluene/ethyl acetate 4:1) provided the title compound **14** (140 mg, 82 %) as colorless crystals.
¹H-NMR (300 MHz, CDCl₃): δ 0.95 (s, 9H), 1.28-1.48 (m, overlapped, 2H), 1.40 (s, 9H), 1.49-1.71 (m, 4H), 1.86-2.01 (m, 2H), 3.76 (b, 1H), 4.09-4.23 (m, 1H), 5.32 (b, 1H), 5.91 (b, 1H); ¹³C-NMR (75.5 MHz, CDCl₃): δ 23.6, 23.7, 26.5, 28.3, 32.6, 33.1, 34.5, 51.0, 62.2, 79.4, 155.9, 170.3.

### Example 15: (S)-tert-Butoxycarbonylamino-cyclohexyl-acetic acid methyl ester (15)

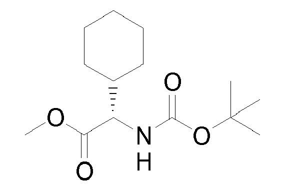

To a solution of Boc-Chg-OH (53 mg, 0.206 mmol) in acetone (3 mL) were added methyl iodide (195 µL, 3.1 mmol) and silver (I) oxide (53 mg, 0.229 mmol). The mixture was allowed to stir over night in a reaction flask that was covered with aluminium foil. Thereafter the solution was filtered through celite and evaporated. Purification by flash column chromatography (toluene/ethyl acetate 15:1) provided methyl ester **15** (56 mg, 100 %) as a colorless oil.
¹H-NMR (300 MHz, CDCl₃): δ 1.00-1.34 (m, 5H), 1.44 (s, 9H), 1.54-1.82 (m, 6H), 3.73 (s, 3H), 4.20 (dd, *J* = 2.8, 5.0 Hz, 1H), 5.05 (bs, 1H); ¹³C-NMR (75.5 MHz, CDCl₃): δ 26.0, 28.2, 28.3, 29.5, 41.1, 52.0, 58.3, 79.7, 155.6, 172.9.

### Example 16: (S)-((S)-2-Benzyloxycarbonylamino-3-methyl-butyrylamino)-cyclohexyl-acetic acid methyl ester (16)

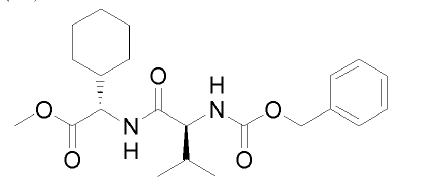

Compound **15** (93 mg, 0.343 mmol) was deprotected and coupled to Z-Val-OH (95 mg, 0.378 mmol) according to the method for the preparation of 14. Flash column chromatography (toluene/ethyl acetate 4:1) gave the title compound **15** (131 mg, 94 %) as a colorless solid. ¹H-NMR (300 MHz, CDCl₃): δ 0.92-1.30 (m, 11H), 1.54-1.88 (m, 6H), 2.02-2.18 (m, 1H), 3.72 (s, 3H), 4.05-4.18 (m, 1H), 4.52 (dd, J = 3.0, 5.5 Hz, 1H), 5.12 (s, 2H), 5.49 (bs, 1H), 6.52 (bs, 1H), 7.34 (s, 5H); ¹³C-NMR (75.5 MHz, CDCl₃): δ 17.8, 19.0, 25.8, 28.2, 29.3, 31.2, 40.5, 51.9, 56.8, 60.0, 66.8, 127.7, 127.9, 128.1, 128.3, 136.2, 156.3, 171.3, 172.2.

### Example 17: Resin bound 2-tert-butoxycarbonylamino-3,3-dimetylbutyric acid (17)

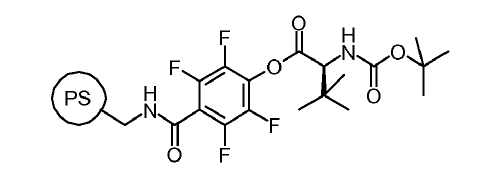

To Argonaut resin PS-TFP (1.38 mmol/g, 10 g) and 2-*tert*-butoxycarbonylamino-3,3-dimethyl-butyric acid (4.5 g, 20.7mmol) was added dichloromethane (40 mL) and DMF (10 mL). DMAP (1 g, 8.28 mmol) and then DIC (9.5 mL, 60.7 mmol) were added to this mixture. After 3 hrs of agitation at RT, the resin was filtered and washed successively with DMF, THF, DCM, THF, DCM and ether and then dried in a vacuum.

### Example 18: [1-(2-Hydroxy-indan-1-ylcarbamoyl)-2,2-dimethyl-propyl]-carbamic acid tert-butyl ester (18)

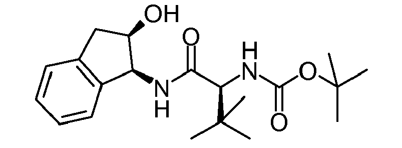

To a portion of **17** (200 mg) in DCM, aminoindanol (0.14 mmol) was added. The mixture was agitated for 2 hrs. The liquid was filtered of and the resin washed with 2xDCM. The combined liquids were combined and concentrated to dryness to afford the title compound **18** (20.5 mg, 0.055 mmol). Purity >95% by HPLC. M+H⁺ 363.15. ¹³C NMR δ_{C}(100 MHz; CDCl₃; Me₄Si) 27.0, 28.5, 34.2, 39. 8, 50.8, 57.9, 68.2, 73.7, 124.8, 125.6, 127.4, 128.5, 140.4, 171.6. ¹H NMR δ_{H} (400 MHz; CDCl₃; Me₄Si) 1.07 (9H, s, CCH₃), 1.44 (9H, s, OCCH₃), 2.93 (1H, dd, *J*_{gem}16.4 Hz, *J*_{3,2} 2.3 Hz, CH₂), 3.15 (1H, dd, *J*_{gem}16.4 Hz, *J*_{3,2} 5.2 Hz, CH₂),

### Example 19: 2-Amino-N-(2-hydroxy-indan-1-yl)-3,3-dimethyl butyramide (19)

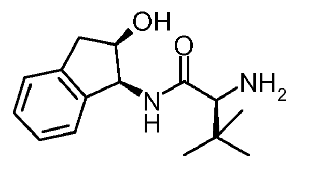

Compound **18** was kept in DCM-TFA 2:1 (2 mL) for 60 min at RT. The solution was co-evaporated with toluene to dryness, which gave the title compound **19**.

### Example 20: (2-tert-Butoxycarbonylamino-3,3-dimethyl-butyrylamino)-cyclohexyl-acetic acid methyl ester (20)

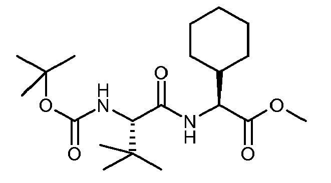

To a solution of 2-*tert*-butoxycarbonylamino-3,3-dimethyl butyric acid (500 mg, 2.16 mmol), amino-cyclohexyl-acetic acid methyl ester (444 mg, 2.59 mmol) and HATU (2 g, 5.40 mmol) in DMF (20 mL), was added diisopropylethylamine (1.88 mL, 10.8 mmol). The solution was stirred for 1 hrs at RT and diluted with dichloromethane (40 mL). This solution was washed with aqueous. NaHCO₃ (sat.) and water (x2), dried and concentrated. The product **20** was >95 % pure. M+H⁺ 385.4.

### Example 21: {1-[(Cyclohexyl-methylcarbamoyl-methyl)-carbamoyl]-2,2-dimethylpropyl}-carbamic acid tert-butyl ester (21)

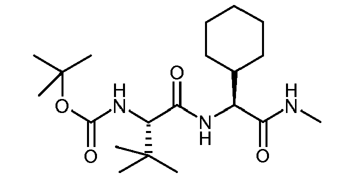

To compound **20** in EtOH-THF 1:2, was added a large excess of methylamine (30% in water) and left at RT for 2 weeks. The solution was concentrated to dryness and the residue subjected to a short silica gel column eluted with 2% MeOH in dichloromethane to give a pure (>95%) product **21** M+H⁺ 384.5.

### Example 22: 2-Amino-N-(cyclohexyl-methylcarbamoyl-methyl)-3,3-dimethylbutyramide (22)

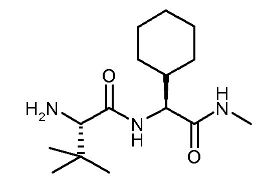

Compound 61 was kept in dichloromethane-trifuoroacetic acid 2:1 for 1 h at RT and concentrated to dryness. The residue was dried in a vacuum for 16 hrs. Reversed phase C18 HPLC showed >95% purity M+H⁺ 283.1.

### Example 23: 1-(2-Amino-4-methoxyphenyl)ethanone (23)

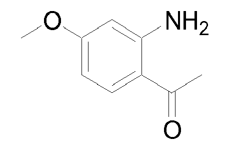

*m*-Anisidine (10.0 g, 82 mmol) was dissolved in CH₂Cl₂ (50 mL), and the solution was cooled to -50°C. BCl₃ (1 M in CH₂Cl₂, 82 mL, 82 mmol) was added slowly during 20 min, after which the mixture was stirred at -50°C for 30 min, followed by sequential addition of AcCl (6.0 mL, 84 mmol) and AlCl₃ (11 g, 82 mmol). The mixture was stirred at -50°C for 1 h and was then allowed to assume RT. After stirring at RT overnight, the solution was heated at 40°C for 4 h, after which the mixture was poured over ice. The aqueous mixture was made alkaline with 10 % NaOH (w/v) and extracted with EtOAc (4 x 200 mL). The combined organic phases were washed with brine, dried (MgSO₄), and evaporated to give a black solid, which was purified by flash column chromatography (ether/CH₂Cl₂ 20:80). The resulting solid was recrystallized from ether/hexane to give the title compound **23** as shiny tan leaflets (5.6 g, 42 %).

### Example 24: N-(tert-Butyl)-N'-isopropylthiourea (24)

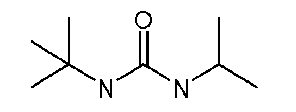

To a solution of *tert*-butylisothiocyanate (5.0 mL, 39 mmol) in CH₂Cl₂ (200 mL) were added isopropylamine (4.0 mL, 47 mmol) and diisopropylethylamine (DIEA) (6.8 mL, 39 mmol), and the mixture was stirred at RT for 2h. The reaction mixture was diluted with EtOAc, washed with 10 % citric acid (2x), saturated NaHCO₃ (2x), H₂O (2x), and brine (1x). The organic layer was dried (MgSO₄) and evaporated to yield the title compound **24** (3.3 g, 52 %) as a white solid, which was used without further purification.

### Example 25: N-Isopropylthiourea (25)

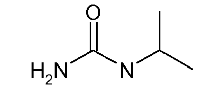

Compound **24** (3.3 g, 20 mmol) was dissolved in conc. HCl (45 mL) and the solution was refluxed for 40 min. The mixture was allowed to cool to RT and then further cooled in an ice bath and basified to pH 9.5 with solid and saturated NaHCO₃, after which the product was extracted into EtOAc (3x). The combined organic phases were washed with H₂O (2x) and brine (1x), dried (MgSO₄), and evaporated to yield the crude title compound **25** (2.1 g, 90 %), which was used without further purification.

### Example 26: 2-(Isopropylamino)-1,3-thiazole-4-carboxylic acid hydrobromide (26)

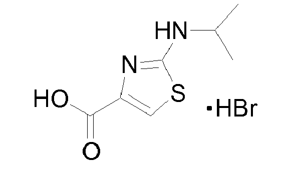

A suspension of compound **25** (2.1 g, 18 mmol) and 3-bromopyruvic acid (3.0 g, 18 became clear, and soon thereafter, the product started to precipitate as a white solid. After 2 h of heating, the reaction mixture was cooled to RT and the precipitate was filtered off and collected. This yielded pure title product **26** (4.4 g, 94 %).

### Example 27: N-(2-Acetyl-5-methoxyphenyl)-2-(isopropylamino)-1,3-thiazole-4-carboxamide (27)

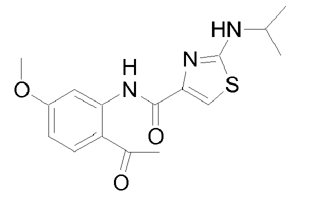

A mixture of compound **26** (4.4 g, 16.5 mmol) and the aniline derivative 66 (2.75 g, 16.5 mmol) in pyridine (140 mL) was cooled to -30°C (upon cooling, the clear solution became partially a suspension). POCl₃ (3.3 mL, 35 mmol) was added slowly over a 5 min period. The mixture was stirred at -30°C for 1 h, and was then allowed to assume RT. After stirring at RT for 1.5 h the reaction mixture was poured over ice, and the pH was adjusted to about 9-10 using solid and saturated NaHCO₃. The crude product was extracted into CH₂Cl₂ (3x) and the combined organic phases were dried (MgSO₄) and evaporated. The crude dark-beige solid was purified by flash column chromatography (hexane/EtOAc 55:45) to give the title compound **27** (5.6 g, 76 %) as a pale yellow solid.

### Example 28: 2-[2-(Isopropylamino)-1,3-thiazol-4-yl]-7-methoxyquinolin-4-ol (28)

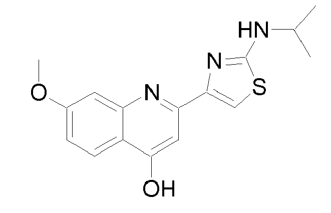

A solution of t-BuOK (2.42 g, 21 mmol) in anhydrous t.BuOH (40 mL) was heated to reflux. Compound **27** (1.8 g, 5.4 mmol) was added portion-wise over a 5 min period, and the dark red solution formed was stirred at reflux for an additional 20 min. The mixture was cooled to RT, and HCl (4 M in dioxane, 8.0 mL, 32 mmol) was added, after which the reaction mixture was concentrated under vacuum. In order to assure that all of the HCl and dioxane were removed, the crude product was re-dissolved in CH₂Cl₂twice and thoroughly evaporated to obtain the slightly impure HCl salt of compound **28** (1.62 g) as a brown solid. The product was dissolved in CH₂Cl₂ and washed with saturated NaHCO₃, after which the aqueous phase was extracted several times with CH₂Cl₂. The combined organic phases were dried (MgSO₄) and evaporated to give the title compound **28** (1.38 g, 81 %) as a light brown solid (> 95 % pure according to HPLC tests). ¹H-NMR (MeOH-*d₄*, 400 MHz): δ 1.30 (d, *J* = 6.0 Hz, 6H), 3.93 (s, 3H), 3.95-4.07 (m, 1H), 6.73 (s, 1H), 6.99 (dd, *J* = 2.4, 9.2 Hz, 1H), 7.26 (d, *J* = 2.4 Hz, 1H), 7.37 (s, 1H), 8.10 (d, *J* = 9.2 Hz, 1H).

### Example 29: (1R,4R,5R)-N-[(16)-1-[[[(1S)-1-Cyclohexyl-2-(methylamino)-2-oxoethyl]amino]carbonyl]-2,2-dimethylpropyl]-3-oxo-2-oxabicyclo[2.2.1]heptane-5-carboxamide (29)

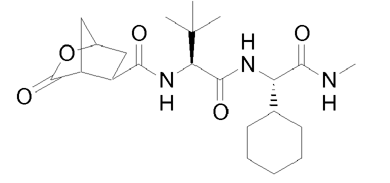

To a solution of compound 6 (53 mg, 0.34 mmol) in DMF (9 mL) was added compound 22 (80 mg, 0.28 mmol) and DIEA (290 µL, 1.66 mmol). The solution was cooled to 0°C and HATU (127 mg, 0.33 mmol) was added. After stirring at 0°C for 1 h and at RT for 1 h, the solvent was evaporated, and the crude product was purified by flash column chromatography (EtOAc/toluene 2:1) to give compound **29** (110 mg, 92 %) as a white solid.

### Example 30: 2-Amino-3,3-dimethyl-N-thiophen-2-yl-methyl-butyramide (30)

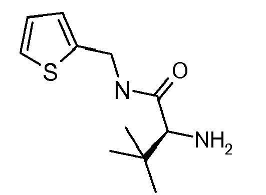

Compound **30** was prepared as described in example 18 but with the use of thiophene-2-methylamine instead of aminoindanole followed by removal of the Boc group as described in example 19.

### Example 31: 2-Amino-N-(6-hydroxy-4,5,6,7-tetrahydro-benzo[b]thiophen-5-yl)-3,3-dimethyl-butyramide (31)

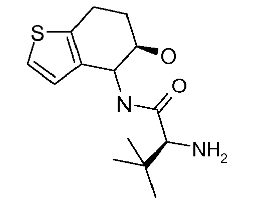

Compound **31** was prepared as described in example 18 but with the use of 2-amino-4,5,6,7-tetrahydro-benzo[b]thiophen-5-ol instead of aminoindanole followed by removal of the Boc group as described in example 19.

### Example 32: 2-Amino-N-(2-diethylamino-ethyl)-3,3-dimethyl-butyramide (32)

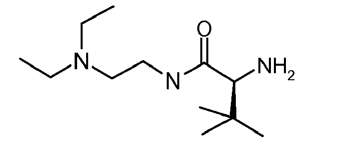

The title compound **32** was prepared as described in example 18 but with the use of N,N-diethylethylenediamine instead of aminoindanole followed by removal of the Boc group as described in example 19.

### Example 33: 2-Amino-N-[2-(2-methoxy-phenoxy)-ethyl]-3,3-dimethyl-butyramide (33)

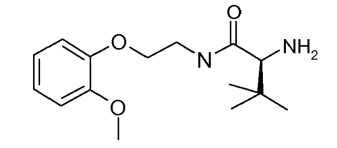

Compound **33** was prepared as described in example 18 but using 2-methoxyphenoxyethylamine instead of aminoindanole followed by removal of the Boc group as in example 19.

### Example 34: 2-Amino-1-(3-hydroxy-pyrrolidin-1-yl)-3,3-dimethyl-butan-1-one (34)

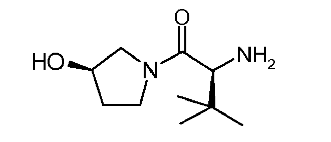

Compound **34** was prepared as described in example 18 but with the use of (R)-3-pyrrolidinone instead of aminoindanole followed by removal of the Boc group as described in example 19.

### Example 35: 2-Amino-N-(1,1-dioxo-tetrahydro-1-thiophen-3-yl)-3,3-dimethylbutyramide (35)

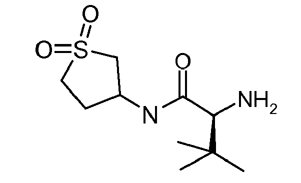

The title compound **35** was prepared as described in example 18 but with the use of 2-methoxyphenoxyethylamine instead of aminoindanole followed by removal of the Boc group as described in example 19.

### Example 36: Acetic acid (1S,2R)-1-((2S)-2-amino-3,3-dimethyl-butyrylamino)-indan-2-yl ester (36)

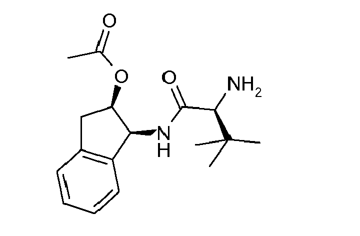

A solution of compound **18** (4g) was kept in pyridine-acetic anhydride 2:1 for 30 min. DCM was added and the solution was washed with citric acid (aq) and NaHCO₃ (aq). The organic layer was concentrated to dryness which gave the acetylated product >90% pure by HPLC. The afforded compound was then kept in a solution of 30% TFA in DCM for 1.5 hrs and then concentrated to dryness. Co-evaporation twice from toluene gave the title product **36**, >90% pure, by HPLC.

### Example 37: (2S)-Methanesulphonic acid 2-tert-butoxycarbonylamino-4-methyl-pentyl ester (37)

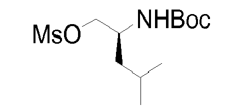

To a solution of ((1S)-1-hydroxymethyl-3-methyl-butyl)-carbamic acid *tert*-butyl ester (25 g, 115 mmol) in dichloromethane (500 mL) cooled by an ice-water bath was successively added diisopropylethylamine (35.7 g, 276 mmol) and methanesulphonyl chloride (15.81 g, 138 mmol). The resulting solution was stirred over night during which time the mixture was allowed to gradually warm up to ambient temperature. The mixture was washed successively with water, 10 % citric acid (aq), water and saturated NaHCO₃ (aq), then dried with Na₂SO₄ and concentrated to a brown solid (32.6 g, 96 %), which was used in the next reaction without further purification.

### Example 38: ii) ((1S)-1-Azidomethyl-3-methyl-butyl)-carbamic acid tert-butyl ester (38)

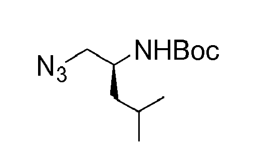

The mesylate **37** from example 37 (32.6 g, 110 mmol) was treated with sodium azide (21.45 g, 330 mmol) in DMF at 80 °C for 24 hrs. The solvent was evaporated and the residue was taken up in DCM, filtered and washed with saturated NaHCO₃ (aq). The solution was dried with Na₂SO₄ and concentrated to a brown oil which was purified by flash chromatography using a gradient of ethyl acetate and hexane to afford the title compound **38** as a white solid (19.55 g, 73 %).

### Example 39: (1S)-1-Azidomethyl-3-methyl-butylamine (39)

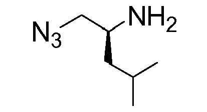

((1S)-1-Azidomethyl-3-methyl-butyl)-carbamic acid *tert*-butyl ester (9.64 g, 39.78 mmol) was treated with TFA (30 mL) in DCM (150 mL) for 3 hrs, the mixture was evaporated under reduced pressure and the residue was dissolved in ethyl acetate and washed with aqueous 1 M K₂CO₃, dried with Na₂SO₄ and concentrated to a yellow liquid **39** (4.55 g, 80 %).

### Example 40: 3-Oxo-2-oxa-bicyclo[2.2.1]heptane-5-carboxylic acid hex-5-enylmethylamide (40)

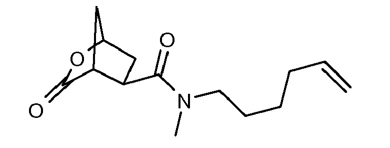

To HATU (2.17 g, 5.7 mmol) and N-methyl hex-5-enylamine hydrochloride (6.47 mmol) in 5 mL DMF, under argon in an ice bath, were added *1R*,*4R,5R*-3-oxo-2-oxa-bicyclo[2.2.1]heptane-5-carboxylic acid (835.6 mg, 5.35 mmol) in 11 mL DMF followed by DIEA (2.80 mL, 16 mmol). After stirring for 40 min, the mixture was stirred at RT for 5 h. The solvent was evaporated, the residue dissolved in EtOAc (70 mL) and washed with saturated NaHCO₃ (10 mL). The aqueous phase was extracted with EtOAc (2 x 25 mL). The organic phases were combined, washed with saturated NaCl (20 mL), dried over Na₂SO₄, and evaporated. Flash column chromatography (150 g silica gel, 2/1 EtOAc - petroleum ether (PE), TLC detection by aqueous KMnO4, Rf 0.55 in 4/1 EtOAc - PE) gave the compound **40** as a yellow oil (1.01 g, 75%).

### Example 42: 1-bromo-3-methylbutan-2-one (42)

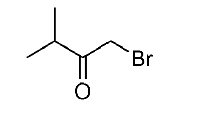

To an ice cooled solution of 3-methyl-2-butanone (25.8g, 300 mmol) in EtOH (250 ml) was added drop wise bromine (12.9 ml, 250 mmol) and the mixture was stirred for two hours in an ice bath. Petroleum ether (600ml) was added. The organic phase was washed twice with water. The combined water phases was extracted twice with petroleum ether. The combined organic phases was washed twice with a cold sodium carbonate solution and with brine. The organic phase was dried over sodium sulphate and evaporated under reduced pressure (room temperature). Yield of **42**: 50%.

### Example 43: Ethyl 4-isoppropylthiazole-2-carboxylate (43)

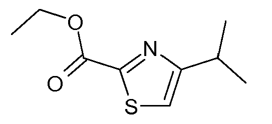

To a boiling solution of ethyl thiooxamate (16.0 g, 120 mmol) in EtOH was added drop wise 1-bromo-3-methyl-2-butanone over a period of 15 minutes. The mixture was refluxed for 1.5 hours. The solution was added to 300 ml of ice water and basified with concentrated ammonia solution. The mixture was extracted twice with ethyl acetate. The organic phase was washed with brine, dried with sodium sulphate and evaporated under reduced pressure. The product was purificated by column on chromatography silica gel eluated with hexane and 20% ethyl acetate. Yielding **43**: 15.2 g, 67%
¹H-NMR-CDCl₃ 1,35 (d, 6H), 1,42 (t, 3H) , 3,25 (m,1H), 4,49 (m, 2H) 7,20 (s, 1H)

### Example 44: 4-isopropylthiazole-2-carboxylic acid (44)

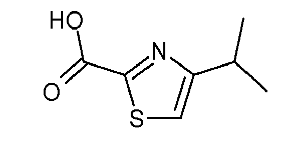

To a solution of ethyl 4-isopropylthiazole-2-carboxylate (9.1g, 46 mmol) in THF (100 ml) and MeOH (30 ml) was added a solution of lithium hydroxide (1.16 g, 48.5 mmol), and the mixture was stirred for two days at room temperature. The mixture was acidified with 2M hydrochloric acid and extracted four times with diethyl ether. The organic phase was dried with sodium sulphate and evaporated under reduced pressure. Yield of **44**: 7.1g, 90%.

### Example 45: 4-methoxy-2-nitro-benzamide (45)

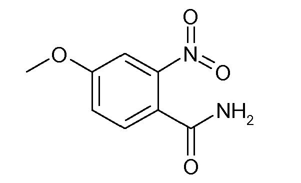

To an ice cooled suspension of 4-methoxy-2-nitro-benzoic acid (14.1 g, 71.5 mmol) and some drops of DMF in DCM (150 ml) was added drop wise oxalyl chloride (19.0 g, 150 mmol) and the mixture was stirred for two hours at room temperature. The solvent was evaporated and water was added. The product was filtered of and washed with water and hexane. The product was dried in vacuum. Yield of **45**: 10 g, 71%.

### Example 46: 4-methoxy-2-amino-benzamide (46)

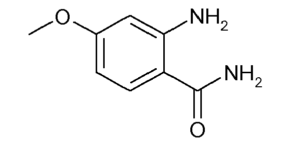

A suspension of 4-methoxy-2-nitro-benzamide (6.9 g, 35.1 mmol) in EtOH (200 ml) was hydrogenated with Raney-Ni (4.0 g) for two days at room temperature and 50 psi. The catalyst was filtered of and washed with DMF. The solvent was evaporated under reduced pressure. Yielding **46**: 5.6 g, 95%.

### Example 47: 4-isopropylthiazole-2-carboxylic acid (2-carbamoyl-5-methoxyphenyl)amide (47)

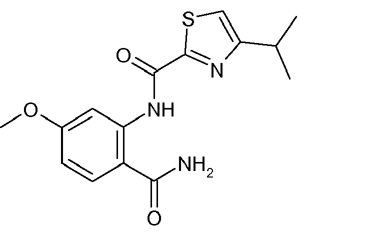

To a cooled solution of 4-methoxy-2-aminobenzamide (5.6 g, 33.7 mmol), 4-isopropylthiazole-2-carboxlic acid (7.1 g, 42 mmol) and Hobt-hydrate (6.4 g, 42 mmol) in DMF (150 ml) was added EDAC (8.6 g, 45 mmol) and TEA (6.4 ml, 45 mmol) and the mixture was stirred overnight at room temperature. A 2.5% aquoeus solution of citric acid (600 ml) was added and the mixture was extracted three times with ethyl acetate. The organic phase was washed with brine and saturated sodium hydrogencarbonate. The solution was dried over sodium sulphate and evaporated under reduced pressure. Yielding **47**: 9.0 g, 91%.

### Example 48: 2-(4-isopopylthiazole-2-yl)-7-methoxy-quinazolin-4-ol (48)

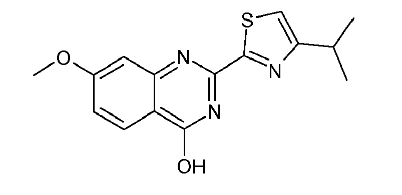

A mixture of 4-isopropyl-2-carboxylic acid (2-carbamoyl-5-methoxy-phenyl)-amide (9.0 g, 28.2 mmol) and sodium carbonate (7.5 g, 71 mmol) in EtOH water 50/50 (300 ml) was refluxed for two houres. The mixture was cooled an acidified with citric acid and extracted four times with ethyl acetate. The organic phase was dried with sodium sulphate and evaporated under reduced pressure. The product was crystallisized from EtOH. Yielding **48**: 4.8 g, 60%.
¹H-NMR-DMSO-D₆ δ 1,30 (d, 6H) , 3,10 (m, 1H) , 3,90 (s, 3H) , 7,10 (dd, 1H) 7,16 (d, 1H), 7,62 (d, 1H), 8,02 (d, 1H).

### Example 49: 4-isopropylthiazole-2-carboxylic acid (2-carbamoyl-phenyl)-amide (49)

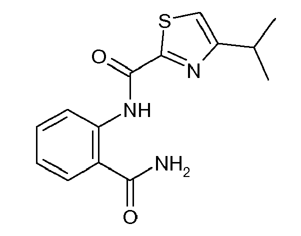

2-Aminobenzamide (2.04 g, 15 mmol) was reacted with 4-isopropylthiazole-2-carboxylic acid (2.5 g, 14.6 mmol) as described in example 47, which gave the title compound **49** (2.4 g, 56%).

### Example 50: 2-(4-isopopylthiazole-2-yl)-quinazolin-4-ol (50)

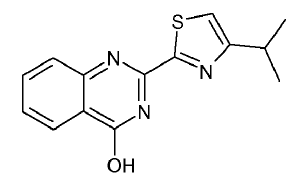

4-isopropylthiazole-2-carboxylic acid (2-carbamoyl-phenyl)-amide **49** (2.4 g, 8.3 mmol) was treated according to the procedure described in example 48 which gave the title compound **50** (1.7 g, 77%). ¹H-NMR CDCl₃ δ1.33 (d, 6H), 3.12 (m, 1H), 7.55 (t, 1H), 7.65 (s, 1H), 7.72 (d, 1H), 7.82 (t, 1H), 8.14 (d, 1H).

### Example 51: 7-Methoxy-2-phenyl-quinazolin-4-ol (51)

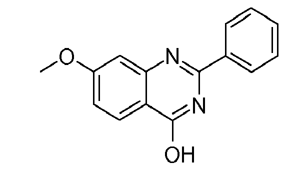

Treatment of 2-amino-5-methoxy-benzamide as described in Tetrah. Letts. 45 (2004) 3475-3476 for the preparation of 2-phenyl-quinazoline 4-ol gave the title compound **51.**

### Example 52: 4-Chloro-2-(4-isopropyl-thiazol-2-yl)-quinazoline (52)

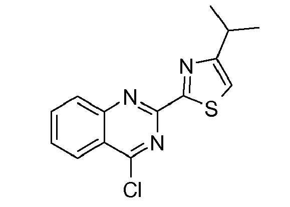

Compound **50** (100mg, 0.37 mmol) was added to phosphorous oxychloride (2 mL) and heated to 100°C for 2 h. The reaction mixture was then poured on ice with vigorous stirring and made basic with NaOH (aq). The resulting slurry was extracted with ether (3x20 mL) and the combined organic phases were dried (MgSO₄) and filtered. Removal of the solvent in vacuo afforded the title compound **57** in quantitative yield. LC/MS >95%, m/z (ESI⁺)= 290(MH⁺).

### Example 53: 4-Chloro-2-(4-isopropyl-thiazol-2-yl)-7-methoxy-quinazoline (53)

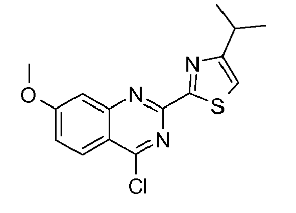

Compound **48** (300mg, 1 mmol) was added to phosphorous oxychloride (6 mL) and heated to 90°C for 4 h. The reaction mixture was then poured on ice with vigorous stirring and made basic with NaOH (aq). The resulting slurry was extracted with ether (3x50 mL) and the combined organic phases were dried (MgSO₄) and filtered. Removal of the solvent in vacuo afforded the title compound **53** in quantitative yield. LC/MS >95%, m/z (ESI⁺)= 320(MH⁺).

### Example 54: N-(2-acetyl-5-methoxyphenyl)-2-thiazolecarboxamide (54)

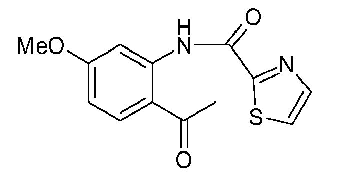

To a solution of 2-thiazolecarboxaldehyde (500 mg, 3.87 mmol) in 35 mL of pyridine, 1-(2-amino-4-methoxyphenyl)ethanone (640 mg, 3.87 mmol) was added and the solution was cooled to -30 °C before POCl₃ (750 µl, 8.13 mmol) was added drop wise. The reaction mixture was stirred at -10 °C for 1 hour and then at room temperature for 2 hours. The mixture was concentrated under vacuum and the residue was triturated with NaHCO₃ to pH 7. The precipitate was filtrated, washed with water and dried. The amide **54** was pure enough for the next step without purification (972 mg, 91%).

### Example 55: 1,4-dihydro-7-methoxy-2-(2-thiazolyl)- 4-quinolinol (55)

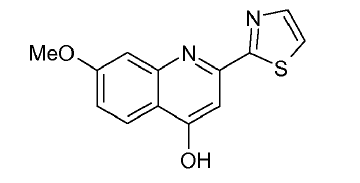

The amide **54** (441 mg, 1.6 mmol) was dissolved in pyridine (20 mL) and potassium hydroxide (180 mg, 3.2 mmol) was added. The mixture was treated by microwaves (180°C, 30 min). The reaction mixture was concentrated under vacuum and dissolved in a small amount of water. The solution was poured in phosphate buffer (136 mL, pH = 7) and stirred for 30 min. The solid was filtered and triturated with EtOAc and dried under vacuum to obtain the quinoline **55** (361 mg, 87%).

### Example 56: 1,1-Dimethyl-thiourea (56)

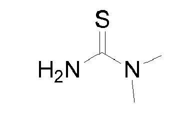

Dimethylamine (2M in THF, 27.5 mL, 55 mmol) was added to a stirred solution of thiocarbonyldiimidazole (10 g, 56.1 mmol) in dry THF (50 mL). The reaction mixture turned clear by addition and was stirred at 50 °C for 2 hrs. After the reaction mixture had reached RT, it was evaporated on silica and purified by flash chromatography (MeOH: DCM 2:98). The solvent was removed by rotary evaporation and the remaining product dried with high vacuum before it was added to a solution of MeOH (125 mL) saturated with NH₃. The reaction mixture was stirred for 60 hrs until TLC indicated complete consumption of the starting material and LC-MS showed the product peak. The product precipitated while removing the solvent by rotary evaporation. The remaining solvent was diluted with diethyl ether and the white crystals were filtered off and dried to give a yield of 1.16 g title compound **56** (20%). The remaining oil was purified by flash chromatography (MeOH: DCM 5:95) and another 1.87 g **56** (32%) was obtained.

### Example 57: 2-Dimethylamino-thiazole-4-carboxylic acid *HBr (57)

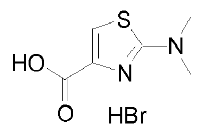

3-Bromopuruvic acid (2.94 g, 17.6 mmol) was added to a stirred solution of **56** (1.87 g, 17.6 mmol) in dry THF (60 mL). The reaction mixture was stirred at RT for 4 hrs. The precipitate that had formed was filtered off, washed with cold THF and dried on high vacuum. LC-MS showed the product peak. The product **57** was obtained as a white solid (2.64 g, 59%).

### Example 58: 2-Dimethylamino-thiazole-4-carboxylic acid (2-acetyl-5-methoxyphenyl)-amide (58)

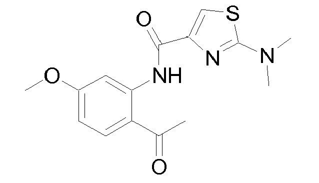

POCl₃ (2.07 mL, 21.8 mmol) was added to a stirred mixture of **57** (2.63 g, 10.4 mmol) in dry pyridine (50 mL). The reaction was stirred at RT for 3 hrs and thereafter the solvent was removed by rotary evaporation and the residue dissolved in H₂O. The brown precipitate that was formed was filtered, washed with water and was found to be pure by LC-MS and NMR. The product **58** was obtained as a brown solid (2.85 g, 86%). LRMS (M+H) 320.

### Example 59: 2-(2-Dimethylamino-thiazole-4-yl)-7-methoxy-quinolin-4-ol (59)

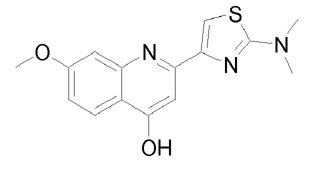

2-Dimethylamino-thiazole-4-carboxylic acid (2-acetyl-5-methoxy-phenyl)-amide (1.29 g, 4.04 mmol) was dissolved in 20 mL pyridine in a microwave vial. Grinded KOH (0.48 g, 8.48 mmol) was added to the solution and the enclosed vial was reacted in the microwave oven at 100°C for 30 minutes. The mixture was transferred to a round-bottom flask and the solvent was removed by rotary evaporation. The residue was transferred to a beaker with phosphate buffer (pH 7.0, 0.1 M, 300 mL) where the product precipitated while stirring. The precipitate was filtered, rinsed with water and dried with high vacuum. The product **59** was found to be pure by LC-MS and NMR and was obtained in 74% yield (0.90 g). LRMS (M+H) 302.

### Example 60: 4-Chloro-2-(4-isopropyl-thiazol-2-yl)-7-methoxy-quinoline (60)

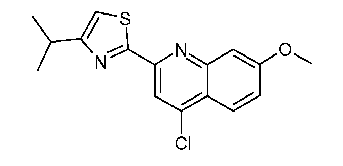

2-(4-Isopropylthiazol-2-yl)-7-methoxyquinolin-4-ol, prepared as described in WO00/59929, (3.6 g) was mixed with 20 ml of phosphorus oxychloride and heated at 100 °C for 40 min. The reaction was monitored by LC MS. After 40 min of heating, the excess of phosphorus oxychloride was removed by rotary evaporation. The residual oil was mixed with sat. sodium bicarbonate solution and extracted into ether (3x70 ml). The combined organic extracts were washed with brine, dried over magnesium sulfate, concentrated by rotary evaporation and passed through short pad of silica (hexane) to give the title compound **60** as a white powder 3.6 g (yield 62%).

### Example 61: 4-[2-(4-Isopropyl-thiazol-2-yl)-7-methoxy-quinolin-4-yloxy]-pyrrolidine-1,2-dicarboxylic acid 1-tert-butyl ester (61)

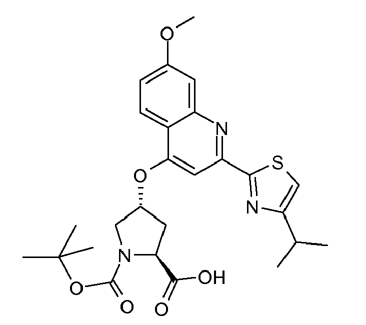

To a stirred solution ofN-Boc-trans-4-hydroxy-L-proline (2.6 g, 11.2 mmol) in DMSO (80 mL) was added potassium *tert*-butoxide (3.8 g, 3 eq). After approx. 1 hr of stirring, 4-chloro-2-phenyl-7-methoxy quinoline (3.6 g, 11.2 mmol) was added and the reaction mixture was stirred at room temperature overnight. The mixture was diluted with water (350 mL) and neutralized with 1N HCl. The resulting suspension was extracted into ethylacetate (3x100ml), washed with brine and dried over magnesium sulfate. Filtration and concentration by rotary evaporation gave after drying overnight on high vacuum 3.6 g of the title compound **61** (yield 62 %). Purity by HPLC >95%. M+H⁺ 514.

### Example 62: 1-(2-Nitro-4-trifluoromethyl-phenyl)-1H-pyrazole (62)

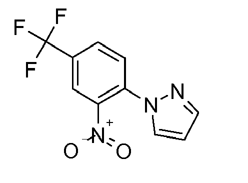

1-Fluoro-2-nitro-4-trifluoromethyl-benzene (209 mg, 1 mmol) was dissolved in EtOH (4.5 mL) in a 5 mL microwave reaction vessel. 1H-pyrazole (83.5 mg, 1.2 mmol), DIPEA (329 µL, 2 mmol) and a stirrbar were added followed by sealing of the reaction vessel. The reaction mixture was then heated in the microwave for 30 min at 120°C. The reaction was concentrated *in vacuo* and the residue purified by flash chromatography (Silica, Hexane: EtOAc) to afford the title compound **62** (206 mg, 81%). LC/MS >95%, m/z (ESI⁺)= 258(MH⁺).

### Example 63: 3-Methyl-1-(2-nitro-4-trifluoromethyl-phenyl)-1H-pyrazole (63)

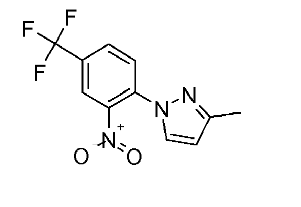

Compound **63** was synthesised as described in Example 62, except that 3-methyl-1H-pyrazole replaced 1H-pyrazole. TLC (Silica; Hexane: EtOAc, 4:1): Rf = 0.3; LC/MS >95%, m/z (ESI⁺)= 272(MH⁺).

### Example 64: 5-Methyl-1-(2-nitro-4-trifluoromethyl-phenyl)-1H-pyrazole (64)

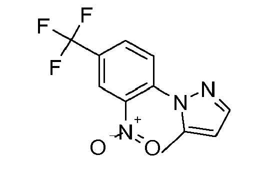

The title compound **64** was synthesised according to the procedure described in Example 62, except that 3-methyl-1H-pyrazole replaced 1H-pyrazole. The 3-methyl-1H-pyrazole partially rearranges to 5-methyl-1H-pyrazole during the conditions in Example 10-1. TLC (Silica; Hexane: EtOAc, 4:1): Rf = 0.4; LC/MS >95%, m/z (ESI⁺)= 272(MH⁺).

### Example 65: 1-(4-Fluoro-2-nitro-phenyl)-3-methyl-1H-pyrazole (65)

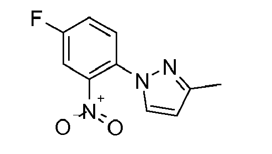

The title compound **65** was synthesised according to the procedure described in Example 62, except that 3-methyl-1H-pyrazole replaced 1H-pyrazole. TLC (Silica; Hexane: EtOAc, 4:1): Rf = 0.3; LC/MS >95%, m/z (ESI⁺)= 222(MH⁺).

### Example 66: 2-Pyrazol-1-yl-5-trifluoromethyl-phenylamine (66)

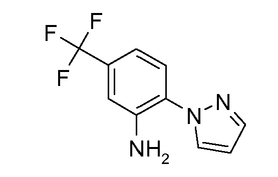

Compound **62** (206 mg, 0.8 mmol) was dissolved in EtOH (25 mL) in a 50 mL flask. Two spatulas of 5 % Pd on Activated Carbon and a stirrbar were added followed by evacuation and N₂(g) purging of the flask. H₂(g) was then introduced into the flask by a balloon and the reaction stirred at room temperature under H₂-atmosphere for 2 h. The H₂(g) inlet was closed and the flask evacuated and N₂(g) purged 3 times. LC/MS analysis showed complete hydrogenation and the mixture was filtered through a plug of Celite before removal of the solvent *in vacuo* to afford the crude aniline **66** (163 mg, 90%). LC/MS, >95%, m/z (ESI⁺)= 228(MH⁺).

### Example 67: 2-(3-Methyl-pyrazol-1-yl)-5-trifluoromethyl-phenylamine (67)

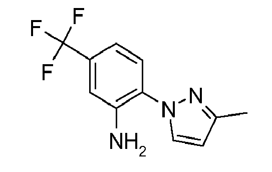

Compound **67** was synthesised according to the procedure described in Example 66. LC/MS >95%, m/z (ESI⁺)= 242(MH⁺).

### Example 68: 2-(5-Methyl-pyrazol-1-yl)-5-trifluoromethyl-phenylamine (68)

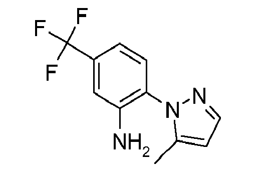

Compound **68** was synthesised according to the procedure described in Example 66. LC/MS >95%, m/z (ESI⁺)= 242(MH⁺).

### Example 69: 5-Fluoro-2-(3-methyl-pyrazol-1-yl)-phenylamine (69)

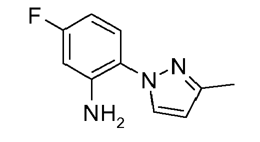

Compound **69** was synthesised according to the procedure described in Example 66. LC/MS >95%, m/z (ESI⁺)= 192(MH⁺).

### Example 70

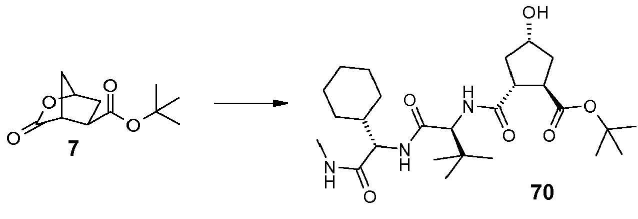

Lactone **7** (263 mg, 1.55 mmol) and **22** (930 mg, 2.42 mmol) are dissolved in dry THF (20 mL). DIPEA (530 uL, 3.04 mmol) and 2-hydroxypyridine (260 mg, 2.73 mmol) are added and the mixture refluxed for five days. The solvent is evaporated and the crude product purified by flash column chromatography to give the target product **70**: *m*/*z* = 496 (M+H)⁺.

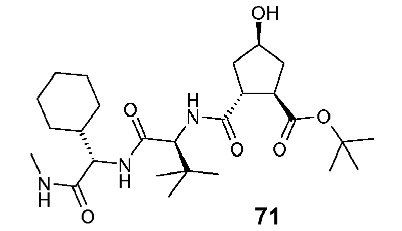

To a solution of alcohol (7, 366 mg, 0.738 mmole), paranitrobenzoic acid (123 mg, 1 eq) and triphenylphosphine (194 mg, 1 eq) in dry THF (10 mL) are added dropwise at -20°C, under nitrogen DIAD (149 mg, 1 eq). After 1h at 20°C the reaction mixture is allowed to warm up to RT. After 16h, the reaction mixture is diluted with EtOAc and washed with water, dried (MgSO₄), filtered and evaporated. Purification by column chromatography (Gradient of EtOAc 0 to 90% in CH₂Cl₂ affords the target ester, which is dissolved in THF (3 mL) and cooled to 0°C. To this solution, is added LiOH monohydrate (100 mg) dissolved in water (1 mL). The mixture is stirred at 0°C for 2 h, then acidified with a diluted solution of HCl (pH=6) and extracted with AcOEt. The organic layer is extracted with a saturated solution of NaHCO₃, washed with brine, dried (MgSO₄) and evaporated to give the target product **71:** *m*/*z* = 496 (M+H⁺).

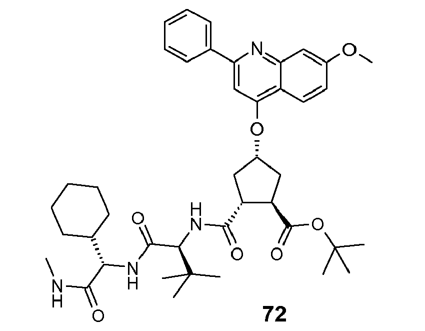

To a solution of alcohol **71** (610 mg, 1.23 mmoles), quinoline **1** (401 mg, 1.6 mmol) and triphenylphosphine (450 mg, 1.72 mmol) in dry THF (15 mL) is added dropwise DIAD (370 mg, 1.85 mmoles, 1.5 eq) at -20°C, under nitrogen. After 1h at -20°C the reaction mixture is allowed to warm up to room temperature. After 20h, the mixture is poured in ice/water, and extracted with EtOAc. The organic layers are combined, washed with brine, dried (MgSO₄) and concentrated under reduced pressure. Purification by column chromatography (Gradient of EtOAc in CH₂Cl₂0 to 90%) affords the title product **72:** *m*/*z* = 729 (M+H)⁺.

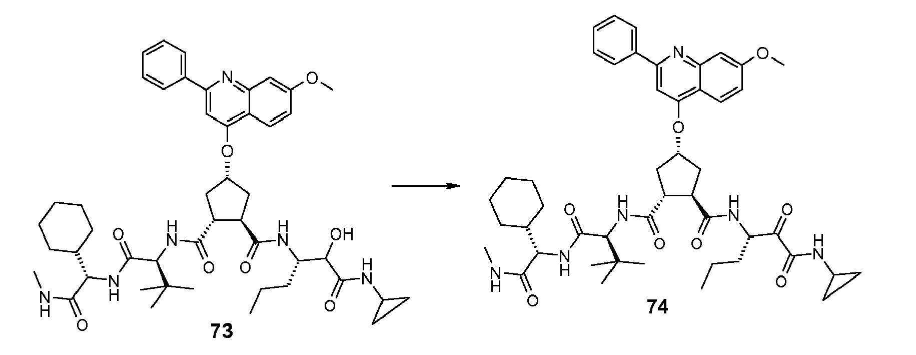

A solution of **72** (250 mg, 0.345 mmol) is stirred in a mixture of TFA and dichloromethane for 5h, then evaporated several times with toluene. The resulting free acid is dissolved in DMF (10 mL) and PyBOP (186 mg, 0.36 mmol) and NMM (0.113 mL, 1.03 mmol) are added at 0°C under nitrogen. Then, the reaction mixture is stirred 1h at room temperature after which a solution of 3-Amino-2-hydroxyhexanoic acid cyclopropylamide (84 mg, 0.37 mmol) and 0.113 mL of NMM is slowly added. The resulting solution is stirred for 12 h at room temperature, diluted with AcOEt and successively washed with water, citric acid and brine. The organic layer is dried (Na₂SO₄) and concentrated in vacuo. Purification by flash chromatography (Gradient CH₂Cl₂ to CH₂Cl₂/MeOH, 95:5) yields the target product **73:** *m*/*z* = 841 (M+H)⁺.
Dess-Martin periodinane (541 mg, 1.2 mmol) and *tert*-butanol (0.540 mL) are added to a solution of **73** (1.0 g, 1.2 mmol) in CH₂Cl₂ (8 mL). After 3 h at room temperature, the reaction mixture is quenched with sodium thiosulfate 1M. The organic layer is diluted with CH₂Cl₂, washed with NaHCO₃ 1N, dried (Na₂SO₄) and evaporated. Purification by flash chromatography yields compound **74:** *m*/*z* = 839 (M+H)⁺.

### Example 71: Activity of compounds of formula I; Replicon assay

The compounds of formula I were examined for activity in the inhibition of HCV RNA replication in a cellular assay. The assay demonstrated that the compounds of formula I exhibited activity against HCV replicons functional in a cell culture. The cellular assay was based on a bicistronic expression construct, as described by Lohmann et al. (1999) Science vol. 285 pp. 110-113 with modifications described by Krieger et al. (2001) Journal of Virology 75: 4614-4624, in a multi-target screening strategy. In essence, the method was as follows. The assay utilized the stably transfected cell line Huh-7 luc/neo (hereafter referred to as Huh-Luc). This cell line harbors an RNA encoding a bicistronic expression construct comprising the wild type NS3-NS5B regions of HCV type 1b translated from an Internal Ribosome Entry Site (IRES) from encephalomyocarditis virus (EMCV), preceded by a reporter portion (FfL-luciferase), and a selectable marker portion (neo^{R}, neomycine phosphotransferase). The construct is bordered by 5' and 3' NTRs (non-translated regions) from HCV type 1b. Continued culture of the replicon cells in the presence of G418 (neo^{R}) is dependent on the replication of the HCV RNA. The stably transfected replicon cells that express HCV RNA, which replicates autonomously and to high levels, encoding *inter alia* luciferase, are used for screening the antiviral compounds.

The replicon cells were plated in 384 well plates in the presence of the test and control compounds which were added in various concentrations. Following an incubation of three days, HCV replication was measured by assaying luciferase activity (using standard luciferase assay substrates and reagents and a Perkin Elmer ViewLux™ ultraHTS microplate imager). Replicon cells in the control cultures have high luciferase expression in the absence of any inhibitor. The inhibitory activity of the compound on luciferase activity was monitored on the Huh-Luc cells, enabling a dose-response curve for each test compound. EC50 values were then calculated, which value represents the amount of the compound required to decrease by 50% the level of detected luciferase activity, or more specifically, the ability of the genetically linked HCV replicon RNA to replicate.

### Inhibition assay

The aim of this *in vitro* assay was to measure the inhibition of HCV NS3/4A protease complexes by the compounds of the present invention. This assay provides an indication of how effective compounds of the present invention would be in inhibiting HCV NS3/4A proteolytic activity. The inhibition of full-length hepatitis C NS3 protease enzyme was measured essentially as described in Poliakov, 2002 Prot Expression & Purification 25 363 371. Briefly, the hydrolysis of a depsipeptide substrate, Ac-DED(Edans)EEAbuψ[COO]ASK(Dabcyl)-NH₂ (AnaSpec, San José, USA), was measured spectrofluorometrically in the presence of a peptide cofactor, KKGSVVIVGRIVLSGK (Åke Engström, Department of Medical Biochemistry and Microbiology, Uppsala University, Sweden). [Landro, 1997 #Biochem 36 9340-9348]. The enzyme (1 nM) was incubated in 50 mM HEPES, pH 7.5, 10 mM DTT, 40% glycerol, 0.1% n-octyl-D-glucoside, with 25 µM NS4A cofactor and inhibitor at 30°C for 10 min, whereupon the reaction was initiated by addition of 0.5 µM substrate. Inhibitors were dissolved in DMSO, sonicated for 30 sec. and vortexed. The solutions were stored at - 20°C between measurements. The final concentration of DMSO in the assay sample was adjusted to 3.3%. The rate of hydrolysis was corrected for inner filter effects according to published procedures. [Liu, 1999 Analytical Biochemistry 267 331-335]. Ki values were estimated by non-linear regression analysis (GraFit, Erithacus Software, Staines, MX, UK), using a model for competitive inhibition and a fixed value for Km (0.15 µM). A minimum of two replicates was performed for all measurements.

## Claims

1. A compound of formula I: 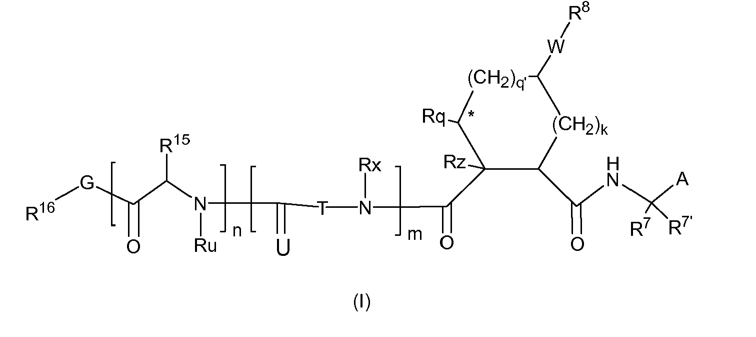 a pharmaceutically acceptable salt, or a stereoisomeric form thereof; wherein
**A** is selected from 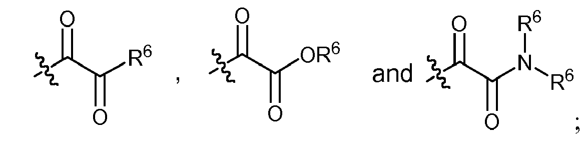 wherein
each **R⁶** independently is hydrogen, hydroxyl, C₁₋₆alkoxy, C₃₋₇cycloalkyl, aryl, Het, C₁₋₆alkyl or C₂₋₆alkenyl, the latter two being optionally substituted with C₃₋₇cycloalkyl, aryl or with Het; wherein one, two or three hydrogen atoms in the C₁₋₆alkyl, C₂₋₆alkenyl or C₃₋₇cycloalkyl moieties, or one, two or three of the optional substituents of Het or aryl may be optionally replaced by one, two or three substituents selected from halogen, -O-R⁴, nitro, cyano, polyhaloC₁₋₆alkyl, polyhaloC₁₋₆alkoxy, oxo, thioxo, =N(R⁴), =N(OR⁴), -N(R⁴)₂, -SR⁴, -SOR⁴, -SO₂R⁴, -SO₂N(R⁴)₂, -C(O)R⁴, -C(O)C(O)R⁴, -C(O)C(O)-OR⁴, -C(O)C(O)N(R⁴)₂, -C(O)CH₂C(O)R⁴, -C(O)OR⁴, -OC(O)R⁴, -C(O)N(R⁴)₂, -OC(O)N(R⁴)₂, -N(R⁴)N(R⁴)COR⁴, -N(R⁴)N(R⁴)C(O)OR⁴, -N(R⁴)N(R⁴)CON(R⁴)₂, -N(R⁴)SO₂R⁴, -N(R⁴)SO₂N(R⁴)₂, -N(R⁴)C(O)OR⁴, -N(R⁴)C(O)R⁴, -N(R⁴)C(O)N(R⁴)₂, -N(COR⁴)COR⁴, -N(OR⁴)R⁴, -C(=NH)N(R⁴)₂, -C(O)N(OR⁴)R⁴, -C(=NOR⁴)R⁴; and
each **R**⁴ is independently selected from hydrogen, aryl, C₃₋₇cycloalkyl, or C₁₋₆-alkyl optionally substituted with aryl, C₃₋₇cycloalkyl or with Het; or two **R**⁶ groups taken together with the nitrogen to which they are bonded form a radical (N)Het;
**R⁷** is C₃₋₇cycloalkyl, aryl, Het, C₁₋₆alkyl or C₂₋₆alkenyl, the latter two being optionally substituted with C₃₋₇cycloalkyl, aryl, Het, with 1-3 halo atoms, amino, mono- or di-C₁₋₆alkylamino, -SH; or R⁷ is J;
**R^{7'}** is H; or
**R**^{7'} and **R**⁷ taken together with the carbon atom to which they are attached form a C₃₋₇cycloalkyl ring which may be optionally substituted with R^{7a} wherein;
**R**^{7a} is C₁₋₆alkyl, C₃₋₇cycloalkyl, C₂₋₆alkenyl each of which may be optionally substituted with 1, 2 or 3 halo atoms; or **R**^{7a} can be J;
**q'** is 0, 1,2 or 3; and **k** is 0, 1, 2 or 3;
**Rz** is H, or together with one hydrogen atom on the carbon atom marked with an asterisk forms an additional bond;
**Rq** is H or C₁₋₆alkyl;
**W** is -CH₂-, -O-, -OC(=O)NH-, -OC(=O)-, -S-, -NH-, -NR²-, -NHS(=O)₂-, -NHC(=O)NH-, -NHC(=O)-, -NHC(=S)NH-, or a bond;
**R**⁸ is a ring system containing 1 or 2 saturated, partially saturated or unsaturated rings each of which has 4-7 ring atoms and each of which has 0 to 4 hetero atoms independently selected from S, O and N, the ring system being optionally spaced from W by a C₁₋₃alkylene group; or R⁸ is C₁₋₆alkyl; any of which R⁸ groups can be optionally mono-, di-, tri-, or tetra-substituted with R⁹;
each **R⁹** independently is halo, oxo, cyano, azido, nitro, -C(=O)NH₂, polyhaloC₁₋₆alkyl, C₃₋₇cycloalkyl, aryl, Het, C₁₋₆alkyl being optionally substituted with C₃₋₇cycloalkyl, with aryl or with Het; or **R⁹** is also -Y-NR²R³, -Y-O-R³, -Y-C(=O)R³, -Y-(C=O)NR²R³, -Y-NR²C(=O)R³, -Y-NHS(=O)ₚR³, -Y-S(=O)ₚR³, -Y-S(=O)ₚNR²R³, -Y-C(=O)OR³, -Y-NR²C(=O)OR³; wherein said C₃₋₇cycloalkyl, aryl, or Het, is optionally substituted with one, two or three R¹⁰ radicals, wherein
each **Y** is independently a bond or C₁₋₄alkylene;
each **R²** is independently H or C₁₋₆alkyl;
each **R³** is independently H, C₃₋₇cycloalkyl, aryl, Het, C₁₋₆alkyl, the latter being optionally substituted with C₃₋₇cycloalkyl, aryl or with Het;
or **R² and R³** and the nitrogen to which they both are attached define (N)Het;
each **p** is independently 1 or 2;
each **R**¹⁰ independently is C₁₋₆alkyl, C₃₋₇cycloalkyl, C₁₋₆alkoxy, amino, mono- or diC₁₋₆alkylamino, aminocarbonyl or mono- or diC₁₋₆alkylaminocarbonyl, amido, sulfonyl, (C₁₋₃alkyl)sulfonyl, nitro, cyano, hydroxy, mercapto, halo, haloC₁₋₆alkyl, carboxyl, pyrrolidinyl, piperidinyl, piperazinyl, 4-C₁₋₆alkylpiperazinyl (e.g. 4-methylpiperazinyl) or morpholinyl; and wherein the morpholinyl and piperidinyl groups may be optionally substituted with one or two C₁₋₆alkyl radicals;
**Rx** is H, C₁₋₆alkyl or J;
**T** is -CHR¹¹-, -NRd-, wherein **Rd** is H, C₁₋₆alkyl, or J;
**R¹¹** is H, C₁₋₆alkyl, C₃₋₇cycloalkyl, aryl, Het, any of which can be substituted with halo, oxo, cyano, azido, nitro, C₁₋₆alkyl, C₃₋₇cycloalkyl, aryl, Het, -C(=O)NH₂, -Y-NR²R³, -Y-O-R³, -Y-C(=O)R³, -Y-(C=O)NR²R³, -Y-NR²C(=O)R³, -Y-NHSOₚR³, -Y-S(=O)ₚR³, -Y-S(=O)ₚNR²R³, -Y-C(=O)OR³, -Y-NR²C(=O)OR³; or R¹¹ is J;
**J**, if present, is a single 3 to 10-membered saturated or partially unsaturated alkylene chain extending from the R⁷/R^{7'} C₃₋₇cycloalkyl or from the carbon atom to which R⁷ is attached to one of Rj, Rx, Ry or R¹¹ to form a macrocycle, which chain is optionally interrupted by one to three heteroatoms independently selected from: -O-, -S- or -NR¹²-, and wherein 0 to 3 carbon atoms in the chain are optionally substituted with R¹⁴; wherein;
**R¹²** is H, C₁₋₆alkyl, C₃₋₇cycloalkyl, or -C(=O)R¹³;
**R¹³** is C₃₋₇cycloalkyl, aryl, Het, C₁₋₆alkyl; the latter being optionally substituted with C₃₋₇cycloalkyl, aryl or with Het;
**R¹⁴** is independently selected from the group consisting of H, C₁₋₆alkyl, haloC₁₋₆alkyl, C₁₋₆alkoxy, hydroxy, halo, amino, oxo, mercapto, and C₁₋₆thioalkyl;
**Ru** is independently H or C₁₋₆alkyl;
**m** is 0 or 1; **n** is 0 or 1;
**U** is O or is absent;
**R¹⁵** is H, -Y-C(=O)R³, -Y-C(=O)OR³, -Y-S(=O)pR³, C₃₋₇cycloalkyl, aryl, Het, or C₁₋₆alkyl optionally substituted with C₃₋₇cycloalkyl, aryl, or with Het; wherein any of C₃₋₇cycloalkyl, aryl, Het, and C₁₋₆alkyl, can be substituted with halo, oxo, cyano, azido, nitro, C₁₋₆alkyl, amino, mono- or diC₁₋₆alkylamino, hydroxy, C₁₋₆alkoxy, C₁₋₆alkylcabonyl, aminocarbonyl, mono- or diC₁₋₆alkylaminocarbonyl, C₁₋₆alkylthio, mercapto, carboxyl, C₁₋₆alkoxycarbonyl;
**G** is -O-, -NRy-, -NRjNRj-, where one Rj is H or C₁₋₆alkyl and the other Rj is H, C₁₋₆alkyl or J;
**Ry** is H, C₁₋₆alkyl; or Ry is J;
**R¹⁶** is H, -Y-C(=O)R³, -Y-C(=O)OR³, -Y-S(=O)pR³, C₃₋₇cycloalkyl, aryl, Het, or C₁-₆alkyl optionally substituted with C₃₋₇cycloalkyl, aryl, or with Het; wherein any of C₃₋₇cycloalkyl, aryl, Het, and C₁₋₆alkyl, can be substituted with halo, oxo, cyano, azido, nitro, C₁₋₆alkyl, amino, mono- or diC₁₋₆alkylamino, hydroxy, C₁₋₆alkoxy, C₁₋₆alkylcabonyl, aminocarbonyl, mono- or diC₁₋₆alkylaminocarbonyl, C₁₋₆alkylthio, mercapto, carboxyl, C₁₋₆alkoxycarbonyl;
**aryl** as a group or part of a group is phenyl, naphthyl, indanyl, or 1,2,3,4-tetrahydronaphthyl, each of which may be optionally substituted with one, two or three substituents selected from halo, C₁₋₆alkyl, polyhaloC₁₋₆alkyl, hydroxy, C₁₋₆alkoxy, polyhaloC₁₋₆alkoxy, C₁₋₆alkoxyC₁₋₆alkyl, carboxyl, C₁₋₆alkylcarbonyl, C₁₋₆alkoxycarbonyl, cyano, nitro, amino, mono- or diC₁₋₆alkylamino, aminocarbonyl, mono- or diC₁₋₆alkylaminocarbonyl, azido, mercapto, C₃₋₇cycloalkyl, phenyl, pyridyl, thiazolyl, pyrazolyl, pyrrolidinyl, piperidinyl, piperazinyl, 4-C₁₋₆alkylpiperazinyl, 4-C₁₋₆alkylcarbonyl-piperazinyl, and morpholinyl; wherein the morpholinyl and piperidinyl groups may be optionally substituted with one or with two C₁₋₆alkyl radicals; and the phenyl, pyridyl, thiazolyl, pyrazolyl groups may be optionally substituted with 1, 2 or 3 substituents each independently selected from C₁₋₆alkyl, C₁₋₆alkoxy, halo, amino, mono- or diC₁₋₆alkylamino;
**Het** as a group or part of a group is a 5 or 6 membered saturated, partially unsaturated or completely unsaturated heterocyclic ring containing 1 to 4 heteroatoms each independently selected from nitrogen, oxygen and sulfur, said heterocyclic ring being optionally condensed with a benzene ring, and wherein the group Het as a whole may be optionally substituted with one, two or three substituents each independently selected from the group consisting of halo, C₁₋₆alkyl, polyhalo-C₁₋₆alkyl, hydroxy, C₁₋₆alkoxy, polyhaloC₁₋₆alkoxy, C₁₋₆alkoxyC₁₋₆alkyl, carboxyl, C₁₋₆alkylcarbonyl, C₁₋₆alkoxycarbonyl, cyano, nitro, mercapto, amino, mono- or diC₁₋₆alkylamino, aminocarbonyl, mono- or diC₁₋₆alkylaminocarbonyl, C₃₋₇cycloalkyl, phenyl, pyridyl, thiazolyl, pyrazolyl, pyrrolidinyl, piperidinyl, piperazinyl, 4-C₁₋₆alkylpiperazinyl, 4-C₁₋₆alkylcarbonyl-piperazinyl, and morpholinyl; wherein the morpholinyl and piperidinyl groups may be optionally substituted with one or with two C₁₋₆alkyl radicals; and the phenyl, pyridyl, thiazolyl, pyrazolyl groups may be optionally substituted with 1, 2 or 3 substituents each independently selected from C₁₋₆alkyl, C₁₋₆alkoxy, halo, amino, mono- or diC₁₋₆alkylamino;
**(N)Het** as a group or part of a group is a 3 to 7 membered saturated, partially unsaturated or completely unsaturated heterocyclic ring containing 1 to 4 heteroatoms each independently selected from nitrogen, oxygen and sulfur, of which at least one is nitrogen, which nitrogen is linked to the group -CO-CO- in radical A; said heterocyclic ring being optionally condensed with a benzene ring; and wherein the group (N)Het as a whole may be optionally substituted with one, two or three substituents each independently selected from the group of substituents specified in the definition of Het.

2. A compound according to claim 1, wherein the 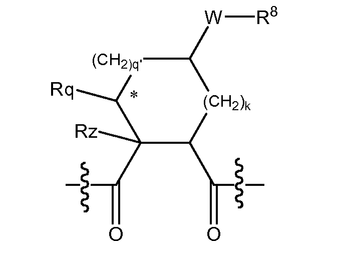 moiety in the compounds of formula I has the structure:

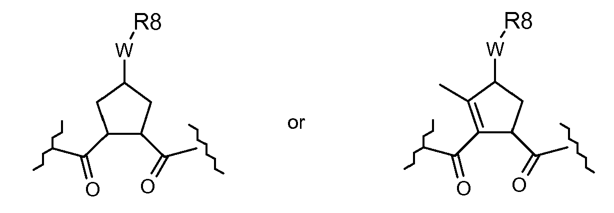

3. A compound according to claim 1, with the structure 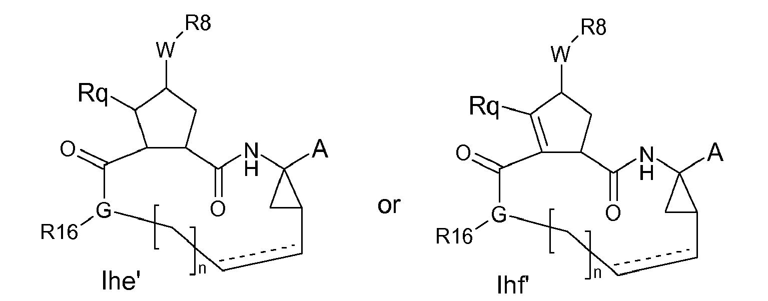 wherein
A, W and R⁸ are as defined in claim 1 or 2; G is -NRy- or -NRjRj-, where one Rj is H or C₁₋₆alkyl and the other Rj is H, C₁₋₆alkyl or J; Ry is J; R¹⁶ is H or C₁₋₆alkyl; n is 3,4,5 or 6; the dashed line is an optional double bond.

4. A compound according to claim 3, wherein R¹⁶ is H or methyl and/or n is 4 or 5.

5. A compound according to claim 1 with the structure:

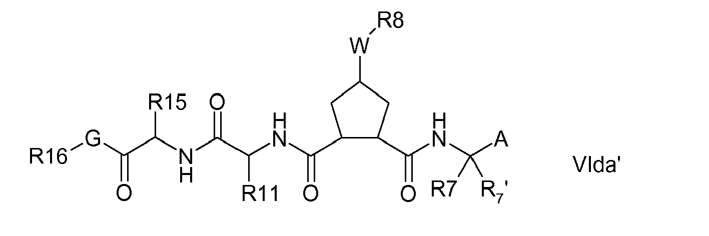

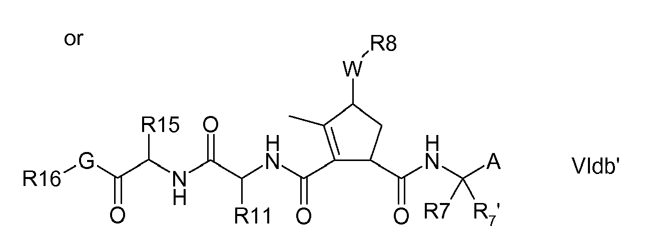

6. A compound according to any of claims claims 1-5, wherein R¹¹ is C₃₋₇cycloalkyl, aryl, Het, C₁₋₆alkyl optionally substituted with C₃₋₇cycloalkyl, Het or aryl.

7. A compound according to claim 7, wherein R¹⁵ is *tert*-butyl, isopropyl, or isobutyl, cyclohexyl, or cyclohexylmethyl.

8. A compound according to claim 1, wherein G is NRy, wherein Ry is H or methyl or wherein G is -NRjNRj-, wherein one Rj is J or H, and the other Rj is H or methyl.

9. A compound according to claim 1, wherein W is -O- and R⁸ is a radical of formula 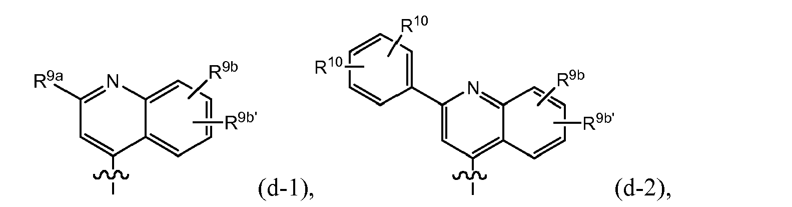 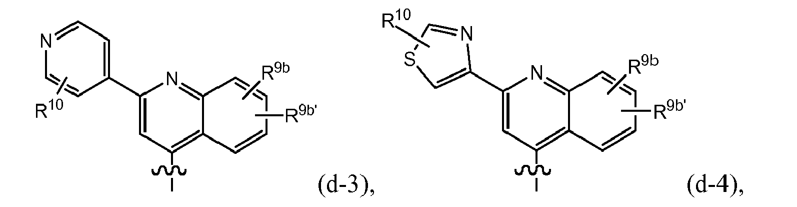 or in particular a radical of formula 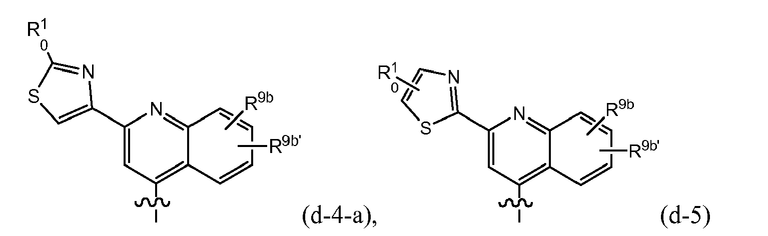 or in particular 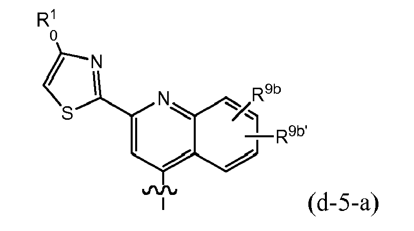 wherein in radicals (d-1) - (d-5), as well as in (d-4-a) and (d-5-a): each R^{9a} , R^{9b} , R^{9b'} are independently any of the substituents selected from those mentioned as possible substituents on the monocyclic or bicyclic ring systems of R⁸, as specified in claim 1; each R¹⁰ is independently any of the substituents selected from those mentioned as possible substituents of R⁹, as specified in the definitions of claim 1.

10. A compound according to claim 1, wherein A is -CO-CO-NR⁶R⁶
wherein R⁶ is aryl, C₃₋₇cycloalkyl, or C₁₋₆alkyl optionally substituted with aryl, C₃₋₇cycloalkyl, or with Het; or two R⁶ groups, which are bound to the same nitrogen atom, form together with that nitrogen a 3- to 7-membered nitrogen-containing heterocyclic ring.
